# EUROPEAN PATENT SPECIFICATION

(11) **EP 1 421 112 B1**
(45) Date of publication and mention of the grant of the patent: **07.10.2009**
(21) Application number: 02748459.1
(22) Date of filing: 30.07.2002
(51) Int. Cl.: C07K 14/65, C07K 14/71, C12N 15/12, A61K 38/48, A61K 38/57, A61K 31/7088, A61P 15/00

(54) **NOVEL SERINE PROTEASE**
NEUE SERINPROTEASE
NOUVELLE SERINE PROTEASE

(30) Priority: 30.07.2001 AU PP670701
(43) Date of publication of application: 26.05.2004
(73) Proprietor: Prince Henry's Institute of Medical Research, Clayton, VIC 3168 (AU)
(72) Inventor: NIE, Guiying, Melbourne, Victoria 3148 (AU); SALAMONSEN, Lois, Adrienne, Melbourne, Victoria 3101 (AU); FINDLAY, John, Kerr, Melbourne, Victoria 3122 (AU)
(74) Representative: Webber, Philip Michael
(86) International application number: PCT/AU2002/001010
(87) International publication number: WO 2003/011905

(56) References cited:
- DATABASE Geneseq [Online] 3 October 2000 (2000-10-03), "A HtrA-2 (high temperature requirement A-2) protein." XP002309774 retrieved from EBI accession no. GSN:AAY93961 Database accession no. AAY93961 -& WO 00/39149 A (MILLENNIUM PHARM INC) 6 July 2000 (2000-07-06)
- DATABASE UniProt [Online] 1 May 1999 (1999-05-01), "Serine protease (Fragment)." XP002309775 retrieved from EBI accession no. UNIPROT:Q9Z1Y2 Database accession no. Q9Z1Y2 -& HU SHOU-IH ET AL: "Human HtrA, an evolutionarily conserved serine protease identified as a differentially expressed gene product in osteoarthritic cartilage" JOURNAL OF BIOLOGICAL CHEMISTRY, vol. 273, no. 51, 18 December 1998 (1998-12-18), pages 34406-34412, XP002309772 ISSN: 0021-9258
- DATABASE UniProt [Online] 16 October 2001 (2001-10-16), "Probable serine protease HTRA3 precursor (EC 3.4.21.-)." XP002309776 retrieved from EBI accession no. UNIPROT:HRA3_HUMAN Database accession no. P83110 -& MAMMALIAN GENE COLLECTION (MGC) PROGRAM TEAM: "Generation and initial analysis of more than 15,000 full-length human and mouse cDNA sequences." PROCEEDINGS OF THE NATIONAL ACADEMY OF SCIENCES OF THE UNITED STATES OF AMERICA, vol. 99, no. 26, 24 December 2002 (2002-12-24), pages 16899-16903, XP002309773 ISSN: 0027-8424
- DATABASE SWISS-PROT [Online] XP002309776 Database accession no. (P83110)
- DATABASE PROTEIN [Online] XP002985112 Database accession no. (AAK71475)
- KAWAI J. ET AL.: 'Functional annotation of a full-length mouse cDNA collection' NATURE vol. 409, 08 February 2001, pages 685 - 690, XP002207921
- ZUMBRUNN J. ET AL.: 'Primary structure of a putative serine protease specific for IGF-binding proteins' FEBS LETTERS vol. 398, no. 2-3, 1996, pages 187 - 192, XP002931381

### SEQUENCE LISTING

<110> Prince Henry's Institute of Medical Research
<120> Novel Serine Protease
<130> P42952
<140> PR6707
   <141> 2001-07-30
<160> 55
<170> Patent In version 3.1
<210> 1
   <211> 14
   <212> DNA
   <213> artificial sequence
<220>
   <223> 3' primer
<220>
   <221> misc_feature
   <222> (13)..(13)
   <223> n=g,a or c
<400> 1
   tttttttttt ttna 14
<210> 2
   <211> 14
   <212> DNA
   <213> artificial sequence
<220>
   <223> 3' primer
<220>
   <221> misc_feature
   <222> (13)..(13)
   <223> n=g,a or c
<400> 2
   tttttttttt ttnc 14
<210> 3
   <211> 14
   <212> DNA
   <213> artificial sequence
<220>
   <223> 3' primer
<220>
   <221> misc_feature
   <222> (13)..(13)
   <223> n=g,a or c
<400> 3
   tttttttttt ttng 14
<210> 4
   <211> 14
   <212> DNA
   <213> artificial sequence
<220>
   <223> 3' primer
<220>
   <221> misc_feature
   <222> (13)..(13)
   <223> n=g,c or a
<400> 4
   tttttttttt ttnt 14
<210> 5
   <211> 10
   <212> DNA
   <213> artificial sequence
<220>
   <223> 3' primer
<400> 5
   caggcccttc 10
<210> 6
   <211> 10
   <212> DNA
   <213> artificial sequence
<220>
   <223> 3' primer
<400> 6
   tgccgagctg 10
<210> 7
   <211> 10
   <212> DNA
   <213> artificial sequence
<220>
   <223> 3' primer
<400> 7
   agtcagccac 10
<210> 8
   <211> 10
   <212> DNA
   <213> artificial sequence
<220>
   <223> 3' primer
<400> 8
   aatcgggctg 10
<210> 9
   <211> 10
   <212> DNA
   <213> artificial sequence
<220>
   <223> 3' primer
<400> 9
   aggggtcttg 10
<210> 10
   <211> 10
   <212> DNA
   <213> artificial sequence
<220>
   <223> 3' primer
<400> 10
   ggtccctgac 10
<210> 11
   <211> 10
   <212> DNA
   <213> artificial sequence
<220>
   <223> 3' primer
<400> 11
   gaaacgggtg 10
<210> 12
   <211> 10
   <212> DNA
   <213> artificial sequence
<220>
   <223> 3' primer
<400> 12
   gtgacgtagg 10
<210> 13
   <211> 10
   <212> DNA
   <213> artificial sequence
<220>
   <223> 3' primer
<400> 13
   gggtaacgcc 10
<210> 14
   <211> 10
   <212> DNA
   <213> artificial sequence
<220>
   <223> 3' primer
<400> 14
   gtgatcgcag 10
<210> 15
   <211> 10
   <212> DNA
   <213> artificial sequence
<220>
   <223> 3' primer
<400> 15
   caatcgccgt 10
<210> 16
   <211> 10
   <212> DNA
   <213> artificial sequence
<220>
   <223> 3' primer
<400> 16
   tcggcgatag 10
<210> 17
   <211> 10
   <212> DNA
   <213> artificial sequence
<220>
   <223> 3' primer
<400> 17
   cagcacccac 10
<210> 18
   <211> 10
   <212> DNA
   <213> artificial sequence
<220>
   <223> 3' primer
<400> 18
   tctgtgctgg 10
<210> 19
   <211> 10
   <212> DNA
   <213> artificial sequence
<220>
   <223> 3' primer
<400> 19
   ttccgaaccc 10
<210> 20
   <211> 10
   <212> DNA
   <213> artificial sequence
<220>
   <223> 3' primer
<400> 20
   agccagcgaa 10
<210> 21
   <211> 10
   <212> DNA
   <213> artificial sequence
<220>
   <223> 3' primer
<400> 21
   gaccgcttgt 10
<210> 22
   <211> 10
   <212> DNA
   <213> artificial sequence
<220>
   <223> 3' primer
<400> 22
   aggtgaccgt 10
<210> 23
   <211> 10
   <212> DNA
   <213> artificial sequence
<220>
   <223> 3' primer
<400> 23
   caaacgtcgg 10
<210> 24
   <211> 10
   <212> DNA
   <213> artificial sequence
<220>
   <223> 3' primer
<400> 24
   gttgcgatcc 10
<210> 25
   <211> 359
   <212> DNA
   <213> Mus musculus
<400> 25
   tctgtgctgg ccaggatgga caggaagatg agtttcataa tcacatggtc tccaaccctg     60
   acagctcatt ctcccaaggt gactacacgg tggccaaaga ggagcggaca cctgcctgag    120
   gtgcaaggac tgagccactt cacctctgca tgcagttctg ggtgcggcag ctgtctatga    180
   agatggcgcc acccagcagc cagcaggctc ccaagggcat ctttgttctc cctagtgttt    240
   caagtgtatt tgtgagcatt gctgtaaagt ttctcccact acccacattg cttgtactgt    300
   atgtttctct actgtatggc attaaagttt acaagcacat agctgccaaa aaaaaaaaa    359
<210> 26
   <211> 2450
   <212> DNA
   <213> Mus musculus
<400> 26
   gaagctcggc tgagagaggc ccgggtcagt ccccacacca tgccctgttt gcgctccggg 60
   ccagagtgcg cctgagcggt tcgggcctcg gtatccccgc gggtcttgcg ccgccgcctc 120
   tccgcgatgc aggcgcgcgc gctgctcccc gccacgctgg ccattctggc cacgctggct 180
   gtgttggctc tggcccggga gcccccagcg gctccgtgtc ctgcgcgctg cgacgtgtcg 240
   cgctgtccga gccctcgctg ccctgggggc tatgtgcctg acctctgcaa ctgctgcctg 300
   gtgtgcgctg ccagcgaggg cgagccctgc ggccgccccc tggactctcc gtgcggggac 360
   agtctggagt gcgtgcgcgg cgtgtgccgc tgccgttgga cccacactgt gtgtggcaca 420
   gacgggcata cttatgccga cgtgtgcgcg ctgcaggccg ccagccgtcg tgcgttgcag 480
   gtctccggga ctccagtgcg ccagctgcag aagggtgcct gtccctctgg tctccaccag 540
   ctgaccagtc cgcggtacaa gttcaacttc atcgccgatg tggtggagaa gattgcgcca 600
   gctgtggtcc acatagagct ctttctgaga caccccctgt ttggccggaa tgtgccgctg 660
   tccagtggct cgggcttcat catgtcagaa gccggtttga tcgtcaccaa cgcccacgtg 720
   gtctccagct ccagcactgc ctccggccgg cagcagctga aggtgcagct gcagaatggg 780
   gatgcctatg aggccaccat ccaggacatc gacaagaagt cggacattgc cacgattgta 840
   atccacccca agaaaaagct ccctgtgttg ctgctgggtc actcagcaga cctgcggcct 900
   ggcgagttcg tggtggccat cggcagcccc tttgccctgc agaacaccgt gacaacgggc 960
   attgtcagca ctgcccagcg ggatggcaag gagctgggtc tccgggactc agacatggac 1020
   tatatccaga ccgatgccat catcaattac gggaactcag gaggacccct ggtgaacctg 1080
   gatggcgagg tcatcggcat caacacgctc aaggttgcag ctggcatctc ctttgccatc 1140
   ccctcagatc gcatcacacg cttcctctct gagttccaaa acaagcatgt gaaagactgg 1200
   aagaagcgct tcattggcat ccggatgcgg accatcacgc caagtttggt ggaggaactg 1260
   aaggccgcca acccagactt tccagcggtc agcagtggaa tatatgttca agaggtggtt 1320
   cccaattcac cttctcagag aggaggcatc caagatggcg acatcatcgt caaagtcaat 1380
   ggccgccccc tggcggattc cagcgagctg caggaggcag tcctgaacga gtcttcactc 1440
   ctgctggagg tgcggcgagg caatgatgat ctcctcttca gcatcatccc tgaggtggtc 1500
   atgtgaggct actctcatcc agtgccatgc caaagcctac agaaggtggg gttccggcct 1560
   tcatgaaatc aggacaaacg gctgctgtgg tcctcagcag gatcaacagt ctcctctctg 1620
   ggtccagcgc tgagtccaag gctggatcta accaggggtc cggatctcag ccttgaccct 1680
   taatttcagc tccagtagag gaagcacagc gtcctttgga ccagatgctc ctgatgttac 1740
   cgtctgagtt ctctaggcct agaagctctt agaaacctcc ctggaagtct gcccttcccc 1800
   cacccccacc ccagctttct gcctctgccc tcaggaaggc ccacccggct cccatcccac 1860
   ctcttctccc ttgtatccca gtgcctcaac ctctccctgt tacaggcact ttcctgacac 1920
   taccaggctt ccatctgcct cagcacaccc cacccccatg gtaagacagg ggctgcttgc 1980
   cctaccaccc ggtatccctg gagggcaggc cctgtagctg tcccctggag aagccagggt 2040
   cctgacctgg agcaggttaa catccctcac tgctgagctg agccctgtgc tggccaggat 2100
   ggacaggaag atgagtttca taatcacgtg gtctccaacc ctgacagctc attctcccaa 2160
   ggtgactaca cggtggccaa agaggagcgg acacctgcct gaggtgcaag gactgagcca 2220
   cttcacctct gcatgcagtt ctgggtgcgg cagctgtctg tgaagatggc gccacccagc 2280
   agccagcagg ctcccaaggg catctttgtt ctccctagtg tttcaagtgt atttgtgagc 2340
   attgctgtaa agtttctccc actacccaca ttgcttgtac tgtatgtttc tctactgtat 2400
   ggcattaaag tttacaagca catagctgtc aaccagaaaa aaaaaattcc 2450
<210> 27
   <211> 459
   <212> PRT
   <213> Mus musculus
<400> 27
   Met Gln Ala Arg Ala Leu Leu Pro Ala Thr Leu Ala Ile Leu Ala Thr
   1 5 10 15
   Leu Ala Val Leu Ala Leu Ala Arg Glu Pro Pro Ala Ala Pro Cys Pro
   20 25 30
   Ala Arg Cys Asp Val Ser Arg Cys Pro Ser Pro Arg Cys Pro Gly Gly
   35 40 45
   Tyr Val Pro Asp Leu Cys Asn Cys Cys Leu Val Cys Ala Ala Ser Glu
   50 55 60
   Gly Glu Pro Cys Gly Arg Pro Leu Asp Ser Pro Cys Gly Asp Ser Leu
   65 70 75 80
   Glu Cys Val Arg Gly Val Cys Arg Cys Arg Trp Thr His Thr Val Cys
   85 90 95
   Gly Thr Asp Gly His Thr Tyr Ala Asp Val Cys Ala Leu Gln Ala Ala
   100 105 110
   Ser Arg Arg Ala Leu Gln Val Ser Gly Thr Pro Val Arg Gln Leu Gln
   115 120 125
   Lys Gly Ala Cys Pro Ser Gly Leu His Gln Leu Thr Ser Pro Arg Tyr
   130 135 140
   Lys Phe Asn Phe Ile Ala Asp Val Val Glu Lys Ile Ala Pro Ala Val
   145 150 155 160
   Val His Ile Glu Leu Phe Leu Arg His Pro Leu Phe Gly Arg Asn Val
   165 170 175
   Pro Leu Ser Ser Gly Ser Gly Phe Ile Met Ser Glu Ala Gly Leu Ile
   180 185 190
   Val Thr Asn Ala His Val Val Ser Ser Ser Ser Thr Ala Ser Gly Arg
   195 200 205
   Gln Gln Leu Lys Val Gln Leu Gln Asn Gly Asp Ala Tyr Glu Ala Thr
   210 215 220
   Ile Gln Asp Ile Asp Lys Lys Ser Asp Ile Ala Thr Ile Val Ile His
   225 230 235 240
   Pro Lys Lys Lys Leu Pro Val Leu Leu Leu Gly His Ser Ala Asp Leu
   245 250 255
   Arg Pro Gly Glu Phe Val Val Ala Ile Gly Ser Pro Phe Ala Leu Gln
   260 265 270
   Asn Thr Val Thr Thr Gly Ile Val Ser Thr Ala Gln Arg Asp Gly Lys
   275 280 285
   Glu Leu Gly Leu Arg Asp Ser Asp Met Asp Tyr Ile Gln Thr Asp Ala
   290 295 300
   Ile Ile Asn Tyr Gly Asn Ser Gly Gly Pro Leu Val Asn Leu Asp Gly
   305 310 315 320
   Glu Val Ile Gly Ile Asn Thr Leu Lys Val Ala Ala Gly Ile Ser Phe
   325 330 335
   Ala Ile Pro Ser Asp Arg Ile Thr Arg Phe Leu Ser Glu Phe Gln Asn
   340 345 350
   Lys His Val Lys Asp Trp Lys Lys Arg Phe Ile Gly Ile Arg Met Arg
   355 360 365
   Thr Ile Thr Pro Ser Leu Val Glu Glu Leu Lys Ala Ala Asn Pro Asp
   370 375 380
   Phe Pro Ala Val Ser Ser Gly Ile Tyr Val Gln Glu Val Val Pro Asn
   385 390 395 400
   Ser Pro Ser Gln Arg Gly Gly Ile Gln Asp Gly Asp Ile Ile Val Lys
   405 410 415
   Val Asn Gly Arg Pro Leu Ala Asp Ser Ser Glu Leu Gln Glu Ala Val
   420 425 430
   Leu Asn Glu Ser Ser Leu Leu Leu Glu Val Arg Arg Gly Asn Asp Asp
   435 440 445
   Leu Leu Phe Ser Ile Ile Pro Glu Val Val Met
   450 455
<210> 28
   <211> 7
   <212> PRT
   <213> artificial sequence
<220>
   <223> Bacterial HtrA activwe site motif
<400> 28
   Gly Asn Ser Gly Gly Ala Leu
   1 5
<210> 29
   <211> 7
   <212> PRT
   <213> artificial sequence
<220>
   <223> Mammalian HtrA active site motif
<400> 29
   Gly Asn Ser Gly Gly Pro Leu
   1 5
<210> 30
   <211> 5
   <212> PRT
   <213> artificial sequence
<220>
   <223> HtrA second active site motif
<400> 30
   Thr Asn Ala His Val
   1 5
<210> 31
   <211> 2543
   <212> DNA
   <213> Homo sapiens
<400> 31
   gtgcgctccc tgcgccctgg ggatgcccct gccgccctga cgcccgccag cctgagccac    60
   cggcgcatgt gaccgcgcgt ccgccccagt cccatccgta ggcgcccggc gcccggcccc   120
   gcagcggcct cgttgtcccc gccggccccc gcccggtctc ccgcgctgcc acccgccgcc   180
   ggccctgccg ccatgcaggc gcgagcgctg ctcctggccg cgttggccgc gctggcgctg   240
   gcccgggagc cccctgcggc gccgtgtccc gcgcgctgcg acgtgtcgcg gtgtcccagc   300
   ccccgctgcc ccggcggcta cgtgcccgac ctctgcaact gctgcctggt gtgcgccgcc   360
   agcgagggcg agccctgtgg cggccctctg gactcgcctt gcggcgagag cctggagtgc   420
   gtgcgcggcc tatgccgctg ccgctggtcg cacgccgtgt gtggcaccga cgggcacacc   480
   tatgccaacg tgtgcgcgct gcaggcggcc agccgccgcg cgctgcagct ctccgggacg   540
   cccgtgcgcc agctgcagaa gggcgcctgc ccgttgggtc tccaccagct gagcagcccg   600
   cgctacaagt tcaacttcat tgctgacgtg gtggagaaga tcgcaccagc cgtggtccac   660
   atagagctct tcctgagaca cccgctgttt ggccgcaacg tgcccctgtc cagcggttct 720
   ggcttcatca tgtcagaggc cggcctgatc atcaccaatg cccacgtggt gtccagcaac 780
   agtgctgccc cgggcaggca gcagctcaag gtgcagctac agaatgggga ctcctatgag 840
   gccaccatca aagacatcga caagaagtcg gacattgcca ccatcaagat ccatcccaag 900
   aaaaagctcc ctgtgttgtt gctgggtcac tcggccgacc tgcggcctgg ggagtttgtg 960
   gtggccatcg gcagtccctt cgccctacag aacacagtga caacgggcat cgtcagcact 1020
   gcccagcggg agggcaggga gctgggcctc cgggactccg acatggacta catccagacg 1080
   gatgccatca tcaactacgg gaactccggg ggaccactgg tgaacctgga tggcgaggtc 1140
   attggcatca acacgctcaa ggtcacggct ggcatctcct ttgccatccc ctcagaccgc 1200
   atcacacggt tcctcacaga gttccaagac aagcagatca aagactggaa gaagcgcttc 1260
   atcggcatac ggatgcggac gatcacacca agcctggtgg atgagctgaa ggccagcaac 1320
   ccggacttcc cagaggtcag cagtggaatt tatgtgcaag aggttgcgcc gaattcacct 1380
   tctcagagag gcggcatcca agatggtgac atcatcgtca aggtcaacgg gcgtcctcta 1440
   gtggactcga gtgagctgca ggaggccgtg ctgaccgagt ctcctctcct actggaggtg 1500
   cggcggggga acgacgacct cctcttcagc atcgcacctg aggtggtcat gtgaggggcg 1560
   cattcctcca gcgccaagcg tcagagcctg cagacaacgg agggcagcgc ccccccgaga 1620
   tcaggacgaa ggaccaccgt cggtcctcag cagggcggca gcctcctcct ggctgtccgg 1680
   ggcagagcgg aggctgggct tggccagggg cccgaatttc cgcctgggga gtgttggatc 1740
   cacatcccgg tgccggggag ggaagcccaa catccccttg tacagatgat cctgaaagtc 1800
   acttccaagt tctccggata ttcacaaaac tgccttccat ggaggtcccc tcctctccta 1860
   gcttcccgcc tctgcccctg tgaacaccca tctgcagtat cccctgctcc tgcccctcct 1920
   actgcaggtc tgggctgcca agcttcttcc cccctgacaa acgcccacct gacctgaggc 1980
   cccagcttcc ctctgcccta ggacttacca agctgtaggg ccagggctgc tgcctgccag 2040
   cctggggtcc ctggaggaca ggtcacatct gatccctttg gggtgcgggg gtggggtcca 2100
   gcccagagca ggcactgagt gaatgccccc tggctgcgga gctgagcccc gccctgccat 2160
   gaggttttcc tccccaggca ggcaggaggc cgcggggagc acgtggaaag ttggctgctg 2220
   cctggggaag cttctcctcc ccaaggcggc catggggcag cctgcagagg acagtggacg 2280
   tggagctgcg gggtgtgagg actgagccgg cttccccttc ccacgcagct ctgggatgca 2340
   gcagccgctc gcatggaagt gccgcccaga ggcatgcagg ctgctgggca ccaccccctc 2400
   atccagggaa cgagtgtgtc tcaaggggca tttgtgagct ttgctgtaaa tggattccca 2460
   gtgttgcttg tactgtatgt ttctctactg tatggaaaat aaagtttaca agcacacggt 2520
   tctcaaaaaa aaaaaaaaaa aaa 2543
<210> 32
   <211> 1953
   <212> DNA
   <213> Homo sapiens
<400> 32
   ccagtcccat ccgtaggcgc ccggcgcccg gccccgcagc ggcctcgttg tccccgccgg 60
   cccccgcccg gtctcccgcg ctgccacccg ccgccggccc tgccgccatg caggcgcgag 120
   cgctgctcct ggccgcgttg gccgcgctgg cgctggcccg ggagccccct gcggcgccgt 180
   gtcccgcgcg ctgcgacgtg tcgcggtgtc ccagcccccg ctgccccggc ggctacgtgc 240
   ccgacctctg caactgctgc ctggtgtgcg ccgccagcga gggcgagccc tgtggcggcc 300
   ctctggactc gccttgcggc gagagcctgg agtgcgtgcg cggcctatgc cgctgccgct 360
   ggtcgcacgc cgtgtgtggc accgacgggc acacctatgc caacgtgtgc gcgctgcagg 420
   cggccagccg ccgcgcgctg cagctctccg ggacgcccgt gcgccagctg cagaagggcg 480
   cctgcccgtt gggtctccac cagctgagca gcccgcgcta caagttcaac ttcattgctg 540
   acgtggtgga gaagatcgca ccagccgtgg tccacataga gctcttcctg agacacccgc 600
   tgtttggccg caacgtgccc ctgtccagcg gttctggctt catcatgtca gaggccggcc 660
   tgatcatcac caatgcccac gtggtgtcca gcaacagtgc tgccccgggc aggcagcagc 720
   tcaaggtgca gctacagaat ggggactcct atgaggccac catcaaagac atcgacaaga 780
   agtcggacat tgccaccatc aagatccatc ccaagaaaaa gctccctgtg ttgttgctgg 840
   gtcactcggc cgacctgcgg cctggggagt ttgtggtggc catcggcagt cccttcgccc 900
   tacagaacac agtgacaacg ggcatcgtca gcactgccca gcgggagggc agggagctgg 960
   gcctccggga ctccgacatg gactacatcc agacggatgc catcatcaac tacgggaact 1020
   ccgggggacc actggtgaac ctggatggcg aggtcattgg catcaacacg ctcaaggtca 1080
   cggctggcat ctcctttgcc atcccctcag accgcatcac acggttcctc acagagttcc 1140
   aagacaagca gatcaaagcc ccctcactgg cagttcattg agagcagggg gcttcctcac 1200
   gtttccccct cctccatgac cccgtcagcc aagcacatgg accccagtgc agccaaggct 1260
   ggtgccatga gggctggtca catgaagagc tgctgttgag gatgccgcca ttgttcttct 1320
   gtgtccatta tgggaagaca atctggagcc aggcagagcc tgtctttccc aaagaagctg 1380
   aagtcttctt ctcttgaaca gtggggacca tctaatctct tgagcccttt tcctgttggc 1440
   ttctaggaag ctcagagcta gattcagggg tgcacccaga cctgtcctag catgctcctt 1500
   tccctaatga ccgagtcttt cctgttgaat tatcccattc tccatgggtg cctttgactt 1560
   tggcctcctt actggaaatt agcggagctg ctgtttgcac acactgagct gtgaggtggc 1620
   tttccttgga agtggatgat agtgtcctct tcccttcttg cctctctctt tctcctgaga 1680
   caggatcccc ctggggccta ggtttgctcc tttgttgtac aggggctgtc ccagttagtg 1740
   ctgacctcat cccagaaccc cctgggaaat atcccctgtc ctcagagctg tgtcccctcc 1800
   ccaaggacag tgcagactaa ctgaggagcc tgataaacct tagctgcatg gcacacttgc 1860
   aattttaaaa tccttctgaa gttgactggt gtttgtactt gcttctcttt tttatttaat 1920
   aaaatccaat gatccaaaaa aaaaaaaaaa aaa 1953
<210> 33
   <211> 453
   <212> PRT
   <213> Homo sapiens
<400> 33
   Met Gln Ala Arg Ala Leu Leu Leu Ala Ala Leu Ala Ala Leu Ala Leu
   1 5 10 15
   Ala Arg Glu Pro Pro Ala Ala Pro Cys Pro Ala Arg Cys Asp Val Ser
   20 25 30
   Arg Cys Pro Ser Pro Arg Cys Pro Gly Gly Tyr Val Pro Asp Leu Cys
   35 40 45
   Asn Cys Cys Leu Val Cys Ala Ala Ser Glu Gly Glu Pro Cys Gly Gly
   50 55 60
   Pro Leu Asp Ser Pro Cys Gly Glu Ser Leu Glu Cys Val Arg Gly Leu
   65 70 75 80
   Cys Arg Cys Arg Trp Ser His Ala Val Cys Gly Thr Asp Gly His Thr
   85 90 95
   Tyr Ala Asn Val Cys Ala Leu Gln Ala Ala Ser Arg Arg Ala Leu Gln
   100 105 110
   Leu Ser Gly Thr Pro Val Arg Gln Leu Gln Lys Gly Ala Cys Pro Leu
   115 120 125
   Gly Leu His Gln Leu Ser Ser Pro Arg Tyr Lys Phe Asn Phe Ile Ala
   130 135 140
   Asp Val Val Glu Lys Ile Ala Pro Ala Val Val His Ile Glu Leu Phe
   145 150 155 160
   Leu Arg His Pro Leu Phe Gly Arg Asn Val Pro Leu Ser Ser Gly Ser
   165 170 175
   Gly Phe Ile Met Ser Glu Ala Gly Leu Ile Ile Thr Asn Ala His Val
   180 185 190
   Val Ser Ser Asn Ser Ala Ala Pro Gly Arg Gln Gln Leu Lys Val Gln
   195 200 205
   Leu Gln Asn Gly Asp Ser Tyr Glu Ala Thr Ile Lys Asp Ile Asp Lys
   210 215 220
   Lys Ser Asp Ile Ala Thr Ile Lys Ile His Pro Lys Lys Lys Leu Pro
   225 230 235 240
   Val Leu Leu Leu Gly His Ser Ala Asp Leu Arg Pro Gly Glu Phe Val
   245 250 255
   Val Ala Ile Gly Ser Pro Phe Ala Leu Gln Asn Thr Val Thr Thr Gly
   260 265 270
   Ile Val Ser Thr Ala Gln Arg Glu Gly Arg Glu Leu Gly Leu Arg Asp
   275 280 285
   Ser Asp Met Asp Tyr Ile Gln Thr Asp Ala Ile Ile Asn Tyr Gly Asn
   290 295 300
   Ser Gly Gly Pro Leu Val Asn Leu Asp Gly Glu Val Ile Gly Ile Asn
   305 310 315 320
   Thr Leu Lys Val Thr Ala Gly Ile Ser Phe Ala Ile Pro Ser Asp Arg
   325 330 335
   Ile Thr Arg Phe Leu Thr Glu Phe Gln Asp Lys Gln Ile Lys Asp Trp
   340 345 350
   Lys Lys Arg Phe Ile Gly Ile Arg Met Arg Thr Ile Thr Pro Ser Leu
   355 360 365
   Val Asp Glu Leu Lys Ala Ser Asn Pro Asp Phe Pro Glu Val Ser Ser
   370 375 380
   Gly Ile Tyr Val Gln Glu Val Ala Pro Asn Ser Pro Ser Gln Arg Gly
   385 390 395 400
   Gly Ile Gln Asp Gly Asp Ile Ile Val Lys Val Asn Gly Arg Pro Leu
   405 410 415
   Val Asp Ser Ser Glu Leu Gln Glu Ala Val Leu Thr Glu Ser Pro Leu
   420 425 430
   Leu Leu Glu Val Arg Arg Gly Asn Asp Asp Leu Leu Phe Ser Ile Ala
   435 440 445
   Pro Glu Val Val Met
   450
<210> 34
   <211> 357
   <212> PRT
   <213> Homo sapiens
<400> 34
   Met Gln Ala Arg Ala Leu Leu Leu Ala Ala Leu Ala Ala Leu Ala Leu
   1 5 10 15
   Ala Arg Glu Pro Pro Ala Ala Pro Cys Pro Ala Arg Cys Asp Val Ser
   20 25 30
   Arg Cys Pro Ser Pro Arg Cys Pro Gly Gly Tyr Val Pro Asp Leu Cys
   35 40 45
   Asn Cys Cys Leu Val Cys Ala Ala Ser Glu Gly Glu Pro Cys Gly Gly
   50 55 60
   Pro Leu Asp Ser Pro Cys Gly Glu Ser Leu Glu Cys Val Arg Gly Leu
   65 70 75 80
   Cys Arg Cys Arg Trp Ser His Ala Val Cys Gly Thr Asp Gly His Thr
   85 90 95
   Tyr Ala Asn Val Cys Ala Leu Gln Ala Ala Ser Arg Arg Ala Leu Gln
   100 105 110
   Leu Ser Gly Thr Pro Val Arg Gln Leu Gln Lys Gly Ala Cys Pro Leu
   115 120 125
   Gly Leu His Gln Leu Ser Ser Pro Arg Tyr Lys Phe Asn Phe Ile Ala
   130 135 140
   Asp Val Val Glu Lys Ile Ala Pro Ala Val Val His Ile Glu Leu Phe
   145 150 155 160
   Leu Arg His Pro Leu Phe Gly Arg Asn Val Pro Leu Ser Ser Gly Ser
   165 170 175
   Gly Phe Ile Met Ser Glu Ala Gly Leu Ile Ile Thr Asn Ala His Val
   180 185 190
   Val Ser Ser Asn Ser Ala Ala Pro Gly Arg Gln Gln Leu Lys Val Gln
   195 200 205
   Leu Gln Asn Gly Asp Ser Tyr Glu Ala Thr Ile Lys Asp Ile Asp Lys
   210 215 220
   Lys Ser Asp Ile Ala Thr Ile Lys Ile His Pro Lys Lys Lys Leu Pro
   225 230 235 240
   Val Leu Leu Leu Gly His Ser Ala Asp Leu Arg Pro Gly Glu Phe Val
   245 250 255
   Val Ala Ile Gly Ser Pro Phe Ala Leu Gln Asn Thr Val Thr Thr Gly
   260 265 270
   Ile Val Ser Thr Ala Gln Arg Glu Gly Arg Glu Leu Gly Leu Arg Asp
   275 280 285
   Ser Asp Met Asp Tyr Ile Gln Thr Asp Ala Ile Ile Asn Tyr Gly Asn
   290 295 300
   Ser Gly Gly Pro Leu Val Asn Leu Asp Gly Glu Val Ile Gly Ile Asn
   305 310 315 320
   Thr Leu Lys Val Thr Ala Gly Ile Ser Phe Ala Ile Pro Ser Asp Arg
   325 330 335
   Ile Thr Arg Phe Leu Thr Glu Phe Gln Asp Lys Gln Ile Lys Ala Pro
   340 345 350
   Ser Leu Ala Val His
   355
<210> 35
   <211> 17
   <212> DNA
   <213> artificial sequence
<220>
   <223> Forward primer for splice site
<400> 35
   ggcatcaaca cgctcaa 17
<210> 36
   <211> 39
   <212> DNA
   <213> artificial sequence
<220>
   <223> Backward primer for splice site
<400> 36
   gaccacgcgt atcgatgtcg actttttttt ttttttttv 39
<210> 37
   <211> 476
   <212> DNA
   <213> artificial sequence
<220>
   <223> Probe for short isoform of mouse uterine protease
<400> 37
   ccatgaagaa ctgcaaccga ggagcctcgt tctgttccaa gtggccctat atgaagatga 60
   caggagcagg cagagcctgt cccttccagg aatccgagac accttctggt gaatagtggg 120
   aactagctgc cttttctctt ggccggtagg aagctcagaa ctagaccagg gttcctagac 180
   cattggtagc cttggctctt tgtctagtgg ccagggcttt ccagtttagc ttgtttatgg 240
   ggtcggaaca ccacccacat acactggcct atgggtgatt actgtgctgg aaatgggcca 300
   gcggcctttt gtcccctagc tgtctcatct tttctcagac aagaagtccc cggggcagga 360
   tctgctcctc tgtggcagag caactatcct agtcacagtg acctggtcac tcagcctggg 420
   ctctgcggaa atgctcacac ccatcccaga gttatgttat cacccaagga cagtgc 476
<210> 38
   <211> 1897
   <212> DNA
   <213> Mus musculus
<400> 38
   gaagctcggc tgagagaggc ccgggtcagt ccccacacca tgccctgttt gcgctccggg 60
   ccagagtgcg cctgagcggt tcgggcctcg gtatccccgc gggtcttgcg ccgccgcctc 120
   tccgcgatgc aggcgcgcgc gctgctcccc gccacgctgg ccattctggc cacgctggct 180
   gtgttggctc tggcccggga gcccccagcg gctccgtgtc ctgcgcgctg cgacgtgtcg 240
   cgctgtccga gccctcgctg ccctgggggc tatgtgcctg acctctgcaa ctgctgcctg 300
   gtgtgcgctg ccagcgaggg cgagccctgc ggccgccccc tggactctcc gtgcggggac 360
   agtctggagt gcgtgcgcgg cgtgtgccgc tgccgttgga cccacactgt gtgtggcaca 420
   gacgggcata cttatgccga cgtgtgcgcg ctgcaggccg ccagccgtcg tgcgttgcag 480
   gtctccggga ctccagtgcg ccagctgcag aagggtgcct gtccctctgg tctccaccag 540
   ctgaccagtc cgcggtacaa gttcaacttc atcgccgatg tggtggagaa gattgcgcca 600
   gctgtggtcc acatagagct ctttctgaga caccccctgt ttggccggaa tgtgccgctg 660
   tccagtggct cgggcttcat catgtcagaa gccggtttga tcgtcaccaa cgcccacgtg 720
   gtctccagct ccagcactgc ctccggccgg cagcagctga aggtgcagct gcagaatggg 780
   gatgcctatg aggccaccat ccaggacatc gacaagaagt cggacattgc cacgattgta 840
   atccacccca agaaaaagct ccctgtgttg ctgctgggtc actcagcaga cctgcggcct 900
   ggcgagttcg tggtggccat cggcagcccc tttgccctgc agaacaccgt gacaacgggc 960
   attgtcagca ctgcccagcg ggatggcaag gagctgggtc tccgggactc agacatggac 1020
   tatatccaga ccgatgccat catcaattac gggaactcag gaggacccct ggtgaacctg 1080
   gatggcgagg tcatcggcat caacacgctc aaggttgcag ctggcatctc ctttgccatc 1140
   ccctcagatc gcatcacacg cttcctctct gagttccaaa acaagcatgt gaaagccctc 1200
   tcaccagcac tgcactgaga gcaggggcct tcctcctgct tgccccctcc tttgcggccc   1260
   tgccagccac acacaaggac cccagtacag ccaagactgg tcccatgaag aactgcaacc   1320
   gaggagcctc gttctgttcc aagtggccct atatgaagat gacaggagca ggcagagcct   1380
   gtcccttcca ggaatccgag acaccttctg gtgaatagtg ggaactagct gccttttctc   1440
   ttggccggta ggaagctcag aactagacca gggttcctag accattggta gccttggctc   1500
   tttgtctagt ggccagggct ttccagttta gcttgtttat ggggtcggaa caccacccac   1560
   atacactggc ctatgggtga ttactgtgct ggaaatgggc cagcggcctt ttgtccccta   1620
   gctgtctcat cttttctcag acaagaagtc cccggggcag gatctgctcc tctgtggcag   1680
   agcaactatc ctagtcacag tgacctggtc actcagcctg ggctctgcgg aaatgctcac   1740
   acccatccca gagttatgtt atcacccaag gacagtgctt acctactaca agagggtctg   1800
   acgaggctta gctaagtggg gtccattgac ttaaagtcct tctgaaattt gtgcttattt   1860
   atgcttttcc atttttaaat aaaaacatca gatgatc                            1897
<210> 39
   <211> 363
   <212> PRT
   <213> Mus musculus
<400> 39
   Met Gln Ala Arg Ala Leu Leu Pro Ala Thr Leu Ala Ile Leu Ala Thr
   1               5                   10                  15
   Leu Ala Val Leu Ala Leu Ala Arg Glu Pro Pro Ala Ala Pro Cys Pro
               20                  25                  30
   Ala Arg Cys Asp Val Ser Arg Cys Pro Ser Pro Arg Cys Pro Gly Gly
           35                  40                  45
   Tyr Val Pro Asp Leu Cys Asn Cys Cys Leu Val Cys Ala Ala Ser Glu
       50                  55                  60
   Gly Glu Pro Cys Gly Arg Pro Leu Asp Ser Pro Cys Gly Asp Ser Leu
   65                  70                  75                  80
   Glu Cys Val Arg Gly Val Cys Arg Cys Arg Trp Thr His Thr Val Cys
                   85                  90                  95
   Gly Thr Asp Gly His Thr Tyr Ala Asp Val Cys Ala Leu Gln Ala Ala
   100 105 110
   Ser Arg Arg Ala Leu Gln Val Ser Gly Thr Pro Val Arg Gln Leu Gln
   115 120 125
   Lys Gly Ala Cys Pro Ser Gly Leu His Gln Leu Thr Ser Pro Arg Tyr
   130 135 140
   Lys Phe Asn Phe Ile Ala Asp Val Val Glu Lys Ile Ala Pro Ala Val
   145 150 155 160
   Val His Ile Glu Leu Phe Leu Arg His Pro Leu Phe Gly Arg Asn Val
   165 170 175
   Pro Leu Ser Ser Gly Ser Gly Phe Ile Met Ser Glu Ala Gly Leu Ile
   180 185 190
   Val Thr Asn Ala His Val Val Ser Ser Ser Ser Thr Ala Ser Gly Arg
   195 200 205
   Gln Gln Leu Lys Val Gln Leu Gln Asn Gly Asp Ala Tyr Glu Ala Thr
   210 215 220
   Ile Gln Asp Ile Asp Lys Lys Ser Asp Ile Ala Thr Ile Val Ile His
   225 230 235 240
   Pro Lys Lys Lys Leu Pro Val Leu Leu Leu Gly His Ser Ala Asp Leu
   245 250 255
   Arg Pro Gly Glu Phe Val Val Ala Ile Gly Ser Pro Phe Ala Leu Gln
   260 265 270
   Asn Thr Val Thr Thr Gly Ile Val Ser Thr Ala Gln Arg Asp Gly Lys
   275 280 285
   Glu Leu Gly Leu Arg Asp Ser Asp Met Asp Tyr Ile Gln Thr Asp Ala
   290 295 300
   Ile Ile Asn Tyr Gly Asn Ser Gly Gly Pro Leu Val Asn Leu Asp Gly
   305 310 315 320
   Glu Val Ile Gly Ile Asn Thr Leu Lys Val Ala Ala Gly Ile Ser Phe
   325 330 335
   Ala Ile Pro Ser Asp Arg Ile Thr Arg Phe Leu Ser Glu Phe Gln Asn
   340 345 350
   Lys His Val Lys Ala Leu Ser Pro Ala Leu His
   355 360
<210> 40
   <211> 785
   <212> DNA
   <213> artificial sequence
<220>
   <223> Probe for common region of short and long isoforms of mouse uteri ne protease
<400> 40
   gcggttcggg cctcggtatc cccgcgggtc ttgcgccgcc gcctctccgc gatgcaggcg 60
   cgcgcgctgc tccccgccac gctggccatt ctggccacgc tggctgtgtt ggctctggcc 120
   cgggagcccc cagcggctcc gtgtcctgcg cgctgcgacg tgtcgcgctg tccgagccct 180
   cgctgccctg ggggctatgt gcctgacctc tgcaactgct gcctggtgtg cgctgccagc 240
   gagggcgagc cctgcggccg ccccctggac tctccgtgcg gggacagtct ggagtgcgtg 300
   cgcggcgtgt gccgctgccg ttggacccac actgtgtgtg gcacagacgg gcatacttat 360
   gccgacgtgt gcgcgctgca ggccgccagc cgtcgtgcgt tgcaggtctc cgggactcca 420
   gtgcgccagc tgcagaaggg tgcctgtccc tctggtctcc accagctgac cagtccgcgg 480
   tacaagttca acttcatcgc cgatgtggtg gagaagattg cgccagctgt ggtccacata 540
   gagctctttc tgagacaccc cctgtttggc cggaatgtgc cgctgtccag tggctcgggc 600
   ttcatcatgt cagaagccgg tttgatcgtc accaacgccc acgtggtctc cagctccagc 660
   actgcctccg gccggcagca gctgaaggtg cagctgcaga atggggatgc ctatgaggcc 720
   accatccagg acatcgacaa gaagtcggac attgccacga ttgtaatcca ccccaagaaa 780
   aagct 785
<210> 41
   <211> 384
   <212> DNA
   <213> artificial sequence
<220>
   <223> probe for human HtrA
<400> 41
   aaagccatca ccaagaagaa gtatattggt atccgaatga tgtcactcac gtccagcaaa 60
   gccaaagagc tgaaggaccg gcaccgggac ttcccagacg tgatctcagg agcgtatata 120
   attgaagtaa ttcctgatac cccagcagaa gctggtggtc tcaaggaaaa cgacgtcata 180
   atcagcatca atggacagtc cgtggtctcc gccaatgatg tcagcgacgt cattaaaagg 240
   gaaagcaccc tgaacatggt ggtccgcagg ggtaatgaag atatcatgat cacagtgatt 300
   cccgaagaaa ttgacccata ggcagaggca tgagctggac ttcatgtttc cctcaaagac 360
   tctcccgtgg atgacggatg agga 384
<210> 42
   <211> 20
   <212> DNA
   <213> artificial sequence
<220>
   <223> Mouse long isoform upper primer
<400> 42
   atgcggacga tcacaccaag 20
<210> 43
   <211> 19
   <212> DNA
   <213> artificial sequence
<220>
   <223> Mouse long isoform lower primer
<400> 43
   cgctgccctc cgttgtctg 19
<210> 44
   <211> 20
   <212> DNA
   <213> artificial sequence
<220>
   <223> Mouse short isoform upper primer
<400> 44
   gagggctggt cacatgaaga 20
<210> 45
   <211> 18
   <212> DNA
   <213> artificial sequence
<220>
   <223> Mouse short isoform lower primer
<400> 45
   gctccgctaa tttccagt 18
<210> 46
   <211> 24
   <212> DNA
   <213> artificial sequence
<220>
   <223> HtrA upper primer
<400> 46
   aaagccatca ccaagaagaa gtat 24
<210> 47
   <211> 18
   <212> DNA
   <213> artificial sequence
<220>
   <223> HtrA lower primer
<400> 47
   tcctcatccg tcatccac 18
<210> 48
   <211> 781
   <212> DNA
   <213> artificial sequence
<220>
   <223> Sense probe for in situ hybridization detection of mouse uterine protease
<400> 48
   gtctgattcc tgcaactgct gcctggtgtg cgctgccagc gagggcgagc cctgcggccg 60
   ccccctggac tctccgtgcg gggacagtct ggagtgcgtg cgcggcgtgt gccgctgccg 120
   ttggacccac actgtgtgtg gcacagacgg gcatacttat gccgacgtgt gcgcgctgca 180
   ggccgccagc cgtcgtgcgt tgcaggtctc cgggactcca gtgcgccagc tgcagaaggg 240
   tgcctgtccc tctggtctcc accagctgac cagtccgcgg tacaagttca acttcatcgc 300
   cgatgtggtg gagaagattg cgccagctgt ggtccacata gagctctttc tgagacaccc 360
   cctgcttggc cggaatgtgc cgctgtccag tggctcgggc ttcatcatgt cagaagccgg 420
   tttgatcgtc accaacgccc acgtggtctc cagctccagc actgcctccg gccggcagca 480
   gctgaaggtg cagctgcaga atggggatgc ctatgaggcc accatccagg acatcgacaa 540
   gaagtcggac attgccacga ttgtaatcca ccccaagaaa aagctccctg tgttgctgct 600
   gggtcactca gcagacctgc ggcctggcga gttcgtggtg gccatcggca gcccctttgc 660
   cctgcagaac accgtgacaa cgggcattgt cagcactgcc cagcgggatg gcaaggagct 720
   gggtctccgg gactcagaca tggactatat ccagaccgat gccatcatca attacgggaa 780
   c 781
<210> 49
   <211> 344
   <212> DNA
   <213> Mus musculus
<400> 49
   cggacattgc cacgattgta atccacccca agaaaaagct ccctgtgttg ctgctgggtc 60
   actcagcaga cctgcggcct ggcgagttcg tggtggccat cggcagcccc tttgccctgc 120
   agaacaccgt gacaacgggc attgtcagca ctgcccagcg ggatggcaag gagctgggtc 180
   tccgggactc agacatggac tatatccaga ccgatgccat catcaattac gggaactcag 240
   gaggacccct ggtgaacctg gatggcgagg tcatcggcat caacacgctc aaggttgcag 300
   ctggcatctc ctttgccatc ccctcagatc gcatcacacg cttc 344
<210> 50
   <211> 396
   <212> DNA
   <213> Homo sapiens
<400> 50
   cggcctgatc atcaccaatg cccacgtggt gtccagcaac agtgctgccc cgggcaggca 60
   gcagctcaag gtgcagctac agaatgggga ctcctatgag gccaccatca aagacatcga 120
   caagaagtcg gacattgcca ccatcaagat ccatcccaag aaaaagctcc ctgtgttgtt 180
   gctgggtcac tcggccgacc tgcggcctgg ggagtttgtg gtggccatcg gcagtccctt 240
   cgccctacag aacacagtga caacgggcat cgtcagcact gcccagcggg agggcaggga 300
   gctgggcctc cgggactccg acatggacta catccagacg gatgccatca tcaactacgg 360
   gaactccggg ggaccactgg tgaacctgga tggcga 396
<210> 51
   <211> 11
   <212> PRT
   <213> artificial sequence
<220>
   <223> Antigenic peptide from mouse uterine protease
<400> 51
   Pro Ser Gly Leu His Gln Leu Thr Ser Pro Cys
   1 5 10
<210> 52
   <211> 12
   <212> PRT
   <213> artificial sequence
<220>
   <223> Antigenic peptide from mouse uterine protease
<400> 52
   Ala Leu Gln Val Ser Gly Thr Pro Val Arg Gln Cys
   1 5 10
<210> 53
   <211> 13
   <212> PRT
   <213> artificial sequence
<220>
   <223> Antigenic peptide from region common to both isoforms of mouse ut erine protease
<400> 53
   Gly Pro Leu Val Asn Leu Asp Gly Glu Val Ile Gly Cys
   1 5 10
<210> 54
   <211> 13
   <212> PRT

   <213> artificial sequence
<220>
   <223> Antigenic peptide from HtrA
<400> 54
   Ile Ser Ile Asn Gly Gln Ser Val Val Thr Ala Asn Cys
   1 5 10
<210> 55
   <211> 457
   <212> DNA
   <213> Homo sapiens
<400> 55
   gcggttctgg cttcatcatg tcagaggccg gcctgatcat caccaatgcc cacgtggtgt 60
   ccagcaacag tgctgccccg ggcaggcagc agctcaaggt gcagctacag aatggggact 120
   cctatgaggc caccatcaaa gacatcgaca agaagtcgga cattgccacc atcaagatcc 180
   atcccaagaa aaagctccct gtgttgttgc tgggtcactc ggccgacctg cggcctgggg 240
   agtttgtggt ggccatcggc agtcccttcg ccctacagaa cacagtgaca acgggcatcg 300
   tcagcactgc ccagcgggag ggcagggagc tgggcctccg ggactccgac atggactaca 360
   tccagacgga tgccatcatc aactacggga actccggggg accactggtg aacctggatg 420
   gcgaggtcat tggcatcaac acgctcaagg tcacggc 457

## Description

This invention relates to methods relating to an enzyme which is predicted to be a serine protease, and in particular to this enzyme which is specifically expressed in association with embryo implantation and placentation in pregnant uterus. The enzyme is useful in the evaluation of fertility and monitoring of early pregnancy, fetal development, placental development and function, parturition, and conditions such as pre-eclampsia, intrauterine growth restriction (IUGR), early abortion, abnormal uterine bleeding, endometriosis, and cancers, and may provide a potential target for contraception. It may also be important in diseases of the heart, testis or ovary, and may play a role in muscle function, including cardiac muscle, skeletal muscle, lung and the diaphragm. The enzyme of the invention is useful in the screening of candidate drugs for fertility control or for treatment of the above disorders.

### BACKGROUND OF THE INVENTION

No admission is made that any reference cited herein constitutes prior art. The discussion of the references states what their authors assert, and the applicants reserve the right to challenge the accuracy and pertinency of the cited documents. It will be clearly understood that, although a number of prior art publications are referred to herein, this reference does not constitute an admission that any of these documents forms part of the common general knowledge in the art, in Australia or in any other country.

Embryo implantation, the process by which the blastocyst attaches and implants in the uterus, leads to the establishment of an intimate relationship between the embryo and the endometrium. Implantation is one of the most important limiting factors in establishing a successful pregnancy. It is a complex process involving active interactions between the blastocyst and the uterus. The uterus must undergo dramatic morphological and physiological changes to transform itself from a non-receptive to a receptive state. This differentiation process is largely mediated by the coordinated effects of the ovarian hormones, which act through their intracellular receptors to regulate gene expression, and hence to influence cellular proliferation and differentiation. It is also regulated by the blastocyst.

While the details of the exact molecular events occurring in the uterus during this differentiation process towards receptivity are still unknown, in principle it can be predicted that a unique set of genes is up- or downregulated in a temporally and spatially specific manner. Indeed, induction of specific genes in the uterus during the peri-implantation period, including those encoding some growth factors and cytokines, has been reported (Huet-Hudson *et al.*, 1990; Stewart *et al.*, 1992; Robb *et al.*, 1998; Zhu *et al.*, 1998; Das *et al.*, 1999). However, given the complexity and the as-yet imprecisely defined molecular mechanism of the process, many other molecules critical for implantation are still unidentified.

We have used the mouse as a model in a search for hitherto unrecognised molecules which are important in the early stage of implantation. In the mouse on day 4.5 of pregnancy (vaginal plug = day 0), the uterus undergoes dramatic morphological changes in association with cell proliferation and differentiation, leading to the acquisition of a receptive state (Abrahamsohn and Zorn, 1993). This uterine remodelling is associated with an increase in vascular permeability at implantation sites (Psychoyos, 1973). We hypothesised that the proliferation and differentiation of endometrial cells at this time is associated with up- or down-regulation of a number of genes, many of which are still unknown (Nie *et al.*, 1997). To identify uterine genes which are potentially critical for uterine receptivity, we used the technique of RNA differential display (DDPCR) (Liang and Pardee, 1992; Liang and Pardee, 1993) and compared the mRNA expression patterns of implantation and interimplantation sites on day 4.5 of pregnancy (Nie *et al.*, 2000a; Nie *et al.*, 2000b).

One of the mRNA molecules identified as being differently regulated between the two sites was found to encode a protein molecule, with a predicted serine protease motif (Zumbrunn & Traub, 1996). We isolated the cDNA encoding this protein, and examined its uterine expression during early pregnancy in the mouse; the protein is up-regulated in the pregnant mouse uterus from day 4.5 and further increased in the implantation site (including the maternal deciduum and the fetus and the placenta) from day 8.5 onwards. The observed expression pattern indicated a role for this protein in implantation, placentation and early pregnancy.

We have also identified and isolated the cDNA encoding the corresponding human enzyme, and found that this encodes a protein with a predicted serine protease motif, which is expressed in endometrium, decidua and placenta, and also in ovary, heart, and certain other tissues.

### SUMMARY OF THE INVENTION

In a first aspect the specification discloses an isolated nucleic acid molecule which
(a) is expressed in endometrium and placenta;
(b) is up-regulated in pregnant uterus and highly expressed during placental development; and
(c) encodes a protein which comprises a serine protease site and has an insulin-like growth factor (IGF)-binding motif.

Preferably the protein comprises the serine protease active site sequence GNSGGPL (SEQ ID NO:29); more preferably the protein also comprises the sequence TNAHV (SEQ ID NO:30) in the vicinity of the serine protease active site.

It will be appreciated that although the nucleic acid molecule disclosed herein encodes a protein which has serine protease activity and the ability to bind IGF, it may also have other activities which are significant for biological functions.

The nucleic acid molecule may be a cDNA, a genomic DNA, or an RNA, and may be in the sense or the anti-sense orientation. Preferably the nucleic acid molecule is a cDNA.

Preferably the nucleic acid molecule has a sequence selected from the group consisting of
(a) a cDNA molecule having the sequence set out in Figure 2 (SEQ ID NO:26), Figure 3A (SEQ ID NO:31), Figure 3B (SEQ ID NO:32), or Figure 6A (SEQ ID NO:38);
(b) a nucleic acid molecule which is able to hybridize under at least moderately stringent conditions to the molecule of (a); and
(c) a nucleic acid molecule which has at least 75% sequence identity to the molecule of (a).

More preferably in (b) the nucleic acid molecule is able to hybridize under stringent conditions to the molecule of (a). More preferably in (c) the nucleic acid molecule has at least 80%, even more preferably at least 90% sequence identity to the molecule of (a).

In a second aspect the specification discloses a protein having serine proteases enzymic activity and an IGF-binding motif, which is encoded by the nucleic acid molecule of the invention. This protein is referred to herein as pregnancy-related serine protease (PRSP).

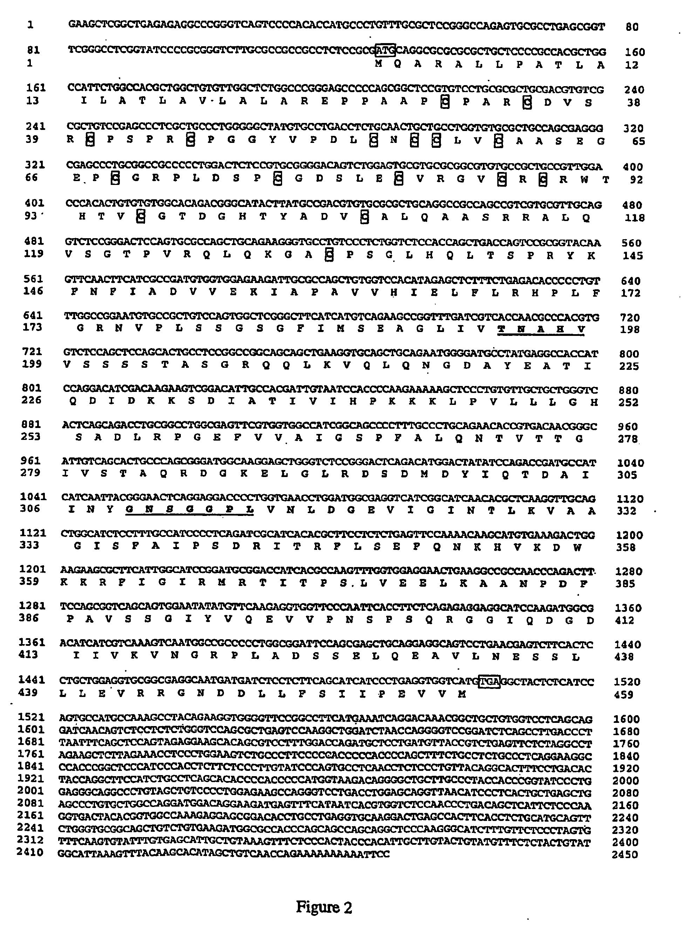

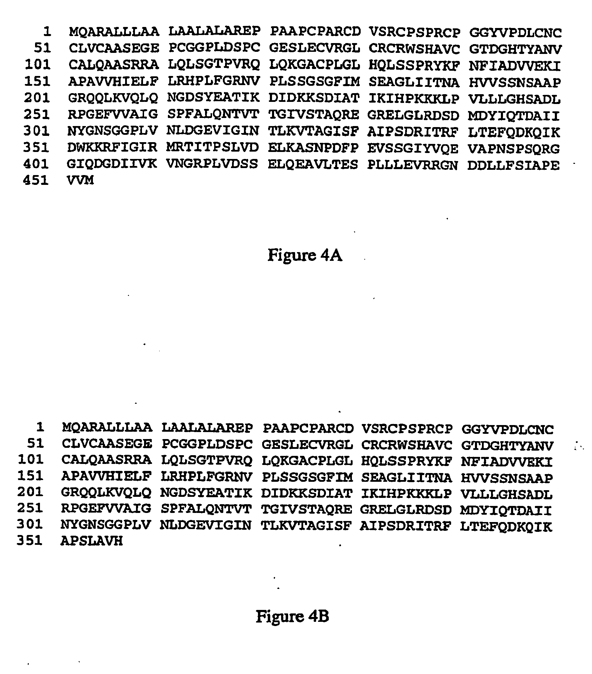

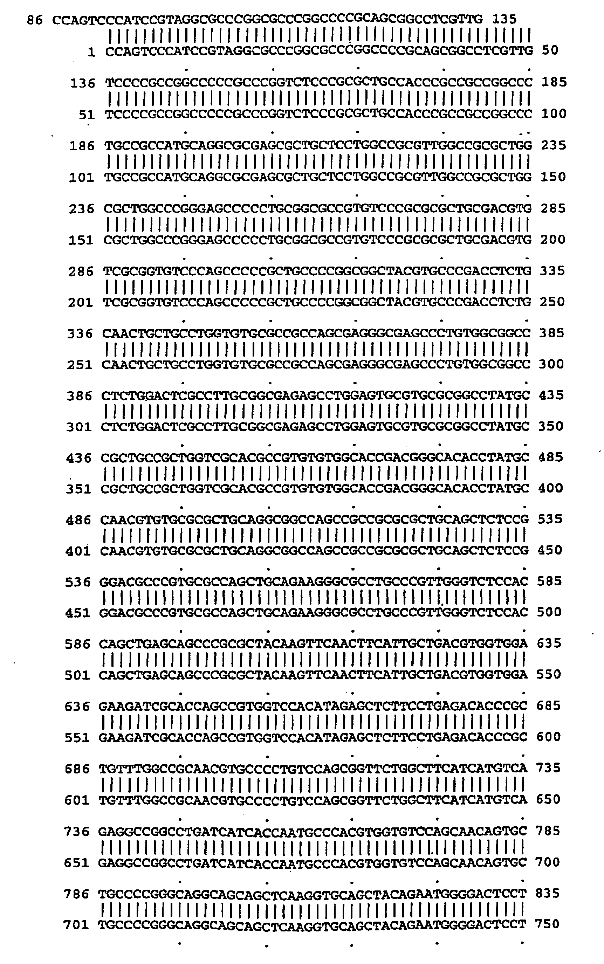

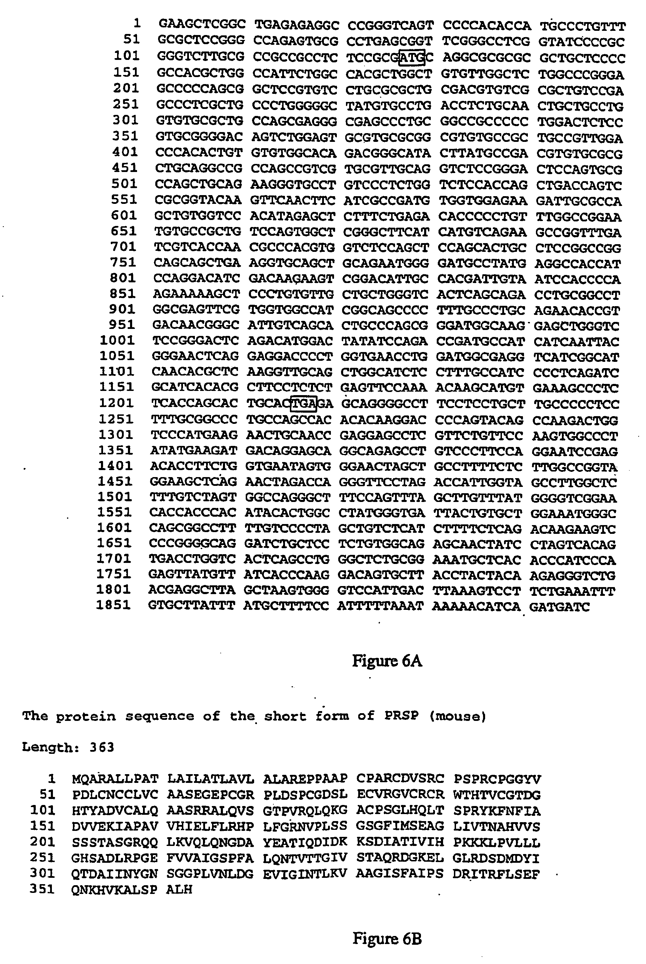

Preferably the protein has a sequence selected from the group consisting of the sequences set out in Figure 2 (SEQ ID NO:27), Figure 6B (SEQ ID NO:39), Figure 4A (SEQ ID NO:33), or Figure 4B (SEQ ID NO:34); more preferably the sequence is the one set out in Figure 4A (SEQ ID NO:33) or Figure 4B (SEQ ID NO:34).

As used herein, the terms "functionally active" and "functional activity" in reference to PRSP mean that the PRSP is able to act as a serine protease and/or to bind IGF, and/or that the PRSP is immunologically cross-reactive with an antibody directed against an epitope of a naturally-occurring PRSP of the invention. It will be appreciated that PRSP may also have other biological functions in addition to those specifically mentioned herein.

Therefore PRSP amino acid sequence variants will generally share at least about 75%, preferably greater than 80%, and more preferably greater than 90% sequence identity with one or more of the deduced amino acid sequences set out in in Figure 2 (SEQ ID NO:27), Figure 6B (SEQ ID NO:39), Figure 4A (SEQ ID NO:33), or Figure 4B (SEQ ID NO:34), after aligning the sequences to provide for maximum homology, for example as determined by the version described by Fitch *et al.*, (1983), of the algorithm described by Needleman *et al.*, (1970).

In a third aspect the specification discloses a composition comprising a nucleic acid molecule according to the invention, together with a pharmaceutically acceptable carrier.

In a fourth aspect the specification discloses a composition comprising a protein according to the invention, together with a pharmaceutically acceptable carrier.

In a fifth aspect the specification discloses a probe for detection of nucleic acid encoding PRSP, comprising at least 15, preferably at least 20, more preferably at least 30 consecutive nucleotides from the nucleic acid molecule of the invention. In a particularly preferred embodiment the probe encompasses at least part of the common region of the two isoforms disclosed herein for mouse PRSP (SEQ ID NO:40), or human PRSP (nucleotides 1-1243 of the long form sequence shown in SEQ ID NO:31).

Thus the specification discloses a method of detecting, diagnosing, or monitoring a condition which involves a change in PRSP expression, comprising the step of using a nucleic acid molecule disclosed herein , or a fragment thereof comprising at least about 15 nucleotides, as a probe in a hybridization assay performed on a biological sample from a mammal suspected to be suffering from such a condition. The sample may be a sample of a biological fluid such as plasma, serum, uterine or bladder washings, or amniotic fluid, or may be a tissue or cell sample or an extract thereof. Such conditions include infertility caused by inability to achieve or sustain embryo implantation or to sustain pregnancy, in which the assay is performed on a sample. In one embodiment , total RNA in a sample of placental or uterine tissue from the mammal is assayed for the presence of PRSP messenger RNA, wherein an alteration in the amount of PRSP messenger RNA is indicative of impaired fertility or of impending miscarriage.

It will be appreciated that probes disclosed herein may be used to identify genetic polymorphisms which are indicative of predisposition or susceptibility to PSRP-related conditions. Such conditions include but are not limited to pre-eclampsia, intrauterine growth restriction (IUGR), early abortion, abnormal uterine bleeding, endometriosis, cancers, and diseases of the heart, testis or ovaries.

In a sixth aspect the specification discloses an antibody directed against PRSP. The antibody may be polyclonal or monoclonal, and is preferably monoclonal. The antibody may suitably be directed against one of the following segments of the mouse protease:
1. Amino acids 133-142; sequence PSGLHQLTSPC. (SEQ ID NO:51).
2. Amino acids 116-126; sequence ALQVSGTPVRQC (SEQ ID NO:52).
3. A sequence common to both isoforms, represented by amino acids 133-142 of SEQ ID NO:26; sequence GPLVNLDGEVIGC (SEQ ID NO:53).

These mouse sequences are highly homologous to corresponding regions of the human protein.

More preferably the antibody is directed to an epitope within the common region of the two isoforms disclosed herein for mouse or human PRSP. In one particularly preferred embodiment the antibody has the ability to inhibit the serine protease activity and/or the IGF-binding activity of the PRSP. The antibody may also be used to detect the PRSP in biological fluids, washings from hollow viscera such as the uterus or bladder, or in tissues, cells or extracts thereof.

In a seventh aspect the specification discloses a method of screening for compounds which have the ability to modulate the activity of PRSP, comprising the step of assessing the ability of a candidate compounds to increase or decrease
(a) the serine protease activity and/or
(b) the IGF-binding activity of PRSP.

It will be appreciated that modulation of PRSP activity may be detected inter alia by monitoring the effects of the candidate compound on levels of a substrate for the enzyme, or on a cellular activity of PRSP. The substrate assay may utilise synthetic substrates, and suitable substrates are well known in the art. Assays for cellular activity may utilise cell lines which have been transfected with nucleic acid encoding PSRP so as to over express this protein; such transformed cell lines are particularly useful for phenotypic assays of biological function.

Thus the specification discloses a method of identifying agonists and antagonists of PRSP. In view of the crucial role of PRSP in implantation and in formation of the placenta indicated by the results reported herein, it is contemplated that antagonists of PRSP will be useful as contraceptives, and that agonists of PRSP will be useful as agents for promoting fertility or for supporting at least the early phases of pregnancy. It is further contemplated that antagonists of PRSP include, but are not limited to, antibodies and anti-sense nucleic acids.

In a further aspect, the invention provides an in vitro method of detecting, diagnosing, or monitoring a condition in a mammal which involves a change in PRSP expression, which condition is infertility caused by inability to achieve or sustain embryo implantation or to sustain pregnancy, the method comprising the step of measuring the amount or activity of a PRSP protein in a biological sample from said mammal suffering from or at risk of such a condition, in which the PRSP protein comprises the sequence provided as SEQ ID NO: 27, 39, 33 or 34. Any suitable biological sample may be used, for example a tissue or cell sample or extract, or a sample of a biological fluid, such as plasma, serum or amniotic fluid, or uterine or bladder washings. For example, the probes disclosed herein may be used to diagnose impaired fertility or impending miscarriage, as described above. The antibodies disclosed herein are expected to be particular useful for detecting PRSP in biological fluids such as plasma, serum or amniotic fluid, or in uterine or bladder washings.

The mammal may be a human, or may be a domestic or companion animal. While it is particularly contemplated that the compounds disclosed herein are suitable for use in medical treatment of humans, they are also applicable to veterinary treatment, including treatment of companion animals such as dogs and cats, and domestic animals such as horses, cattle and sheep, zoo animals such as non-human primates, felids, canids, bovids, and ungulates, or for the control of pest or feral species such as rabbits, rats and mice.

Methods and pharmaceutical carriers for preparation of pharmaceutical compositions are well known in the art, as set out in textbooks such as Remington's Pharmaceutical Sciences, 20th Edition, Williams & Wilkins, Pennsylvania, USA.

The compounds and compositions disclosed herein may be administered by any suitable route, and the person skilled in the art will readily be able to determine the most suitable route and dose for the condition to be treated. Dosage will be at the discretion of the attendant physician or veterinarian, and will depend on the nature and state of the condition to be treated, the age and general state of health of the subject to be treated, the route of administration, and any previous treatment which may have been administered.

The carrier or diluent, and other excipients, will depend on the route of administration, and again the person skilled in the art will readily be able to determine the most suitable formulation for each particular case.

For the purposes of this specification it will be clearly understood that the word "comprising" means "including but not limited to", and that the word "comprises" has a corresponding meaning.

### BRIEF DESCRIPTION OF THE FIGURES

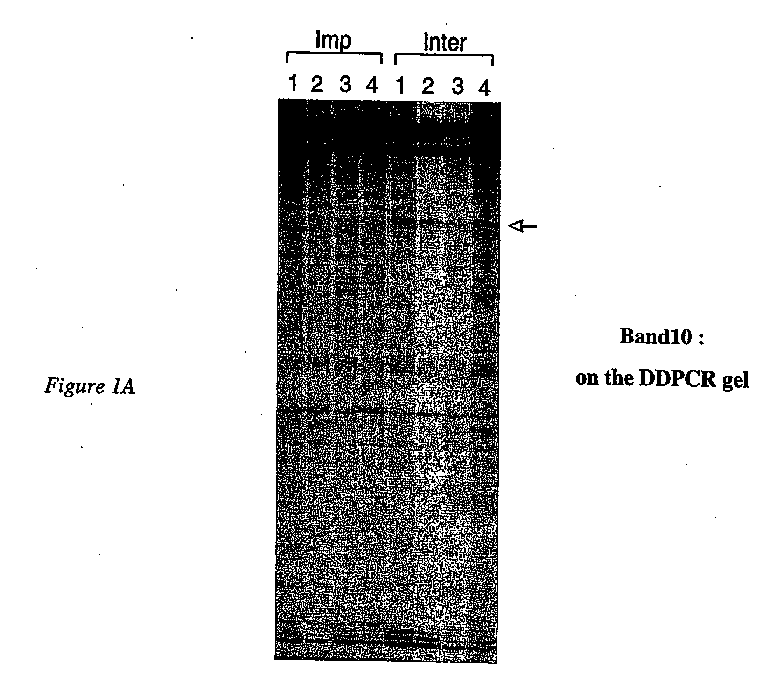

Figure 1A illustrates the results of RNA differential display analysis (DDPCR) of pregnant mouse uterus. The expression pattern of band 10 (identified to be PRSP) on the DDPCR gel is indicated by the arrow, showing much stronger intensities in interimplantation sites (Inter) compared to implantation sites (Imp) in four different mice: lane 1, animal 1; lane 2, animal 2, lane 3, animal 3 and lane 4, animal 4.

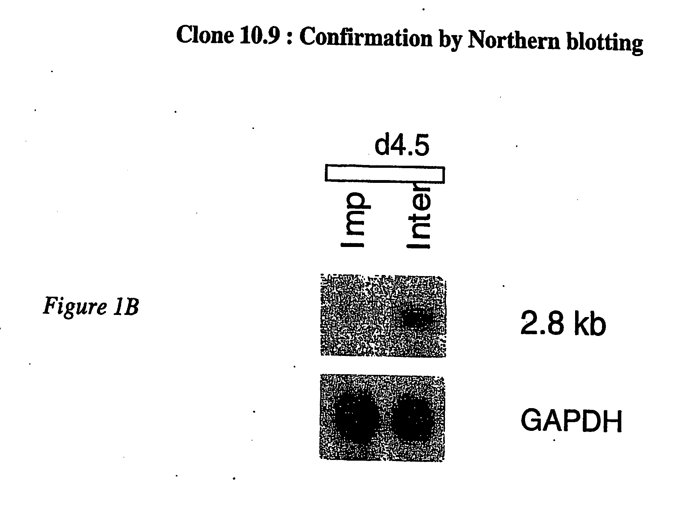

Figure 1B shows the results of Northern blot analysis of mRNA detected using the cDNA extracted from band 10 of the DDPCR gel as a probe. Total RNA (15 µg) was isolated from implantation (Imp) and interimplantation (Inter) sites of day 4.5 pregnant mice. The top panel shows the 2.8 kb band detected for this gene; the lower panel shows the signal detected by the GAPDH probe on the same membrane as in the top panel.
Figure 2 shows the full length cDNA sequence (SEQ ID No. 26) and predicted amino acid sequence (SEQ ID No. 27) of the longer isoform of the novel protein from mouse uterus. The ATG start codon and TGA stop codon are boxed. The 16 cysteine residues are shown in bold and boxed, and the serine protease active site residues GNSCGPL and the additional histidine site residues TNAHV are shown underlined and in bold.

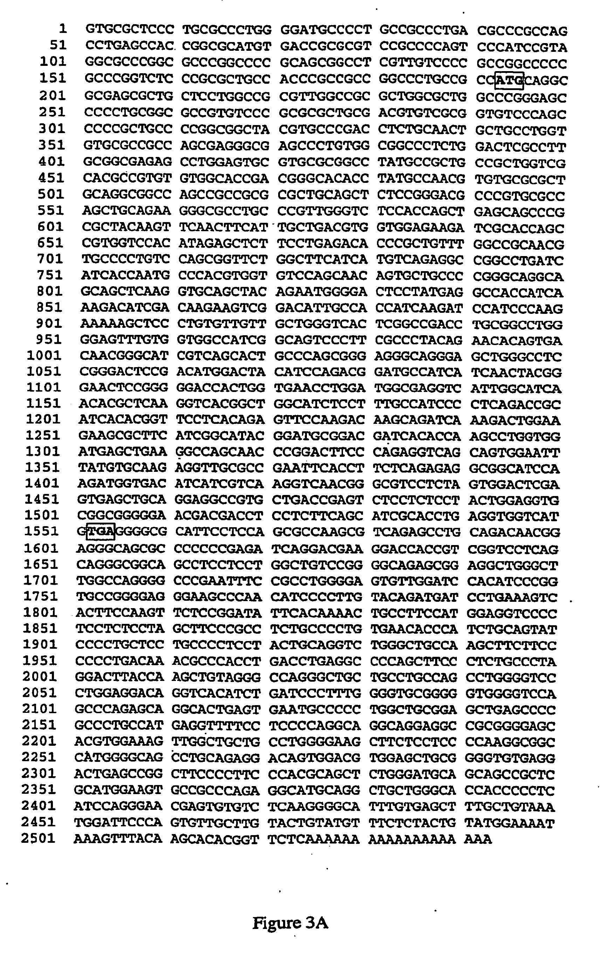

Figure 3A shows the cDNA sequence of the long isofor encoding the human protease (SEQ ID NO: 31; 2543 bp); the start and stop codons are indicated by the box.

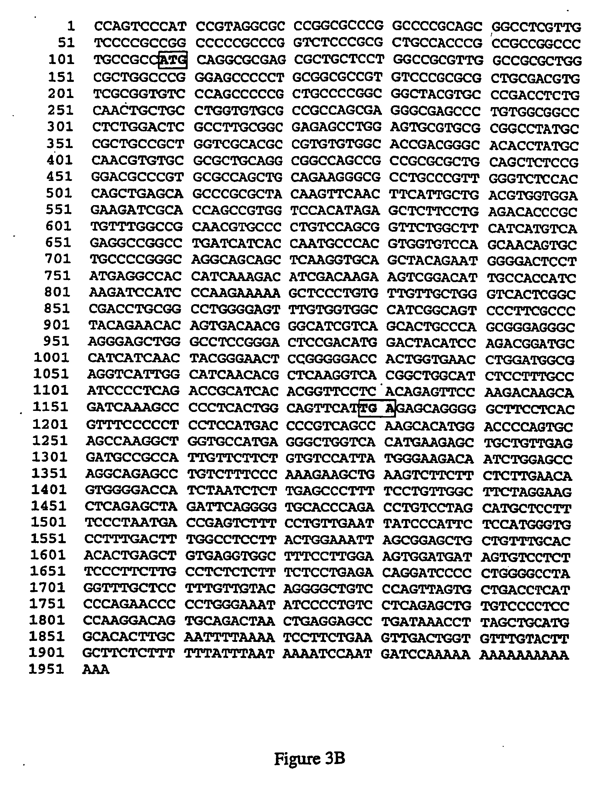

Figure 3B shows the cDNA sequence of the short isoform encoding the human protease (SEQ ID NO: 32; 1953 bp); the start and stop codons are indicated by the box.
Figure 4A shows the deduced amino acid sequence of the long isoform of the human protease (SEQ ID NO: 33; 453 amino acids).
Figure 4B shows the deduced amino acid sequence of the short isoform of the human protease (SEQ ID NO: 34; 357 amino acids).

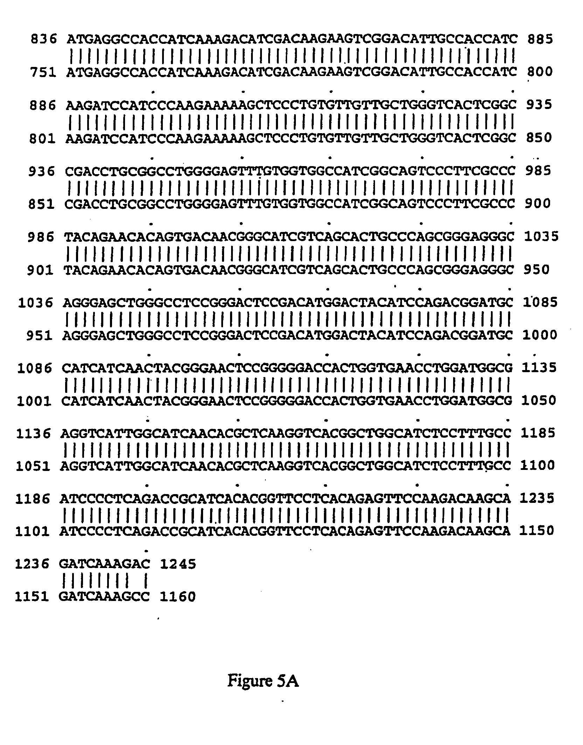

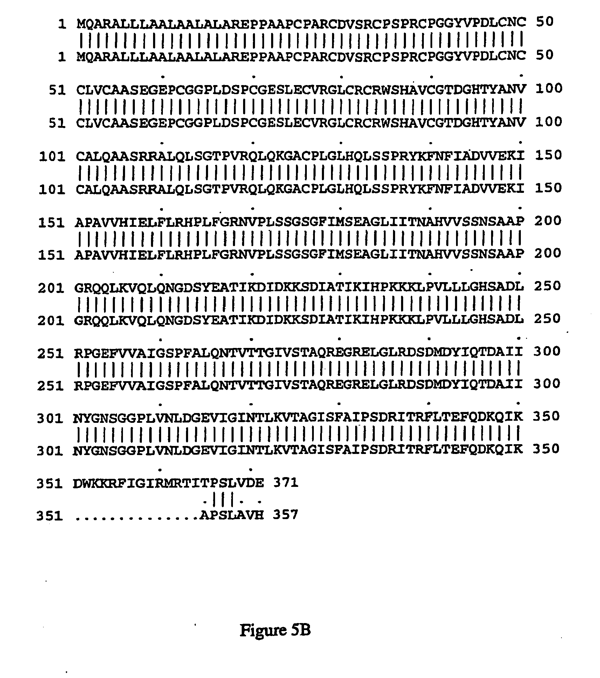

Figure 5A and Figure 5B respectively show the comparison between the cDNA and protein sequences of the two isoforms of the human enzyme. The top sequence represents the long isoform, and the bottom sequence represents the short isoform.
Figure 6A shows the full length cDNA sequence (SEQ ID NO:37) encoding the short isoform of the novel protein from mouse uterus. The ATG start codon and TGA stop codon are indicated by boxes.
Figure 6B shows the deduced amino acid sequence of the short isoform of the mouse protease (SEQ ID NO:38; 363 amino acids).

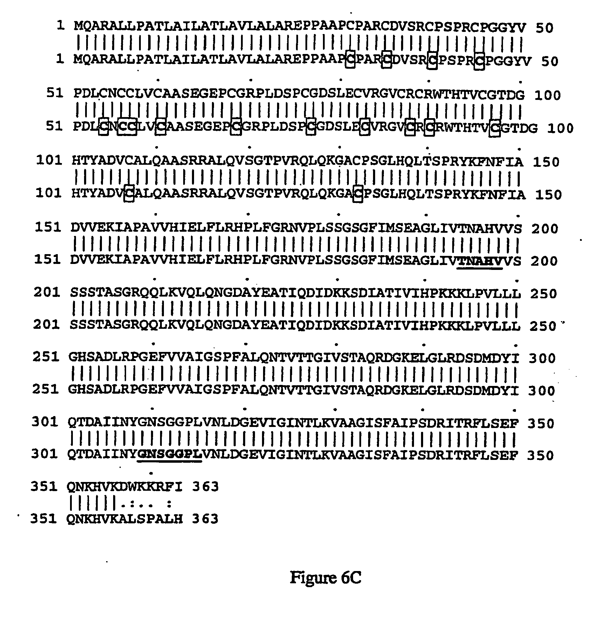

Figure 6C shows a comparison between the deduced protein sequences of the longer (top) and shorter (bottom) isoforms of the mouse enzyme. The 16 cysteine residues are shown in bold and boxed, and the serine protease active site residues GNSGGPL and the additional histidine site residues TNAHV are shown underlined and in bold.

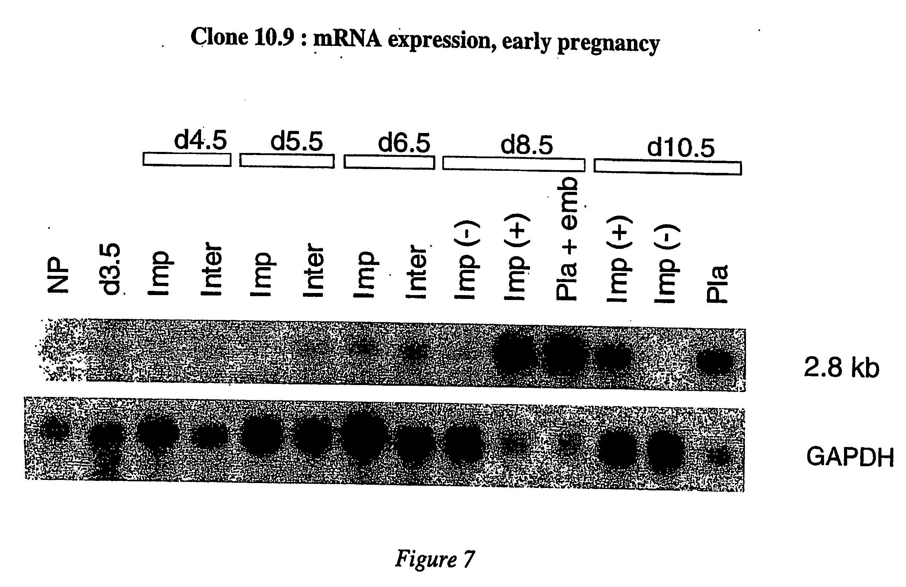

Figure 7 shows the results of Northern blot analysis of the novel gene in the mouse uterus during early pregnancy. A 785 bp cDNA sequence (nt 76-860 of the longer cDNA shown in Figure 2), representing the common region of the two isoforms, was used as a probe. Total RNA (15 µg) was isolated from whole uterus of non-pregnant mice at estrus (NP) and from whole uterus of 3.5 day pregnant (d3.5) mice, and from implantation sites (Imp) and interimplantation sites (Inter) of uterus on days (d) 4.5, 5.5, 6.5, 8.5 and 10.5 of pregnancy (day 0 = day of vaginal plug). On days 8.5 and 10.5, three types of tissue were sampled: (1) the entire implantation unit containing the uterine implantation site, the deciduum, embryo and the developing placenta [Imp (+)], (2) uterine implantation site tissue without the deciduum, embryo and placenta [Imp (-)], and (3) embryo and placenta sampled together (Emb +Pl) on day 8.5, and placenta (Pla) only on day 10.5. The top panel shows the main 2.8 kb transcript detected for this gene, and the lower panel shows the signal detected by the GAPDH probe on the same membrane.

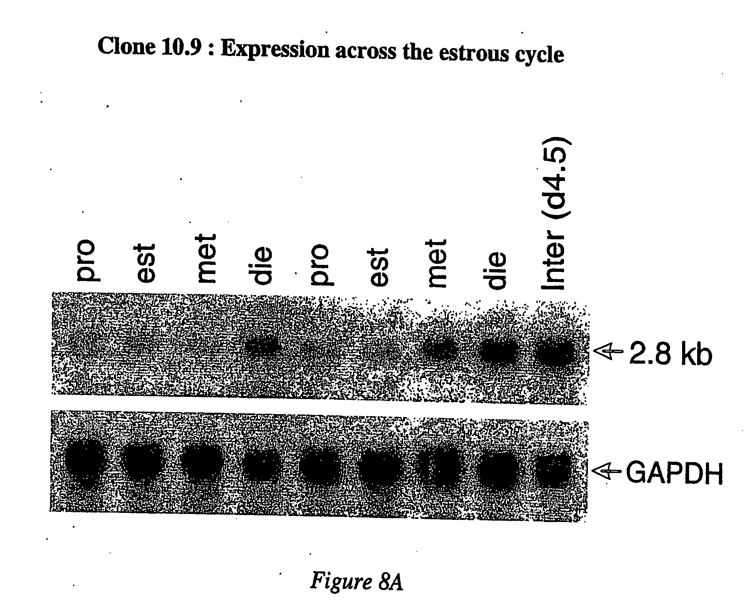

Figure 8A shows the results of Northern blot analysis of total RNA (15 µg) isolated from whole uterus of non-pregnant mouse at metestrus (met), diestrus (die), proestrus (pro) and estrus (est). Two cycles are shown. A 785 bp cDNA sequence (nt 76-860 of the longer cDNA shown in Figure 2), representing the common region of the two isoforms, was used as a probe. The top panel shows the main 2.8 kb transcript detected for this gene; the lower panel shows the signal detected by the GAPDH probe on the same membrane.

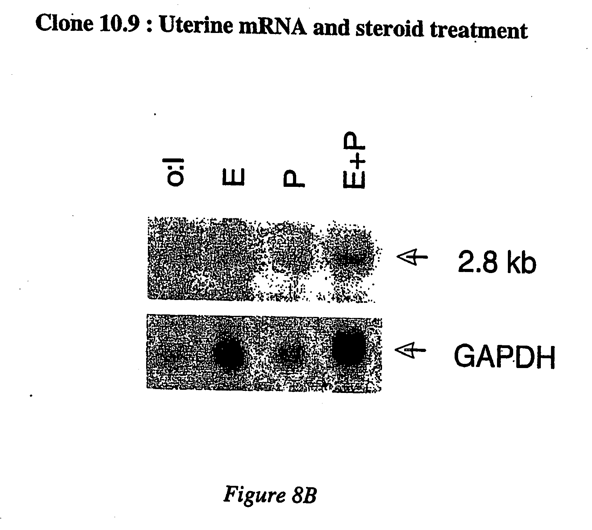

Figure 8B shows the results of Northern blot analysis of total RNA (15 µg) isolated from whole uterus of ovariectomized mice injected with vehicle (oil), 17β-estradiol (E), progesterone (P) or E and P (E+P). A 785 bp cDNA sequence (nt 76-860 of the longer cDNA shown in Figure 2), representing the common region of the isoforms, was used as a probe. The top panel shows the main 2.8 kb signal detected for this gene; the lower panel shows the signal detected by the GAPDH probe on the same membrane.

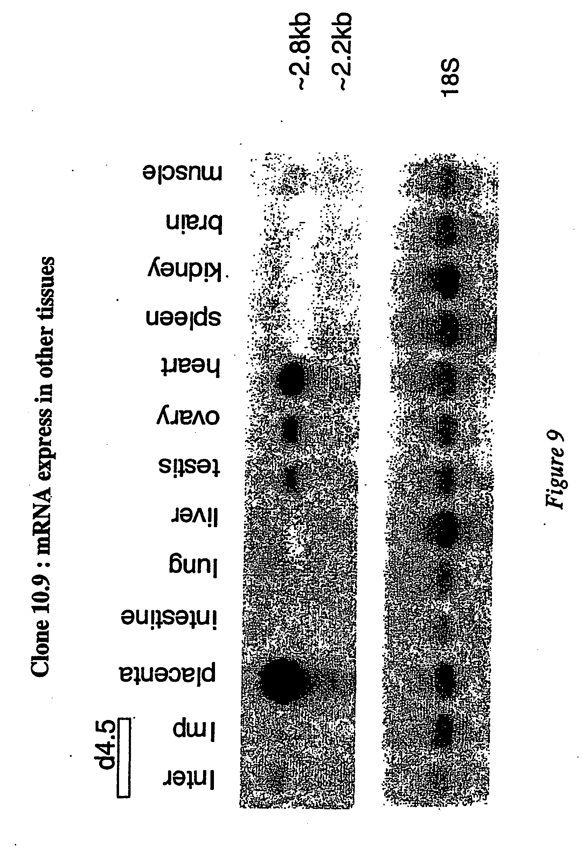

Figure 9 shows the results of Northern blot analysis of the tissue specificity of the novel gene from mouse. Total RNA (15 µg) was isolated from interimplantation (Inter) and implantation (Imp) sites on day 4.5 pregnancy, placenta on day 10.5, intestine, lung, liver, testis, ovary, heart, spleen, kidney, whole brain and muscle. A 785 bp cDNA sequence (nt 76-860 of the longer cDNA shown in Figure 2) representing the common region of the isoforms was used as a probe. The top panel shows the signals detected for this gene, and the lower panel shows the signal detected by ribosomal 18s RNA probe on the same membrane.

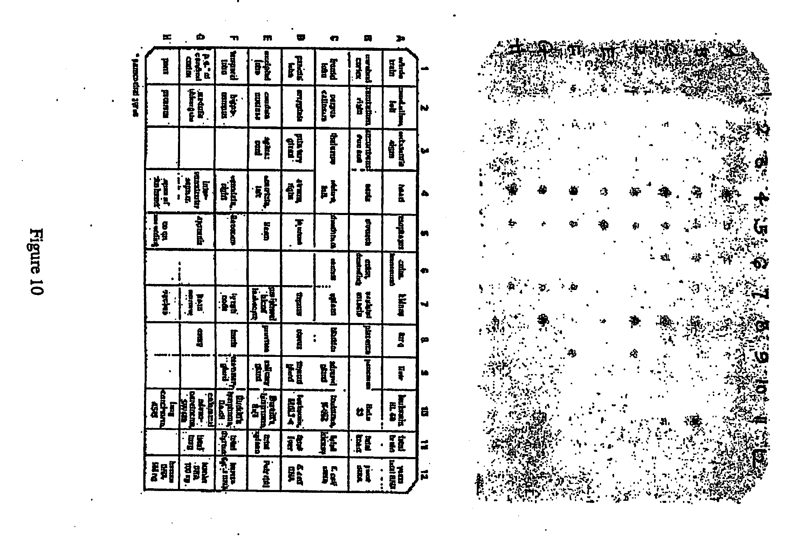

Figure 10 shows the results of probing a human multi-tissue expression array with the same 785bp PRSP cDNA probe as in Figure 7.

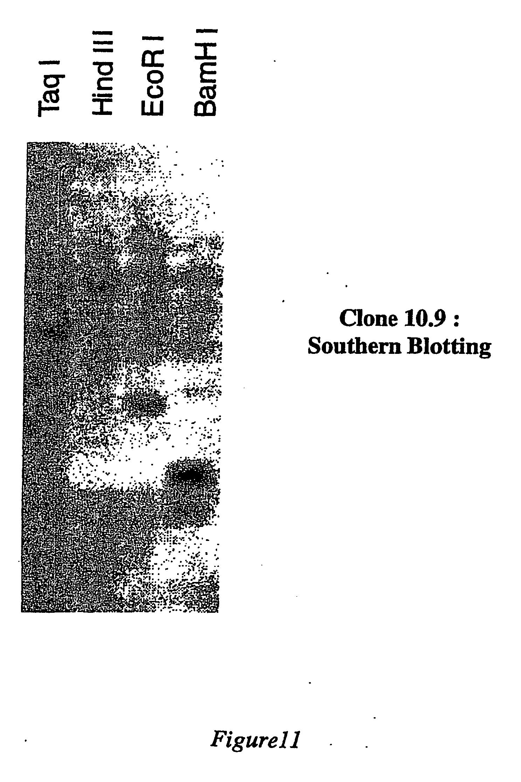

Figure 11 shows the results of Southern blot analysis of the novel gene in the mouse. Genomic DNA was isolated from non-pregnant mouse uterus, and 10 µg was digested with the following four restriction enzymes: TaqI, HindIII, EcoR1 and BamHI, and probed with a radio-labelled 785 bp cDNA sequence (nt 76-860 of the longer cDNA shown in Figure 2), representing the common region of the two isoforms.

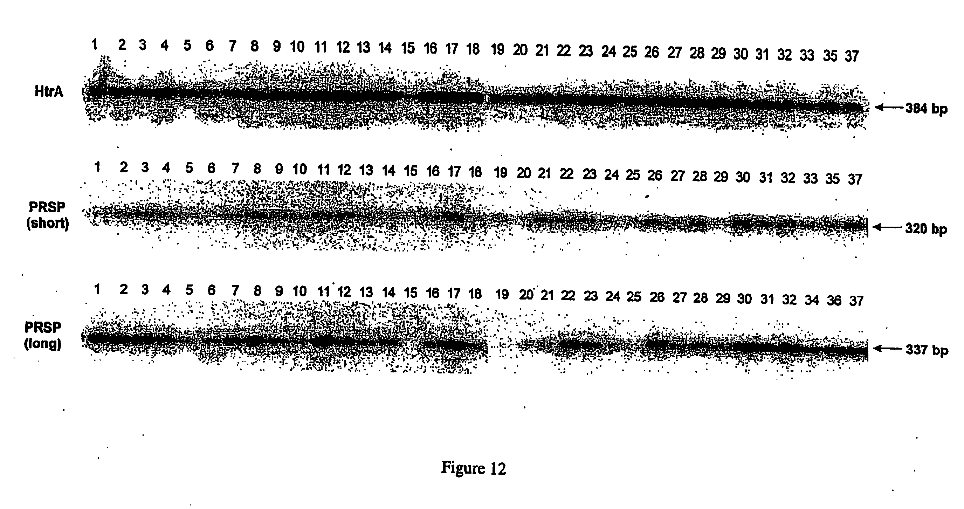

Figure 12 shows the results of semiquantitative reverse transcriptase polymerase chain reaction (RT-PCR) Southern blot analysis of HtrA (a related peptide) and PRSP (short and long forms) in cycling and pregnant human endometrium. Menstrual phase endometrium (lanes 1-3), early proliferative phase endometrium (lanes 4-7), mid-late proliferative phase endometrium (lanes 8-9), early secretory phase endometrium (lanes 10-13), mid-late secretory phase endometrium (lanes 14-18), premenstrual endometrium (lanes 19-22), first trimester decidua (lanes 23, 25, 27, 29, 31), first trimester placenta (lanes 24, 26, 28, 30, 32), term placenta (lane 34), pre-menopausal ovary (lane 35), post-menopausal ovary (lane 37), heart (lane 33), and skeletal muscle (lane 36).

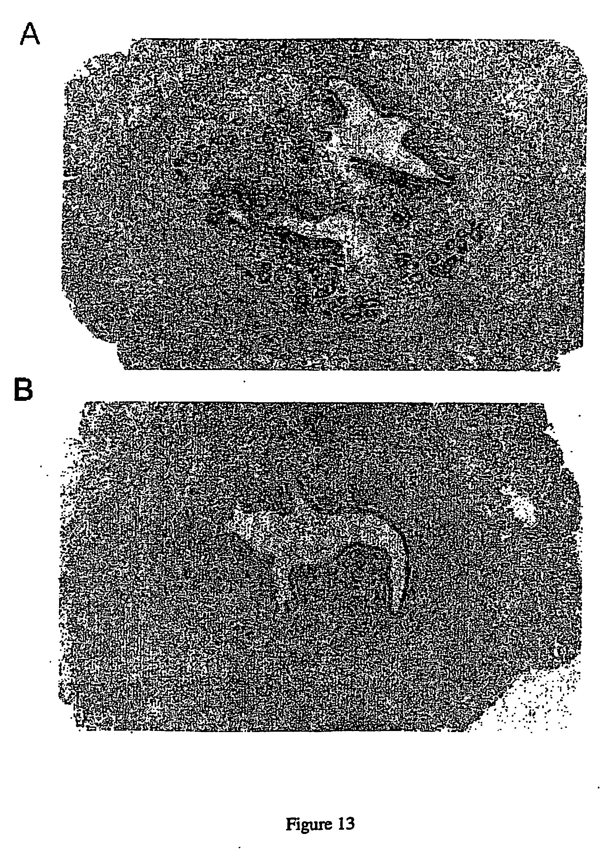

Figure 13 shows the results of *in situ* hybridization to detect the mRNA of the PRSP in sections of mouse uterus on day 5.5 of pregnancy (interimplantation site).

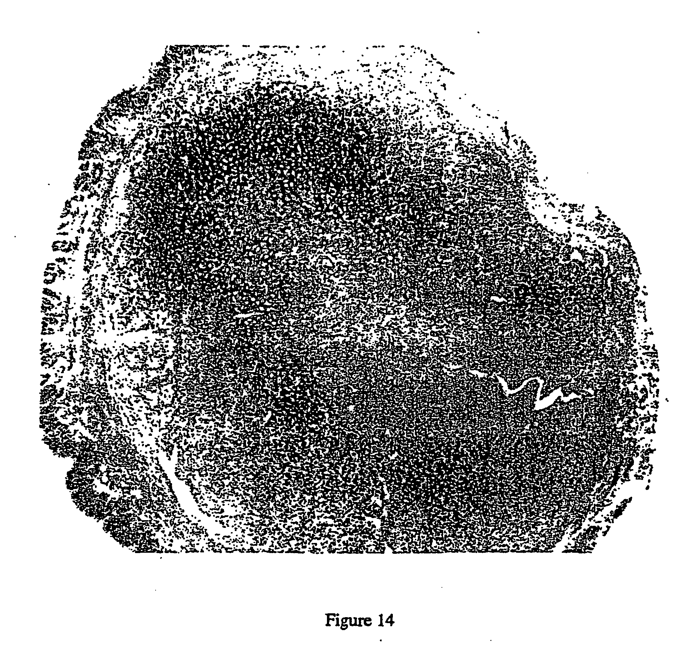

Figure 14 shows the result of *in situ* hybridization to detect PRSP mRNA in mouse uterus on day 5.5 of pregnancy (implantation site).

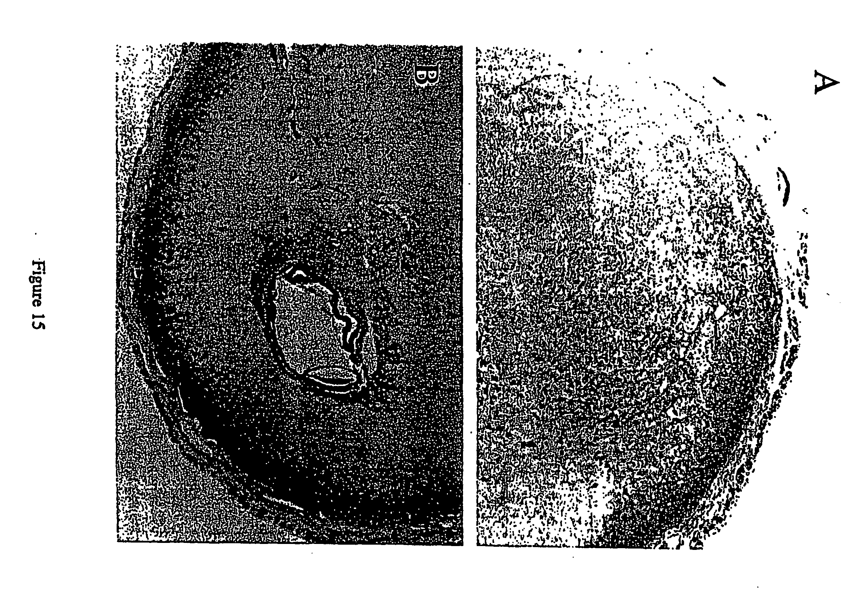

Figure 15 shows the result of *in situ* hybridization to detect PRSP mRNA in mouse uterus on day 8.5 of pregnancy. Figure 15A shows the decidual basalis at the mesimetrial side of the uterus showing the positive staining. Figure 15B shows the decidual capsularis and the fetus showing the positive staining.

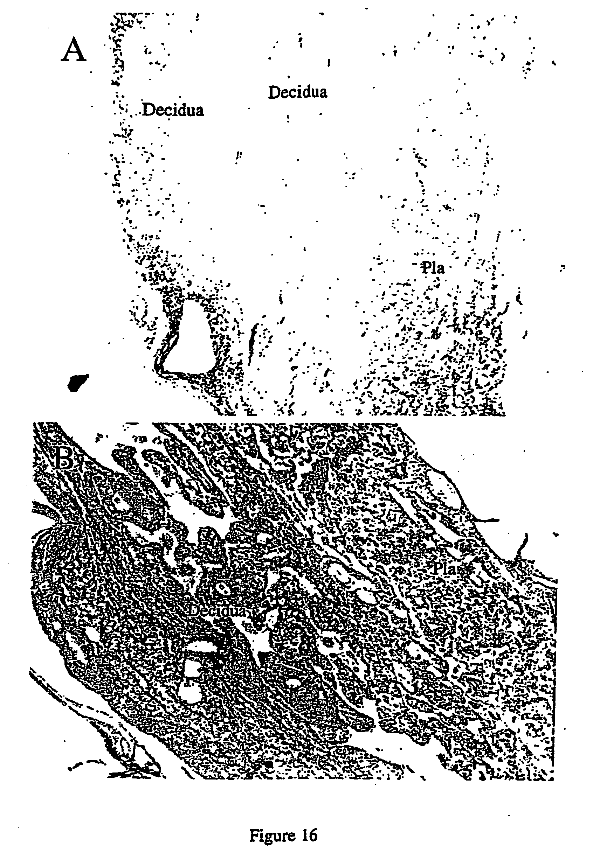

Figure 16A shows the result of *in situ* hybridization to detect PRSP mRNA in mouse uterus on day 10.5 of pregnancy, showing the positive staining in the placenta and part of the decidua close to the uterine wall. The part of the decidua close to the placenta is negative. Figure 16B shows the identification of decidual cells, using immunohistochemical analysis of desmin on the section shown in Figure 16A.

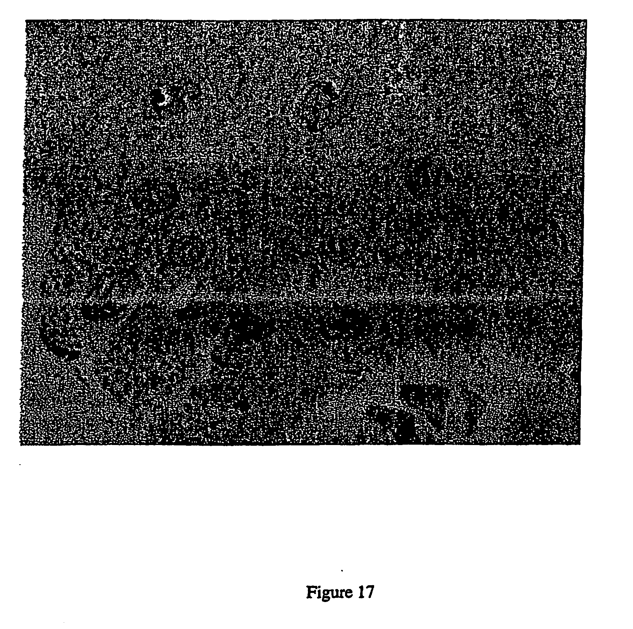

Figure 17 shows the result of *in situ* hybridization to detect PRSP mRNA in cycling human endometrium on day 9 of the menstrual cycle.

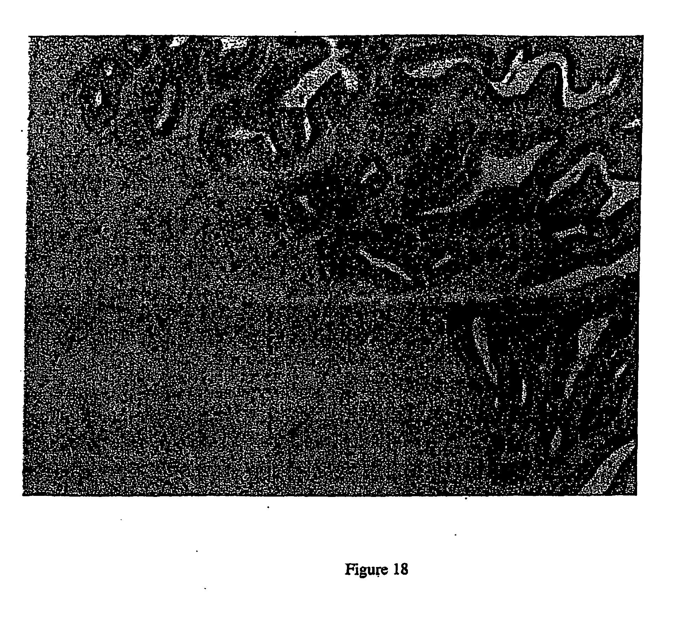

Figure 18 shows the result of *in situ* hybridization to detect PRSP mRNA in cycling rhesus monkey uterus on day 10 after ovulation.

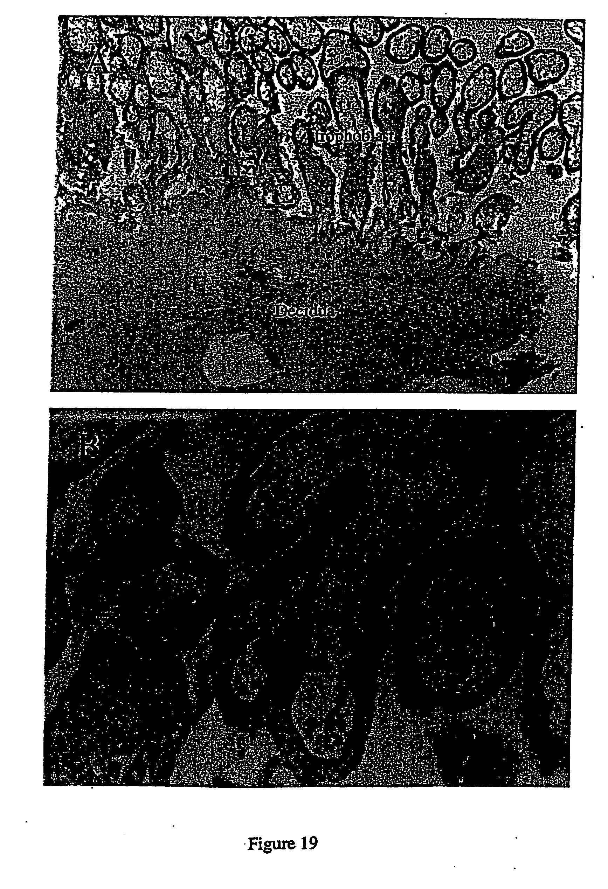

Figure 19 shows the result of *in situ* hybridization to detect PRSP mRNA in pregnant rhesus monkey uterus: the implantation site on day 28 of pregnancy is shown. Panel A shows the implantation site including the trophoblast cells and the maternal decidual cells. Note the positive staining in the trophoblast cells and the decidual cells. Panel B shows a high power view of trophoblast cells.

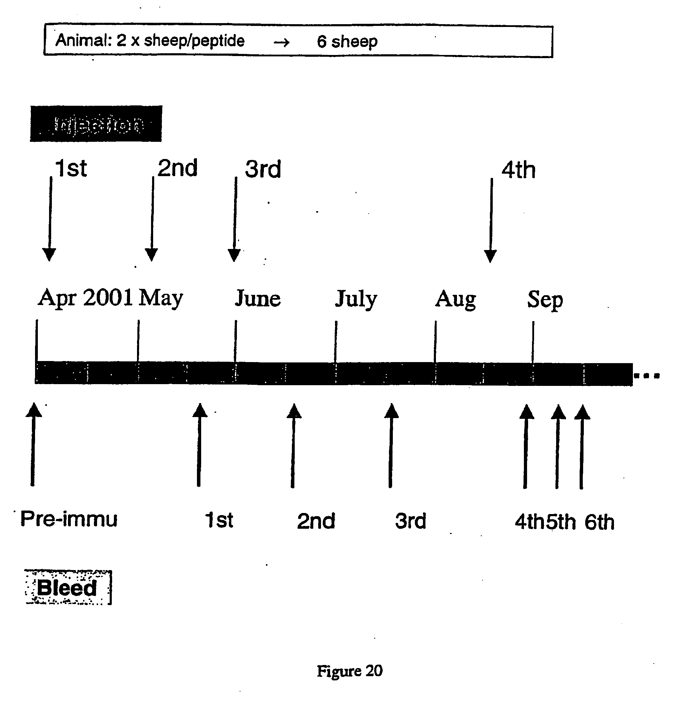

Figure 20 shows the scheme of antibody generation in the sheep against peptides of mouse PRSP protein.

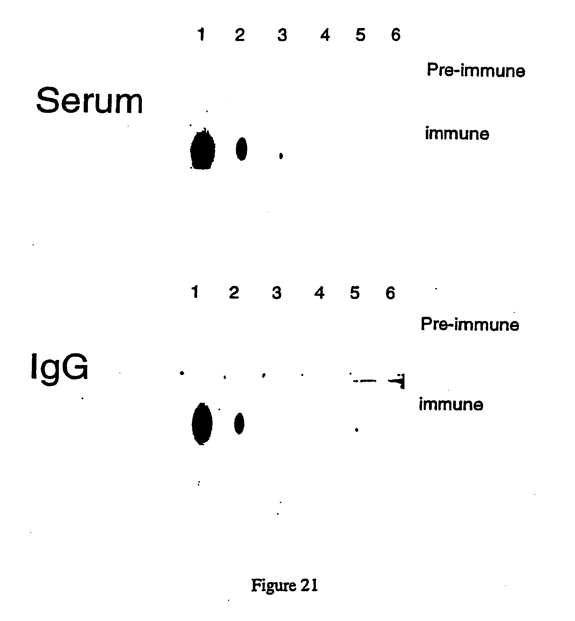

Figure 21 shows the detection of the antibody in the serum of immunized sheep and in IgG prepared from the serum by dot blot of peptides. The result for peptide (2), identified in Example 13, is shown. To show the specificity of the antisera, dots 1 to 4 contain serial dilutions of peptide (2) and dots 5 and 6 contain irrelevant peptides.

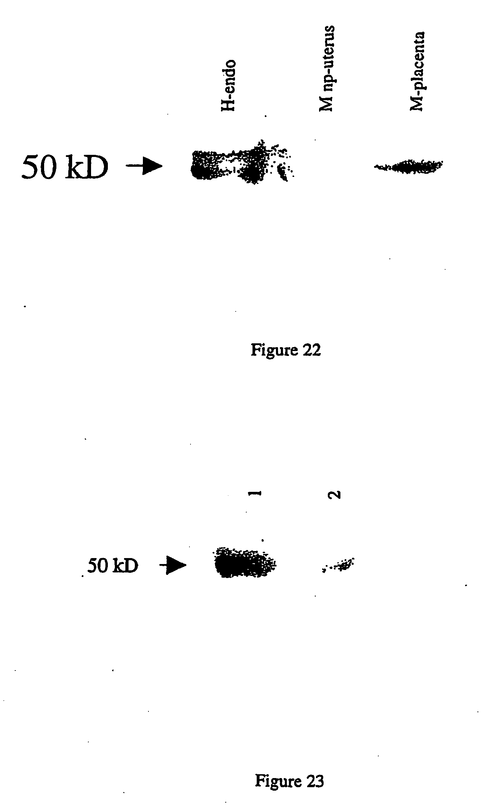

Figure 22 shows the result of western blot analysis of PRSP protein in the non-pregnant mouse uterus (M np-uterus), mouse placenta on day 10.5 of pregnancy (M-placenta) and human endometrium on day 25 of the menstrual cycle (H-endo), using the antibody against peptide (2).
Figure 23 shows the result of western blot analysis of PRSP protein in the serum of two pregnant women using the antibody against peptide (2).

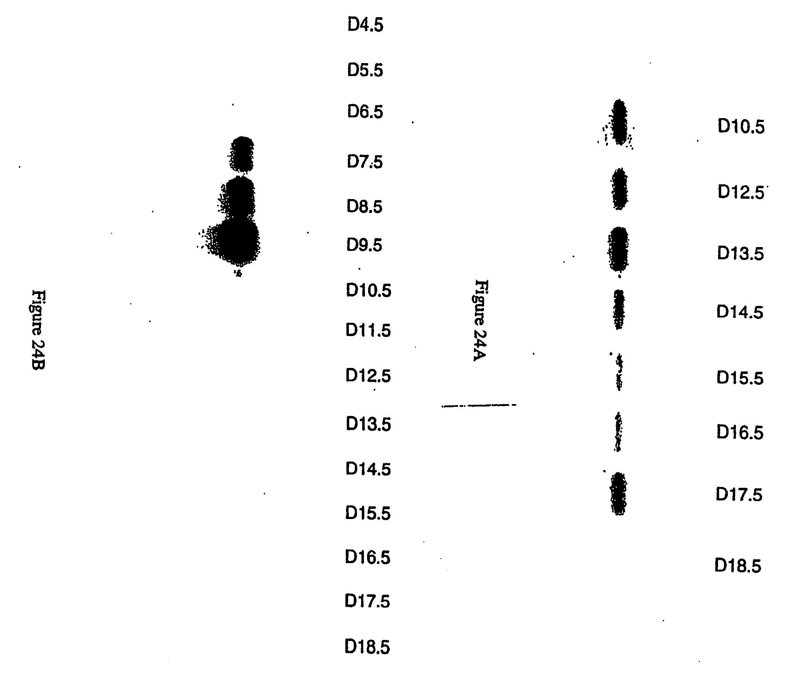

Figure 24 shows the result of Northern analysis of PRSP mRNA in the fetus, placenta and the uterus during placentation and later gestation in the mouse. (A) The expression of PRSP in the placenta from day 10.5 to 18.5 of pregnancy. (B) The expression of PRSP in the fetus from day 4.5 to 18.5 of pregnancy. On day (D) 4.5 and 5.5, the fetal sample includes the whole implantation site. On day 6.5, 7.5, 8.5 and 9.5, the fetal sample includes the fetus, its developing placenta and the maternal deciduum, a mass of uterine decidual cells enclosing a single embryo. From day 10.5 onwards, the fetal sample contains only the fetus.

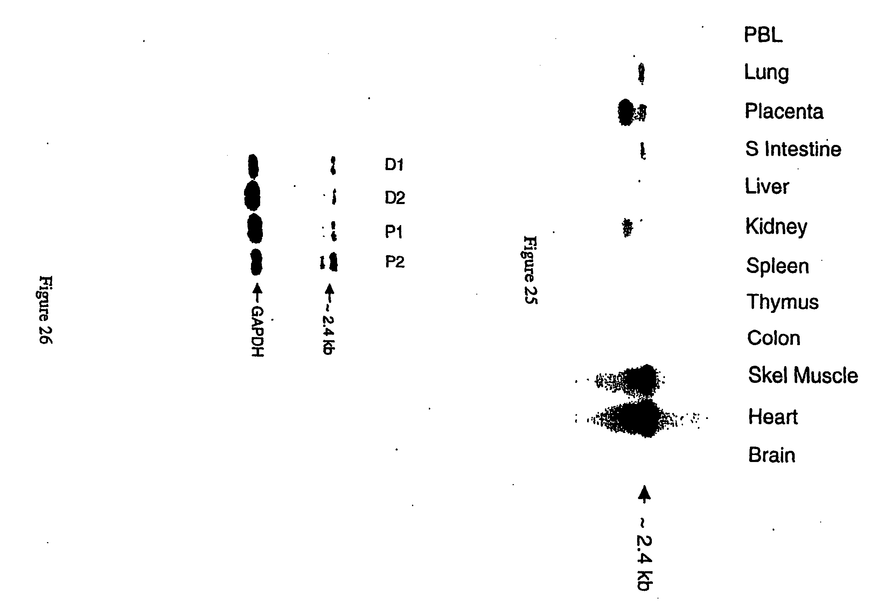

Figure 25 shows the result of Northern analysis of PRSP in a range of human tissues. PBL: peripheral blood leukocytes; S intestine: small intestine; Skel muscle: skeletal muscle.
Figure 26 shows the result of Northern analysis of PRSP in first trimester pregnant human decidua and placenta.

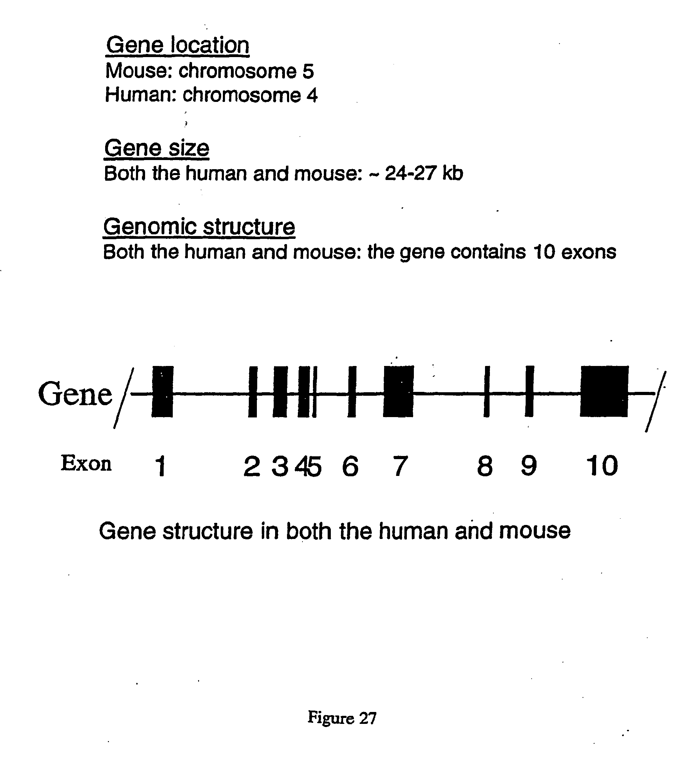

Figure 27 shows the chromosomal location of the PRSP gene and its genomic structure (exon and intron boundaries) in the human and mouse.

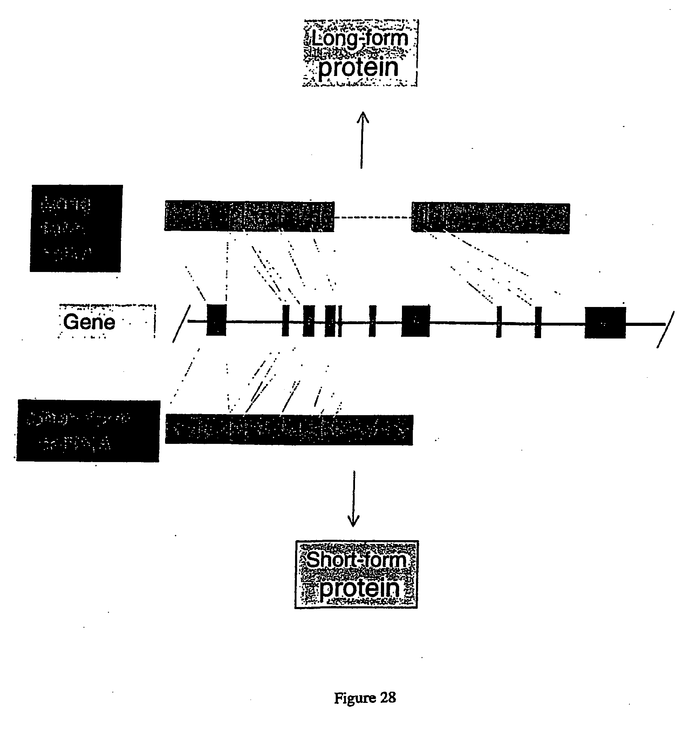

Figure 28 shows a proposed molecular mechanism for the generation of long and short isoforms of PRSP protein due to alternative splicing of the pre-mRNA in the mouse and human.

### DETAILED DESCRIPTION OF THE INVENTION

Amino acid sequence variants of PRSP are prepared by introducing appropriate nucleotide changes into PRSP DNA, and subsequently expressing the resulting modified DNA in a host cell; alternatively amino acid variants may be prepared by *in vitro* synthesis. Such variants include deletions, insertions or substitutions of amino acid residues within the PRSP amino acid sequence set out in Figure 2, Figure 4A, Figure 4B or Figure 6B (mouse short form). Any combination of deletion, insertion, and substitution may be made to arrive at an amino acid sequence variant of PRSP, provided that the variant possesses the desired functional characteristics described herein. Changes made in the amino acid sequence set out in Figure 2, Figure 4A, Figure 4B or Figure 6B to arrive at an amino acid sequence variant of PRSP may also result in further modifications of PRSP when it is expressed in host cells, for example, by virtue of such changes introducing or moving sites of glycosylation, or introducing membrane anchor sequences such as those described in International Patent Application No. WO 89/01041 (published February 9, 1989).

There are two principal variables in the construction of amino acid sequence variants of PRSP: the location of the mutation site and the nature of the mutation. These are variants from the amino acid sequence set out in Figure 2, Figure 4A, Figure 4B or Figure 6B, and may represent naturally-occurring allelic forms of PRSP, or predetermined mutant forms of PRSP made by mutating PRSP DNA, to arrive at either an allele or a variant not found in nature. In general, the location and nature of the mutation chosen will depend upon the PRSP characteristic to be modified.

For example, due to the degeneracy of nucleotide coding sequences, mutations can be made in the PRSP nucleotide sequence set out in Figure 2, Figure 3A, Figure 3B or Figure 6A without affecting the amino acid sequence of the PRSP encoded by this sequence. Other mutations can be made which will result in a PRSP which has an amino acid sequence different from that set out in Figure 2, Figure 4A, Figure 4B or Figure 6B, but which is functionally active. Such functionally active amino acid sequence variants of PRSP are selected, for example, by substituting one or more amino acid residues in the amino acid sequence set out in Figure 2, Figure 4A, Figure 4B or Figure 6B with other amino acid residues of a similar or different polarity or charge.

One useful approach is called "alanine scanning mutagenesis". This method identifies an amino acid residue or group of target residues(for example charged residues such as arg, asp, his, lys, and glu) and, by means of recombinant DNA technology, replaces it by a neutral or negatively-charged amino acid, most preferably alanine or polyalanine, in order to affect the interaction of the amino acids with the surrounding aqueous environment in or outside the cell (Cunningham *et al.*, 1989). Those domains demonstrating functional sensitivity to the substitutions are then refined by introducing further or other variants at or for the sites of substitution.

Thus, while the site for introducing an amino acid sequence variation is predetermined, the nature of the mutation per se need not be predetermined. For example, to optimize the performance of a mutation at a given site, alanine scanning or random mutagenesis is conducted at the target codon or region, and the expressed PRSP variants are screened for functional activity.

Amino acid sequence deletions generally range from about 1 to 30 residues, more preferably about 1 to 10 residues, and typically are contiguous. Deletions from regions of substantial homology with other serine proteases are more likely to affect the functional activity of PRSP. Generally, the number of consecutive deletions will be selected so as to preserve the tertiary structure of PRSP in the affected domain, e.g., β-pleated sheet or α-helix.

Amino acid sequence insertions include amino- and/or carboxyl-terminal fusions ranging in length from one amino acid residue to polypeptides containing a hundred or more residues, as well as intrasequence insertions of single or multiple amino acid residues. Intrasequence insertions, i.e. insertions made within the amino acid sequence set out in Figure 2, Figure 4A, Figure 4B or Figure 6B, may range generally from about 1 to 10 residues, more preferably 1 to 5, most preferably 1 to 3 residues. Examples of terminal insertions include PRSP with an N-terminal methionyl residue, such as may result from the direct expression of PRSP in recombinant cell culture, and PRSP with a heterologous N-terminal signal sequence to improve the secretion of PRSP from recombinant host cells. Such signal sequences generally will be homologous to the host cell used for expression of PRSP, and include STII or lpp for *E*. *coli*, alpha factor for yeast, and viral signals such as herpes gD for mammalian cells. Other insertions include the fusion to the N- or C-terminus of PRSP of immunogenic polypeptides, for example bacterial polypeptides such as beta-lactamase or an enzyme encoded by the *E. coli* trp locus, or yeast protein, and C-terminal fusions with proteins having a long half-life, such as immunoglobulin constant regions, albumin, or ferritin, as described in International Patent Application No. WO 89/02922 (published April 6, 1989).

The third group of variants are those in which at least one amino acid residue in the amino acid sequence set out in Figure 2, Figure 4A, Figure 4B or Figure 6B, preferably one to four, more preferably one to three, even more preferably one to two, and most preferably only one, has been removed, and a different residue inserted in its place. The sites of greatest interest for making such substitutions are in the regions of the amino acid sequence set out in Figure 2, Figure 4A, Figure 4B or Figure 6B which have the greatest homology with other serine proteases of the HtrA type. Such sites are likely to be important to the functional activity of the PRSP. Accordingly, to retain functional activity, those sites, especially those falling within a sequence of at least three other identically conserved sites, are substituted in a relatively conservative manner. Such conservative substitutions are shown in Table 1 under the heading of preferred substitutions. If such substitutions do not result in a change in functional activity, then more substantial changes, denoted exemplary substitutions in Table 1, or as further described below in reference to amino acid classes, may be introduced, and the resulting variant PRSP analyzed for functional activity.

**TABLE 1**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro | pro |
| His (H) | asn; gln; lys; arg | arg |
| Ile (I) | leu; val; met; ala; phe; norleucine; | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala | leu |
| Pro (P) | gly | gly |
| Ser (S) | thr | thr |
| Thr (T) | ser | ser |
| Trp (W) | tyr | tyr |
| Tyr (Y) | trp; phe; thr; ser | phe |
| Val (V) | ile; leu; met; phe; ala; norleucine | leu |

Insertional, deletional, and substitutional changes in the amino acid sequence set out in Figure 2, Figure 4A, Figure 4B or Figure 6B may be made to improve the stability of PRSP. For example, trypsin or other protease cleavage sites are identified by inspection of the encoded amino acid sequence for an arginyl or lysinyl residue. These are rendered resistant to protease by substituting the residue with another residue, preferably a basic residue such as glutamine or a hydrophobic residue such as serine; by deleting the residue; or by inserting a prolyl residue immediately after the residue. In addition, any cysteine residues not involved in maintaining the proper conformation of PRSP for functional activity may be substituted, generally with serine, to improve the stability of the molecule to oxidation and to prevent aberrant crosslinking.

PRSP has sequence similarity to serine proteases of the high temperature requirement-A (HtrA) family. Accordingly, additional sites for mutation are those sites which are conserved amongst species variants of PRSP, but are not conserved between PRSP and HtrA. Such sites are candidate sites for modulating the specificity and selectivity of PRSP.

Covalent modifications of PRSP molecules are also disclosed. For example, covalent modifications may be introduced into PRSP by reacting targeted amino acid residues of the PRSP with an organic derivatizing agent which is capable of reacting with selected amino acid side chains or with the N- or C-terminal residues.

Cysteinyl residues are most commonly reacted with α-haloacetates or corresponding amines, such as chloroacetic acid or chloroacetamide, to give carboxymethyl or carboxyamidomethyl derivatives. Cysteinyl residues may also be derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are suitably derivatized by reaction with diethylpyrocarbonate at pH 5.5-7.0, because this agent is relatively specific for the histidyl side chain. Para-bromophenacyl bromide is also useful for this purpose; the reaction is preferably performed in 0.1 M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing α-amino-containing residues include imidoesters such as methyl picolinimidate; pyridoxal phosphate; pyridoxal; chloroborohydride; trinitrobenzenesulfonic acid; O-methylisourea; 2,4-pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions, because of the high pKₐ of the guanidine functional group. Furthermore, these reagents may react with the amino group of lysine as well as with the arginine epsilon-amino group.

Tyrosyl residues may be specifically modified in order to introduce spectral labels, by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues may also be iodinated using ¹²⁵I or ¹³¹I to prepare labeled proteins for use in radioimmunoassay, for example using the chloramine-T method.

Carboxyl side groups on aspartyl or glutamyl residues are selectively modified by reaction with carbodiimides (R'-N=C=N-R'), where R and R' are different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Derivatization with bifunctional agents is useful for crosslinking PRSP to a water-insoluble support matrix or surface for use in affinity methods for purifying anti-PRSP antibodies, or for therapeutic use. Commonly-used crosslinking agents include 1,1-bis(diazoacetyl)-2-phenylethane, glutaraldehyde, N-hydroxysuccinimide esters, for example esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-[(p-azidophenyl)-dithio]propioimidate yield photoactivatable intermediates that are capable of forming crosslinks in the presence of light. Alternatively, reactive water-insoluble matrices such as cyanogen bromide-activated carbohydrates and the reactive substrates described in U.S. Pat. Nos. 3,969,287; 3,691,016; 4,195,128; 4,247,642; 4,229,537; and 4,330,440 may be employed for protein immobilization.

Glutaminyl and asparaginyl residues are frequently deamidated to the corresponding glutamyl and aspartyl residues, respectively. Alternatively, these residues are deamidated under mildly acidic conditions. Either form of these residues falls within the scope of this invention.

Other modifications include hydroxylation of proline and lysine, phosphorylation of hydroxyl groups of seryl or threonyl residues, methylation of the α-amino groups of lysine, arginine, and histidine side chains, acetylation of the N-terminal amine, and amidation of any C-terminal carboxyl group (Creighton, 1983). PRSP may also be covalently linked to non-proteinaceous polymers, e.g. polyethylene glycol, polypropylene glycol or polyoxyalkylenes, in the manner set out in U.S. Patent Nos. 4,179,337; 4,301,144; 4,496,689; 4,640,835; 4,670,417; or 4,791,192.

"PRSP antagonist" or "antagonist" refers to a substance which opposes or interferes with a functional activity of PRSP.

The terms "cell", "host cell", "cell line", and "cell culture" are used interchangeably, and all such terms should be understood to include progeny of the cells. Thus the words "transformants" and "transformed cells" include the primary subject cell and cultures derived therefrom, without regard for the number of times the cultures have been passaged. It should also be understood that all progeny may not be precisely identical in DNA sequence, due to deliberate or inadvertent mutations.

"Plasmids" are DNA molecules that are capable of replicating within a host cell, either extrachromosomally or as part of the host cell chromosome(s), and are designated by a lower case "p" preceded and/or followed by capital letters and/or numbers. The starting plasmids referred to herein are commercially available, are publicly available on an unrestricted basis, or can be constructed from such available plasmid, either as disclosed herein and/or in accordance with published procedures. In certain instances, as will be apparent to the person of ordinary skill in the art , other plasmids known in the art may be used interchangeably with plasmids described herein.

"Control sequences" refers to DNA sequences necessary for the expression of an operably linked nucleotide coding sequence in a particular host cell. Control sequences suitable for expression in prokaryotes include origins of replication, promoters, ribosome binding sites, and transcription termination sites. Control sequences suitable for expression in eukaryotes include origins of replication, promoters, ribosome binding sites, polyadenylation signals, and enhancers.

An "exogenous" element is one which is foreign to the host cell, or homologous to the host cell but in a position within the host cell in which the element is ordinarily not found.

"Digestion" of DNA refers to the catalytic cleavage of DNA with an enzyme which acts only at certain locations in the DNA. Such enzymes are called restriction enzymes or restriction endonucleases, and the sites within DNA where such enzymes cleave are called restriction sites. If there are multiple restriction sites within the DNA, digestion will produce two or more linearized DNA fragments (restriction fragments). The various restriction enzymes used herein are commercially available, and the appropriate reaction conditions, cofactors, and other requirements recommended by the manufacturers are used. Restriction enzymes are commonly designated by abbreviations composed of a capital letter followed by other letters representing the microorganism from which each restriction enzyme was originally obtained and a number designating the particular enzyme. In general, about 1 µg of DNA is digested with about 1-2 units of enzyme in about 20 µl of buffer solution. Appropriate buffers and substrate amounts for particular restriction enzymes are specified by the manufacturer, and/or are well known in the art.

"Recovery" or "isolation" of a given fragment of DNA from a restriction digest is typically accomplished by separating the digestion products, referred to as "restriction fragments", on a polyacrylamide or agarose gel by electrophoresis, identifying the fragment of interest on the basis of its mobility relative to that of marker DNA fragments of known molecular weight, excising the portion of the gel containing the desired fragment, and separating the DNA from the gel, for example by commercial spin columns.

"Ligation" refers to the process of forming phosphodiester bonds between two double-stranded DNA fragments. Unless otherwise specified, ligation is accomplished using known buffers and conditions with 10 units of T4 DNA ligase per 0.5 µg of approximately equimolar amounts of the DNA fragments to be ligated.

"Oligonucleotides" are short-length, single- or double-stranded polydeoxynucleotides which are chemically synthesized by known methods, involving triester, phosphoramidite, or phosphonate chemistry, such as described by Engels *et al.*, (1989). They are then purified, for example by polyacrylamide gel electrophoresis.

"polymerise chain reaction", or "PCR", as used herein generally refers to a method for amplification of a desired nucleotide sequence *in vitro*, as described in U.S. Patent No. 4,683,195. In general, the PCR method involves repeated cycles of primer extension synthesis, using two oligonucleotide primers capable of hybridizing preferentially to a template nucleic acid. Typically, the primers used in the PCR method will be complementary to nucleotide sequences within the template at both ends of or flanking the nucleotide sequence to be amplified, although primers complementary to the nucleotide sequence to be amplified also may be used (Wang *et al.*, 1990; Ochman *et al.*, 1990; Triglia *et al.*, 1988).

"PCR cloning" refers to the use of the PCR method to amplify a specific desired nucleotide sequence present amongst the nucleic acids from a suitable cell or tissue source, including total genomic DNA and cDNA transcribed from total cellular RNA (Frohman *et al.*, 1988; Saiki *et al.*, 1988; Mullis *et al.*, 1987).

"Stringent conditions" for hybridization or annealing of nucleic acid molecules are those which
(1) employ low ionic strength and high temperature for washing, for example 0.015 M NaCl/0.0015 M sodium citrate/0.1% sodium dodecyl sulfate (SDS) at 50°C, or
(2) employ during hybridization a denaturing agent such as formamide, for example 50% (vol/vol) formamide with 0.1% bovine serum albumin/0.1% Ficoll/0.1% polyvinylpyrrolidone/50 mM sodium phosphate buffer at pH 6.5 with 750 mM NaCl, 75 mM sodium citrate at 42°C.
Another example is use of 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/mL), 0.1% SDS, and 10% dextran sulfate at 42°C, with washes at 42°C in 0.2 x SSC and 0.1% SDS.

"PRSP nucleic acid" is RNA or DNA which encodes PRSP. "PRSP DNA" is DNA which encodes PRSP. PRSP DNA is obtained from cDNA or genomic DNA libraries, or by *in vitro* synthesis. Identification of PRSP DNA within a cDNA or a genomic DNA library, or in some other mixture of various DNAs, is conveniently accomplished by the use of an oligonucleotide hybridization probe which is labeled with a detectable moiety, such as a radioisotope (Keller *et al.*, 1989). To identify DNA encoding PRSP, the nucleotide sequence of the hybridization probe is preferably selected so that the hybridization probe is capable of hybridizing preferentially to DNA encoding the PRSP amino acid sequence set out in Figure 2, Figure 4A, Figure 4B or Figure 6B, or a variant or derivative thereof as described herein, under the hybridization conditions chosen. Preferably the probe sequence is the one encoding the common region of the two isoforms of either the mouse or the human PRSP, as described in Figure 8A. Another method for obtaining PRSP nucleic acid is chemical synthesis, for example using one of the methods described by Engels *et al.*, (1989).

If the entire nucleotide coding sequence for PRSP is not obtained in a single cDNA, genomic DNA, or other DNA, as determined by DNA sequencing or restriction endonuclease analysis, then appropriate DNA fragments (e.g., restriction fragments or PCR amplification products) may be recovered from several DNAs and covalently joined to one another to construct the entire coding sequence. The preferred means of covalently joining DNA fragments is by ligation using a DNA ligase enzyme, such as T4 DNA ligase.

"Isolated" PRSP nucleic acid is PRSP nucleic acid which is identified and separated from, or otherwise substantially free from, contaminant nucleic acid encoding other polypeptides. The isolated PRSP nucleic acid can be incorporated into a plasmid or expression vector, or can be labeled for diagnostic and probe purposes, using a label as described further.

Isolated PRSP nucleic acid may also be used to produce PRSP by recombinant DNA and recombinant cell culture methods.

Host cells may be transformed or transfected with recombinant DNA molecules comprising an isolated PRSP DNA, to obtain expression of the PRSP DNA and thus the production of PRSP in large quantities. DNA encoding amino acid sequence variants of PRSP is prepared by a variety of methods known in the art. These methods include, but are not limited to, isolation from a natural source (in the case of naturally-occurring amino acid sequence variants of PRSP) or preparation by site-directed (or oligonucleotide-mediated) mutagenesis, PCR mutagenesis, and cassette mutagenesis of an earlier prepared DNA encoding a variant or a non-variant form of PRSP.

Site-directed mutagenesis is a preferred method for preparing substitution, deletion, and insertion variants of PRSP DNA. This technique is well known in the art (Zoller *et al.*, 1983; Zoller *et al.*, 1987; Carter 1987; Horwitz *et al.*, 1990), and has been used to produce amino acid sequence variants of proteins (Perry *et al.*, 1984; Craik *et al.*, 1985).

Briefly, in carrying out site-directed mutagenesis of PRSP DNA, the PRSP DNA is altered by first hybridizing an oligonucleotide encoding the desired mutation to a single strand of such PRSP DNA. After hybridization, a DNA polymerase is used to synthesize an entire second strand, using the hybridized oligonucleotide as a primer, and using the single strand of PRSP DNA as a template. Thus the oligonucleotide encoding the desired mutation is incorporated in the resulting double-stranded DNA.

Oligonucleotides for use as hybridization probes or primers may be prepared by any suitable method, such as by purification of a naturally-occurring DNA or by *in vitro* synthesis. For example, oligonucleotides are readily synthesized using various techniques in organic chemistry, such as those described by Narang *et al.*, (1979); Brown *et al.*, (1979); Caruther *et al.*, (1985). The general approach to selecting a suitable hybridization probe or primer is well known (Keller *et al.*, 1989). Typically, the hybridization probe or primer will contain 10-25 or more nucleotides, and will include at least 5 nucleotides on either side of the sequence encoding the desired mutation so as to ensure that the oligonucleotide will hybridize preferentially to the single-stranded DNA template molecule.

Multiple mutations are introduced into PRSP DNA to produce amino acid sequence variants of PRSP comprising several or a combination of insertions, deletions, or substitutions of amino acid residues as compared to the amino acid sequence set out in Figure 2, Figure 4A, or Figure 4B. If the sites to be mutated are located close together, the mutations may be introduced simultaneously using a single oligonucleotide that encodes all of the desired mutations. If, however, the sites to be mutated are located some distance from each other, for example separated by more than about ten nucleotides, it is more difficult to generate a single oligonucleotide encoding all of the desired changes. Instead, one of two alternative methods may be employed.

In the first method, a separate oligonucleotide is generated for each desired mutation. The oligonucleotides are then annealed to the single-stranded template DNA simultaneously, and the second strand of DNA which is synthesized from the template will encode all of the desired amino acid substitutions.

The alternative method involves two or more rounds of mutagenesis to produce the desired mutant. The first round is as described for introducing a single mutation: a single strand of a previously prepared PRSP DNA is used as a template, an oligonucleotide encoding the first desired mutation is annealed to this template, and a heteroduplex DNA molecule is then generated. The second round of mutagenesis utilizes the mutated DNA produced in the first round of mutagenesis as the template. Thus this template already contains one or more mutations. The oligonucleotide encoding the additional desired amino acid substitution(s) is then annealed to this template, and the resulting strand of DNA now encodes mutations from both the first and second rounds of mutagenesis. This resultant DNA can be used as a template in a third round of mutagenesis, and so on.

PCR mutagenesis is also suitable for making amino acid sequence variants of PRSP (Higuchi, 1990); Vallette *et al.*, 1989). Briefly, when small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template. For introduction of a mutation into a plasmid DNA, one of the primers is designed to overlap the position of the mutation and to contain the mutation; the sequence of the other primer must be identical to a nucleotide sequence within the opposite strand of the plasmid DNA, but this sequence can be located anywhere along the plasmid DNA. It is preferred, however, that the sequence of the second primer is located within 200 nucleotides from that of the first, such that in the end the entire amplified region of DNA bounded by the primers can be easily sequenced. PCR amplification using a primer pair like the one just described results in a population of DNA fragments that differ at the position of the mutation specified by the primer, and possibly at other positions, as template copying is somewhat error-prone (Wagner *et al.*, 1991).

If the ratio of template to product amplified DNA is extremely low, the majority of product DNA fragments incorporate the desired mutation(s). This product DNA is used to replace the corresponding region in the plasmid that served as PCR template using standard recombinant DNA methods. Mutations at separate positions can be introduced simultaneously by either using a mutant second primer, or by performing a second PCR with different mutant primers and ligating the two resulting PCR fragments simultaneously to the plasmid fragment in a three (or more)-part ligation.

Another method for preparing variants, cassette mutagenesis, is based on the technique described by Wells *et al.*,(1985). The starting material is the plasmid or other vector comprising the PRSP DNA to be mutated. The codon(s) in the PRSP DNA to be mutated are identified. There must be a unique restriction endonuclease site on each side of the identified mutation site(s). If no such restriction sites exist, they may be generated using the above-described oligonucleotide-mediated mutagenesis method to introduce them at appropriate locations in the PRSP DNA. The plasmid DNA is linearized by cleavage at these sites. A double-stranded oligonucleotide encoding the sequence of the DNA between the restriction sites but containing the desired mutation(s) is synthesized using standard procedures, in which the two strands of the oligonucleotide are synthesized separately and then hybridized together using standard techniques. This double-stranded oligonucleotide is referred to as the cassette. This cassette is designed to have 5' and 3' ends that are compatible with the ends of the linearized plasmid, such that it can be directly ligated to the plasmid. This plasmid now contains the mutated PRSP DNA sequence.

PRSP DNA, whether cDNA or genomic DNA or a product of *in vitro* synthesis, is ligated into a replicable vector for further cloning or for expression. "Vectors" are plasmids and other DNAs which are capable of replicating autonomously within a host cell, and are therefore useful for performing two functions in conjunction with compatible host cells (a vector-host system). One function is to facilitate the cloning of the nucleic acid that encodes the PRSP, i.e., to produce usable quantities of the nucleic acid. The other function is to direct the expression of PRSP. One or both of these functions are performed by the vector-host system. The vectors will contain different components, depending upon the function they are to perform as well as the host cell with which they are to be used for cloning or expression.

To produce PRSP, an expression vector will contain nucleic acid that encodes PRSP as described above. The PRSP is expressed directly in recombinant cell culture, or as a fusion with a heterologous polypeptide, preferably a signal sequence or other polypeptide having a specific cleavage site at the junction between the heterologous polypeptide and the PRSP.

In one example of recombinant host cell expression, mammalian cells are transfected with an expression vector comprising PRSP DNA and the PRSP encoded thereby is recovered from the culture medium in which the recombinant host cells are grown. It will be clearly understood that the expression vectors and methods disclosed herein are suitable for use over a wide range of prokaryotic and eukaryotic organisms.

Prokaryotes may be used for the initial cloning of DNAs and the construction of the vectors useful in the invention. However, prokaryotes may also be used for expression of DNA encoding PRSP. Polypeptides produced in prokaryotic host cells typically will be non-glycosylated.

Plasmid or viral vectors containing replication origins and other control sequences derived from species compatible with the host cell are used in conjunction with prokaryotic host cells, for cloning or expression of an isolated DNA. For example, *E. coli* is typically transformed using pBR322, a plasmid derived from an *E. coli* species (Bolivar *et al.*, 1987). PBR322 contains genes for ampicillin and tetracycline resistance, so that cells transformed by the plasmid can easily be identified or selected. To serve as an expression vector, the pBR322 plasmid, or other plasmid or viral vector, must also contain, or be modified to contain, a promoter that functions in the host cell to provide messenger RNA (mRNA) transcripts of a DNA inserted downstream of the promoter (Rangagwala *et al.*,1991).

In addition to prokaryotes, eukaryotic microbes, such as yeast, may also be used as hosts for the cloning or expression of DNAs useful in the invention. *Saccharomyces cerevisiae,* or common baker's yeast, is the most commonly used eukaryotic microorganism. Plasmids useful for cloning or expression in yeast cells of a desired DNA are well known, as are various promoters that function in yeast cells to produce mRNA transcripts.

Furthermore, cells derived from multicellular organisms also may be used as hosts for the cloning or expression of DNAs useful in the invention. Mammalian cells are most commonly used, and the procedures for maintaining or propagating such cells *in vitro*, which procedures are commonly referred to as tissue culture, are well known (Kruse & Patterson, 1977). Examples of useful mammalian cells are human cell lines such as 293, HeLa, and WI-38, monkey cell lines such as COS-7 and VERO, and hamster cell lines such as BHK-21 and CHO, all of which are publicly available from the American Type Culture Collection (ATCC), Rockville, Maryland 20852 USA.

Expression vectors, unlike cloning vectors, should contain a promoter which is recognized by the host organism and is operably linked to the PRSP nucleic acid. Promoters are untranslated sequences that are located upstream from the start codon of a gene and that control transcription of the gene, ie. the synthesis of mRNA. Promoters typically fall into two classes, inducible and constitutive. Inducible promoters are promoters that initiate high level transcription of the DNA under their control in response to some change in culture conditions, for example the presence or absence of a nutrient or a change in temperature.

A large number of promoters are known, and these may be operably linked to PRSP DNA to achieve expression of PRSP in a host cell. Although the promoter associated with naturally-occurring PRSP DNA is usable, heterologous promoters will generally result in greater transcription and higher yields of expressed PRSP.

Promoters suitable for use with prokaryotic hosts include the β-lactamase and lactose promoters (Goeddel *et al.*, 1979), tryptophan (trp) promoter (Goeddel *et al.*, 1980), and hybrid promoters such as the tac promoter (deBoer *et al.*, 1983). However, other known bacterial promoters are suitable. Their nucleotide sequences have been published (Siebenlist *et al.*, 1980), thereby enabling a skilled worker to ligate them operably to DNA encoding PRSP using linkers or adaptors to supply any required restriction sites (Wu *et al.*, 1987).

Suitable promoters for use with yeast hosts include the promoters for 3-phosphoglycerate kinase (Hitzeman *et al.*, 1980; Kingsman *et al.*, 1990), or other glycolytic enzymes such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase (Dodson *et al.*, 1982; Emr, 1990).

Expression vectors useful in mammalian cells typically include a promoter derived from a virus. For example, promoters derived from polyoma virus, adenovirus, cytomegalovirus (CMV), and simian virus 40 (SV40) are commonly used. Further, it is also possible, and often desirable, to utilize promoter or other control sequences associated with a naturally-occurring DNA which encodes PRSP, provided that such control sequences are functional in the particular host cell used for recombinant DNA expression.

Other control sequences desirable in an expression vector in addition to a promoter are a ribosome binding site, and in the case of an expression vector used with eukaryotic host cells, an enhancer. Enhancers are cis-acting elements of DNA, usually about from 10-300 bp, which act on a promoter to increase the level of transcription. Many enhancer sequences from mammalian genes are now known, for example those from the genes for globin, elastase, albumin, α-fetoprotein and insulin. Typically, however, the enhancer used will be one from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers (Kriegler, 1990).

Expression vectors may also contain sequences necessary for the termination of transcription and for stabilizing the mRNA (Balbas *et al.*, 1990; Levinson, 1990). In the case of expression vectors used with eukaryotic host cells, such transcription termination sequences may be obtained from the untranslated regions of eukaryotic or viral DNAs or cDNAs. These regions contain polyadenylation sites as well as transcription termination sites (Birnsteil *et al.*,1985).

In general, control sequences are DNA sequences necessary for the expression of an operably-linked coding sequence in a particular host cell. "Expression" refers to transcription and/or translation. "Operably-linked" refers to the covalent joining of two or more DNA sequences, by means of enzymatic ligation or otherwise, in a configuration relative to one another such that the normal function of the sequences can be performed. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. Linking is accomplished by ligation at convenient restriction sites. If such sites do not exist, then synthetic oligonucleotide adaptors or linkers are used, in conjunction with standard recombinant DNA methods.

Expression and cloning vectors will also contain a sequence which enables the vector to replicate in one or more selected host cells. Generally, in cloning vectors this sequence is one that enables the vector to replicate independently of the host chromosome(s), and includes origins of replication or autonomously replicating sequences. Such sequences are well known for a variety of bacteria, yeast, and viruses. The origin of replication from the plasmid pBR322 is suitable for most Gram-negative bacteria, the 2µ plasmid origin is suitable for yeast, and various viral origins, for example those from SV40, polyoma, or adenovirus, are useful for cloning vectors in mammalian cells. Most expression vectors are "scuttle" vectors, i.e. they are capable of replication in at least one class of organisms but can be transfected into another organism for expression. For example, a vector may be cloned in B. coli, and then the same vector is transfected into yeast or mammalian cells for expression, even though it is not capable of replicating independently of the host cell chromosome.

The expression vector may also include an amplifiable gene, such as that comprising the coding sequence for dihydrofolate reductase (DHFR). Cells containing an expression vector which includes a DHFR gene may be cultured in the presence of methotrexate, a competitive antagonist of DHFR. This leads to the synthesis of multiple copies of the DHFR gene and, concomitantly, multiple copies of other DNA sequences comprising the expression vector (Ringold *et al.*, 1981), such as a DNA sequence encoding PRSP, enabling the level of PRSP produced by the cells to be increased.

DHFR protein encoded by the expression vector may also be used as a selectable marker of successful transfection. For example, if the host cell prior to transformation is lacking in DHFR activity, successful transformation by an expression vector comprising DNA sequences encoding PRSP and DHFR protein can be determined by cell growth in medium containing methotrexate. Furthermore, mammalian cells transformed by an expression vector comprising DNA sequences encoding PRSP, DHFR protein, and aminoglycoside 3' phosphotransferase (APH) can be selected by cell growth in medium containing an aminoglycoside antibiotic such as kanamycin or neomycin. Because eukaryotic cells do not normally express an endogenous APH activity, genes encoding APH protein, commonly referred to as neo^{r} genes, may be used as dominant selectable markers in a wide range of eukaryotic host cells, by which cells transfected by the vector can easily be identified or selected (Jiminez *et al.*,1980; Colbere-Garapin *et al.*,1981; Okayama & Berg, 1983).

Many other selectable markers which may be used for identifying and isolating recombinant host cells that express PRSP are known. For example, a suitable selection marker for use in yeast is the trp1 gene present in the yeast plasmid YRp7 (Stinchcomb *et al.*,1979; Kingsman *et al.*,1979; Tschemper *et al.*,1980). The trp1 gene provides a selection marker for a mutant strain of yeast lacking the ability to grow in tryptophan, for example, ATCC No. 44076 or PEP4-1 (available from the American Type Culture Collection, Rockville, Maryland 20852 USA, Jones, 1977). The presence of the trp1 lesion in the yeast host cell genome then provides an effective environment for detecting transformation by growth in the absence of tryptophan. Similarly, Leu2-deficient yeast strains (ATCC Nos. 20622 or 38626) are complemented by known plasmids bearing the Leu2 gene.

Expression vectors which provide for the transient expression in mammalian cells of DNA encoding PRSP are particularly useful in the invention. In general, transient expression involves the use of an expression vector which is able to efficiently replicate in a host cell, so that the host cell accumulates many copies of the expression vector and, in turn, synthesizes high levels of a desired polypeptide encoded by the expression vector. Transient expression systems, comprising a suitable expression vector and a host cell, allow for the convenient positive identification of polypeptides encoded by cloned DNAs, as well as for the rapid screening of such polypeptides for desired biological or physiological properties (Yang *et al.*, 1986; Wong *et al.*, 1985; Lee *et al.*, 1985). Thus transient expression systems are particularly useful for expressing DNAs encoding amino acid sequence variants of PRSP, to identify those variants which are functionally active.

Since it is often difficult to predict in advance the characteristics of an amino acid sequence variant of PRSP, it will be appreciated that some screening of such variants will be needed to identify those that are functionally active. Such screening may be performed *in vitro*, using routine assays for serine protease activity and IGF binding activity, or using immunoassays with monoclonal antibodies which selectively bind to functionally active PRSP, such as a monoclonal antibody which selectively binds to the active site or IGF binding site of PRSP.

As used herein, the terms "transformation" and "transfection" refer to the process of introducing a desired nucleic acid, such a plasmid or an expression vector, into a host cell. Various methods of transformation and transfection are available, depending on the nature of the host cell. In the case of *E. coli* cells, the most common methods involve treating the cells with aqueous solutions of calcium chloride and other salts. In the case of mammalian cells, the most common methods are transfection mediated by either calcium phosphate or DEAE-dextran, or electroporation (Sambrook *et al.*, 1989). Following transformation or transfection, the desired nucleic acid may integrate into the host cell genome, or may exist as an extrachromosomal element.

Host cells transformed or transfected with the above-described plasmids and expression vectors are cultured in conventional nutrient medium modified as is appropriate for inducing promoters or selecting for drug resistance or some other selectable marker or phenotype. The culture conditions, such as temperature, pH, and the like, suitably are those previously used for culturing the host cell used for cloning or expression, as the case may be, and will be apparent to those skilled in the art.

Suitable host cells for cloning or expressing the vectors herein are prokaryotes, yeasts, and higher eukaryotes, including plant, insect, vertebrate, and mammalian host cells. Suitable prokaryotes include eubacteria, such as Gram-negative or Gram-positive organisms, for example *E*. *coli, Bacillus* species such as *B*. *subtilis, Pseudomonas* species such as *P*. *aeruginosa, Salmonella typhimurium,* or *Serratia marcescens.*

In addition to prokaryotes, eukaryotic microbes such as filamentous fungi or yeast are suitable hosts for PRSP-encoding vectors. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe*, (Beach and Nurse, 1981), *Pichia pastoris* (Cregg *et al.*, 1987; Sreekrishna *et al.*, 1989), *Neurospora crassa* (Case *et al.*, 1979), and Aspergillus hosts such as *A*. *nidulans* (Ballance *et al.*, 1983; Tilburn *et al.*,1983; Yelton *et al.*,1984), and *A.* niger (Kelly *et al.*.,1985).

Suitable host cells for the expression of PRSP also include those derived from multicellular organisms. Such host cells are capable of complex processing and glycosylation activities. In principle, any higher eukaryotic cell culture, whether from vertebrate or invertebrate culture, is useable. It will be appreciated, however, that because of the species-, tissue-, and cell-specificity of glycosylation (Rademacher *et al.*, 1988), the extent or pattern of glycosylation of PRSP in a foreign host cell typically will differ from that of PRSP obtained from a cell in which it is naturally expressed.

Examples of invertebrate cells include insect and plant cells. Numerous baculoviral strains and variants and corresponding permissive insect host cells from hosts such as *Spodoptera frugiperda* (caterpillar), *Aedes aegypti* or Aedes *albopictus* (mosquito), *Drosophila melanogaster* (fruitfly), and *Bombyx mori* (silkworm) have been identified (Luckow *et al.*, 1988; Miller *et al.*, 1986; Maeda *et al.*,1985).

Plant cell cultures of cotton, corn, potato, soybean, petunia, tomato, and tobacco can be utilized as hosts. Typically, plant cells are transfected by incubation with certain strains of the bacterium *Agrobacterium tumefaciens*, which has been previously altered to contain PRSP DNA. During incubation of the plant cells with A. *tumefaciens,* the DNA encoding the PRSP is transferred into cells, so that they become transfected, and will, under appropriate conditions, express the PRSP DNA. In addition, regulatory and signal sequences compatible with plant cells are available, such as the nopaline synthase promoter and polyadenylation signal sequences, and the ribulose biphosphate carboxylase promoter (Depicker *et al.*, 1982; Herrera-Estrella *et al.*,1984). In addition, DNA segments isolated from the upstream region of the T-DNA 780 gene are capable of activating or increasing transcription levels of plant-expressible genes in recombinant DNA-containing plant tissue. (European Pat. Pub. No. EP 321,196 (published June 21, 1989)).

However, vertebrate cells are generally of greatest interest, and propagation of vertebrate cells in culture has become a routine procedure in recent years (Kruse & Patterson, 1973). Useful mammalian host cells include the monkey kidney CV1 line transformed by SV40 (COS-7, ATCC CRL 1651); human embryonic kidney line 293 (or 293 cells subcloned for growth in suspension culture (Graham *et al.*, 1977); baby hamster kidney cells (BHK, ATCC CCL 10); Chinese hamster ovary cells (including DHFR-deficient CHO cells (Urlaub *et al.*, 1980); mouse Sertoli cells (TM4; Mather, 1980); monkey kidney cells (CV1, ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather *et al.*, 1982); MRC 5 cells; FS4 cells; a human hepatoma line (Hep G2), endometrial cells (HEC-1A, HEC-1B, Ishikawa, RL95, AN3-Ca), and cells of trophoblast origin (Bewo) .

Construction of suitable vectors containing the nucleotide sequence encoding PRSP and appropriate control sequences employs standard recombinant DNA methods. DNA is cleaved into fragments, tailored, and ligated together in the form desired to generate the vectors required.

For analysis to confirm correct sequences in the vectors constructed, the vectors are analyzed by restriction digestion to confirm the presence in the vector of predicted restriction endonuclease cleavage sites, and/or by sequencing by the dideoxy chain termination method of Sanger *et al.*, (1979).

The mammalian host cells used to produce PRSP may be cultured in a variety of media. Commercially available media such as Ham's F10 (Sigma), Minimal Essential Medium (MEM, Sigma), RPMI-1640 (Sigma), and Dulbecco's Modified Eagle's Medium (DMEM, Sigma) are suitable for culturing the host cells. In addition, any of the media described in Ham *et al.*, (1979); Barnes *et al.*, (1980); Bottenstein *et al.*, (1979); U.S. Patent Nos. 4,560,655; 4,657,866; 4,767,704; or 4,927,762; or in International Patent Application No. WO 90/03430 (published April 5, 1990), may be used as culture media for the host cells. Any of these media may be supplemented as necessary with hormones and/or other growth factors such as insulin, transferrin, or epidermal growth factor, salts such as sodium chloride, calcium, magnesium, selenite and phosphate, buffers such as HEPES, nucleosides such as adenosine and thymidine, antibiotics, trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the person of ordinary skill in the art.

The host cells referred to in this disclosure encompass cells in culture *in vitro*, as well as cells that are within a host animal or plant, for example as a result of transplantation or implantation.

It is further contemplated that PRSP may be produced by homologous recombination, for example, as described in PCT Patent Publication No. WO 91/06667 (published May 16, 1991). Briefly, this method involves transforming cells containing an endogenous gene encoding PRSP with a homologous DNA, which comprises an amplifiable gene, such as DHFR, and at least one flanking sequence, having a length of at least about 150 base pairs, which is homologous with a nucleotide sequence in the cell genome which is within or in proximity to the gene encoding PRSP. The transformation is carried out under conditions such that the homologous DNA integrates into the cell genome by recombination. Cells having integrated the homologous DNA are then subjected to conditions which select for amplification of the amplifiable gene, whereby the PRSP gene is amplified concomitantly. The resulting cells then are screened for production of desired amounts of PRSP. Flanking sequences which are in proximity to a gene encoding PRSP are readily identified, for example, by the method of genomic walking, using as a starting point the PRSP nucleotide sequence set out in Figure 2, Figure 3A, Figure 3B or Figure 6A (Spoerel *et al.*, 1987).

Gene amplification and/or gene expression may be measured in a sample directly, for example, by conventional Southern blotting to quantitate DNA, or by Northern blotting to quantitate mRNA, using an appropriately labeled oligonucleotide hybridization probe, based on the sequences provided herein. Various labels may be employed, most commonly radioisotopes, particularly ³²P. However, other techniques may also be employed, such as using biotin-modified nucleotides for introduction into a polynucleotide. The biotin then serves as the site for binding to avidin or antibodies, which may be labeled with a wide variety of labels, such as radioisotopes, fluorophores, chromophores, or the like. Alternatively, antibodies which can recognize specific duplexes, including DNA duplexes, RNA duplexes, and DNA-RNA hybrid duplexes or DNA-protein duplexes, may be employed. The antibodies in turn may be labelled, and the assay may include a step in which the duplex is bound to a surface, so that upon the formation of duplex on the surface, the presence of antibody bound to the duplex can be detected.

Gene expression may alternatively be measured by immunological methods, such as immunohistochemical staining of tissue sections and assay of cell culture or body fluids, to quantitate directly the expression of the gene product, PRSP. With immunohistochemical staining techniques, a cell sample is prepared, typically by dehydration and fixation, followed by reaction with labelled antibodies specific for the gene product coupled, where the labels are usually visually detectable, such as enzymatic labels, fluorescent labels, luminescent labels, and the like. A particularly sensitive staining technique suitable for use in the present invention is described by Hsu *et al.*, (1980). Antibodies useful for immunohistochemical staining and/or assay of sample fluids may be either monoclonal or polyclonal. Conveniently, the antibodies may be prepared against a synthetic peptide based on the DNA sequences provided herein.

PRSP is preferably recovered from the culture medium as a secreted polypeptide, although it also may be recovered from host cell lysates. To obtain PRSP substantially free of contaminating proteins or polypeptides of the host cell in which it is produced it is necessary to purify the PRSP, based on the differential physical properties of PRSP as compared to the contaminants with which it may be associated. For example, as a first step, the culture medium or lysate is centrifuged to remove particulate cell debris. PRSP thereafter is purified from contaminant soluble proteins and polypeptides, for example, by ammonium sulphate or ethanol precipitation, gel filtration, ion-exchange chromatography, immunoaffinity chromatography, reverse phase HPLC, and/or gel electrophoresis. For example, PRSP can be purified by immunoaffinity chromatography using an anti-PRSP-IgG resin. Amino acid sequence variants and derivatives of PRSP are recovered in the same fashion, taking account of any distinguishing features or physical properties of the particular PRSP. For example, in the case of a fusion protein comprising PRSP and another protein or polypeptide, such as a bacterial or viral antigen, a significant degree of purification may be obtained by using an immunoaffinity column containing antibody to the antigen. In any event, the person of ordinary skill in the art will appreciate that purification methods suitable for naturally-occurring PRSP may require modification to account for changes in the character of PRSP or its variants or derivatives produced in recombinant host cells.

PRSP may be used as an immunogen to generate anti-PRSP antibodies. Preferably the PRSP which is used for immunization comprises the region of the PRSP molecule which is common to the two isoforms described herein. Such antibodies, which specifically bind to PRSP, are useful as standards in assays for PRSP, such as by labeling purified PRSP for use as a standard in a radioimmunoassay, enzyme-linked immunoassay, or competitive-type receptor binding assays radioreceptor assay, as well as in affinity purification techniques. Ordinarily, the anti-PRSP antibody will bind PRSP with an affinity of at least about 10⁶ L/mole, and preferably at least about 10⁷ L/mole. The skilled person will readily be able to determine a suitable affinity. It will also be appreciated that if the antibody is an IgM it may be possible to use antibody of lower affinity.

Polyclonal antibodies directed toward PRSP are generally raised in animals by multiple subcutaneous or intraperitoneal injections of PRSP and an adjuvant. If necessary, immunogenicity may be increased by conjugating PRSP or a peptide fragment thereof to a carrier protein which is immunogenic in the species to be immunized, such as keyhole limpet haemocyanin, serum albumin, bovine thyroglobulin, or soybean trypsin inhibitor, using a bifunctional or derivatizing agent, for example, maleimidobenzoyl sulfosuccinimide ester (conjugation through cysteine residues), N-hydroxysuccinimide (conjugation through lysine residues), glutaraldehyde, succinic anhydride, SOCl₂, or R¹N = C = NR, where R and R¹ are different alkyl groups.

Animals are immunized with such PRSP-carrier protein conjugates combining 1 mg or 1 µg of conjugate (for rabbits or mice, respectively) with 3 volumes of Freund's complete adjuvant or some other appropriate adjuvant known to those skilled in the art (eg. Montanide:Marcol) and injecting the solution intradermally at multiple sites. One month later the animals are boosted with 1/5th to 1/10th the original amount of conjugate in Freund's complete adjuvant (or other appropriate adjuvant) by subcutaneous injection at multiple sites. 7 to 14 days later animals are bled and the serum is assayed for anti-PRSP antibody titer. Animals are boosted until the antibody titer plateaus. Preferably, the animal is boosted by injection with a conjugate of the same PRSP with a different carrier protein and/or through a different crosslinking agent. Conjugates of PRSP and a suitable carrier protein also can be made in recombinant cell culture as fusion proteins. Also, aggregating agents such as alum are used to enhance the immune response.

Monoclonal antibodies directed toward PRSP are produced using any method which provides for the production of antibody molecules by continuous cell lines in culture. The modifier "monoclonal" indicates the character of the antibody as being obtained from a substantially homogeneous population of antibodies, and is not to be construed as requiring production of the antibody by any particular method. Examples of suitable methods for preparing monoclonal antibodies include the original hybridoma method of Kohler *et al.*, (1975), and the human B-cell hybridoma method (Kozbor, 1984; Brodeur *et al.*, 1987). The monoclonal antibodies which may be used in the invention specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (Cabilly, et al., U.S. Patent No. 4,816,567; Morrison *et al.*, 1984). Monoclonal antibodies may also be produced using phage display techniques well known to those of skill in the art.

In a preferred embodiment, the chimeric anti-PRSP antibody is a "humanized" antibody. Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain.

Humanization can be performed following methods known in the art (Jones *et al.*, 1986; Riechmann *et al.*, 1988; Verhoeyen *et al.*, 1988), by substituting rodent complementarity-determining regions (CDRs) for the corresponding regions of a human antibody. Alternatively, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge (Jakobovits *et al.*,1993a; Jakobovits *et al.*,1993b; Bruggermann *et al.*,1993). Human antibodies can also be produced in phage-display libraries (Hoogenboom *et al.*, 1991; Marks *et al.*, 1991).

For diagnostic applications, anti-PRSP antibodies typically will be labeled with a detectable moiety. The detectable moiety can be any one which is capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin; radioactive isotopic labels, such as, e.g., ¹²⁵I, ³²P, ¹⁴C, or ³H, or an enzyme, such as alkaline phosphatase, beta-galactosidase or horseradish peroxidase.

Any method known in the art for separately conjugating the antibody to the detectable moiety may be employed, including those methods described by David *et al.*, (1974); Pain *et al.*, (1981); and Bayer *et al.*, (1990).

The anti-PRSP antibodies may be employed in any known assay method, such as competitive binding assays, direct and indirect sandwich assays, and immunoprecipitation assays (Zola, 1987).

Competitive binding assays rely on the ability of a labeled standard (e.g., PRSP or an immunologically reactive portion thereof) to compete with the test sample analyte (PRSP) for binding with a limited amount of antibody. The amount of PRSP in the test sample is inversely proportional to the amount of standard that becomes bound to the antibodies. To facilitate determining the amount of standard that becomes bound, the antibodies generally are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three part complex (David, et al., U.S. Patent No. 4,376,110). The second antibody may itself be labeled with a detectable moiety (direct sandwich assays) or may be measured using an anti-immunoglobulin antibody which is labeled with a detectable moiety (indirect sandwich assay). For example, one type of sandwich assay is an ELISA assay, in which case the detectable moiety is an enzyme.

Neutralizing anti-PRSP antibodies are useful as antagonists of PRSP. The term "neutralizing anti-PRSP antibody" as used herein refers to an antibody which is capable of specifically binding to PRSP, and which is capable of substantially inhibiting or eliminating the functional activity of PRSP in vivo or *in vitro*. Typically a neutralizing antibody will inhibit the functional activity of PRSP by at least about 50%, and preferably greater than 80%, as determined, for example, by an enzyme activity assay.

PRSP is believed to be useful in promoting the implantation of the fertilized egg, development of the placenta and the embryo, and maintenance of pregnancy. Accordingly, PRSP may be utilized in methods for the diagnosis and/or treatment of a variety of fertility-related conditions or other conditions, including infertility due to luteal phase defect, infertility due to failure of implantation, pre-eclampsia, IUGR, early abortion, abnormal uterine bleeding, endometriosis, cancers and parturition, or it may provide a potential target for contraception. It may also play a role in muscle function, including those of the heart, skeletal muscle, lung and the diaphragm.

PRSP may be formulated with other ingredients such as carriers and/or adjuvants, e.g. albumin, nonionic surfactants and other emulsifiers. There are no limitations on the nature of such other ingredients, except that they must be pharmaceutically acceptable, efficacious for their intended administration, and cannot degrade the activity of the active ingredients of the compositions. Suitable adjuvants include collagen or hyaluronic acid preparations, fibronectin, factor XIII, or other proteins or substances designed to stabilize or otherwise enhance the active therapeutic ingredient(s).

PRSP and PRSP antagonists to be used for in vivo administration must be sterile. This is readily accomplished by filtration of a solution of PRSP or anti-PRSP antibody through sterile filtration membranes. Thereafter, the filtered solution may be placed into a container having a sterile access port, for example, an intravenous solution bag or vial having a stopper pierceable by a hypodermic injection needle. The filtered solution also may be lyophilized to produce sterile PRSP or anti-PRSP antibody in a powder form.

Methods for administering PRSP and PRSP antagonists in vivo include injection or infusion by intravenous, intraperitoneal, intracerebral, intrathecal, intramuscular, intraocular, intraarterial, intrauterine, intracervical, intravaginal or intralesional routes, and by means of sustained-release formulations or by topical application to the skin.

Sustained-release formulations generally consist of PRSP or PRSP antagonists and a matrix from which the PRSP or PRSP antagonists are released over some period of time. Suitable matrices include semipermeable polymer matrices in the form of shaped articles, for example, membranes, fibers, or microcapsules. Sustained release matrices may comprise polyesters, hydrogels, polylactides (U.S. Patent No. 3,773,919), copolymers of L-glutamic acid and gamma ethyl-L-glutamate (Sidman *et al.*, 1983), poly (2-hydroxyethyl-methacrylate), or ethylene vinyl acetate (Langer *et al.*, 1981; Langer, 1982).

Therapeutic formulations may comprise PRSP or PRSP antagonist entrapped within or complexed with liposomes. For example, PRSP covalently joined to a glycophosphatidyl-inositol moiety may be used to form a liposome comprising PRSP.

An effective amount of PRSP or PRSP antagonist, e.g., anti-PRSP antibody, to be employed therapeutically will depend upon the therapeutic objectives, the route of administration, and the condition of the patient. Accordingly, it will be necessary for the therapist to titrate the dosage and modify the route of administration as required to obtain the optimal therapeutic effect. A typical daily dosage might range from about 1 ug/kg to up to 100 mg/kg or more, depending on the factors mentioned above. Where possible, it is desirable to determine appropriate dosage ranges first *in vitro,* for example by using assays for serine protease activity and IGF binding activity which are known in the art, and then in suitable animal models, from which dosage ranges for human patients may be extrapolated.

For example, the dose of a protein PRSP antagonist, particularly an antibody, can be about 0.1 mg to about 500 mg, typically about 1.0 mg to about 300 mg, more typically about 25 mg to about 100 mg. The administration frequency can be appropriately selected, depending upon the condition to be treated and the dosage form, for the desired therapeutic effects.

In summary, by providing nucleic acid molecules encoding PRSP, the present disclosure enables for the first time the production of PRSP by recombinant DNA methods, thus providing a reliable source of sufficient quantities of PRSP for use in various diagnostic and therapeutic applications.

The invention will now be described in detail by way of reference only to the following non-limiting examples and drawings.

### MATERIALS AND METHODS

### Animals and Tissue Preparation

Swiss outbred mice were housed and handled according to the Monash University animal ethics guidelines on the care and use of laboratory animals. All experimentation was approved by the Institutional Animal Ethics Committee at the Monash Medical Centre. Adult female mice (6-8 weeks old) were mated with fertile males of the same strain to produce normal pregnant animals, or mated with vasectomized males to produce pseudopregnant mice. The morning of finding a vaginal plug was designated as day 0 of pregnancy. Uterine tissues were collected from non-pregnant mice, or from pregnant mice on days 3-11. A selection of other mouse organs was also collected from non-pregnant mice. Tissues were snap-frozen in liquid nitrogen for Northern analysis, or fixed in 4% buffered formalin (pH 7.6) for *in situ* hybridization.

For non-pregnant and day 3.5 pregnant mice, the entire uterus was collected. For day 4.5 pregnant mice, implantation sites were visualised by intravenous injections of a Chicago Blue dye solution (1% in saline, 0.1 ml/mouse) into the tail vein 5 min before killing the animals; implantation sites were separated from interimplantation sites, and both sites were retained. For pregnant mice on day 5.5 onwards, implantation and interimplantation sites were visualized without dye injection.

For non-pregnant mice, the uterus was also collected from different stages of the estrous cycle: metestrus, diestrus, proestrus and estrus. The stages of the cycle were determined by analysis of vaginal smears (Rugh, 1994). For ovarian hormone treatments, the animals were first ovariectomized under anaesthesia with avertin, without regard to the stage of the estrous cycle (Rugh, 1994). The animals were allowed to rest for two weeks, then treated with daily subcutaneous injections (0.1 ml per mouse) of steroid hormones (Sigma Chemical Co., St Louis, MO, USA) for 3 days, as follows: 17β-estradiol (100 ng), progesterone (1 mg), or a combination of both hormones. The steroids were initially dissolved in minimal amounts of ethanol before dilution in peanut oil. Animals injected with oil alone served as controls. Mice were killed 24 h after the last injection.

### Northern Analysis

For Northern analysis, no attempt was made to separate the embryos from the decidua before day 8 of pregnancy, but for 8- and 11-day pregnant mice, embryos were separated from the uterine tissue. Total RNA was extracted from whole uteri or from pools of implantation or inter-implantation sites by the acid guanidinium thiocyanate-phenol-chloroform extraction (GTC) method (Chomczynski and Sacchi, 1987). RNA (10-15 µg) was denatured at 50°C for 60 min in 50% dimethylsulfoxide (DMSO) and 1M glyoxal, and the denatured RNA was fractionated by electrophoresis through a 1.2% agarose gel in 10 mM sodium phosphate buffer (pH 7.0) and transferred to positively charged nylon membranes (Hybond-N+, Amersham) by overnight capillary blotting in 5 x SSPE (1 x SSPE = 150 mM NaCl, 10 mM NaH₂PO₄, 1 mM EDTA, pH 7.4). Membranes were baked at 80°C for 2 h followed by 3 min UV cross linking. Transcript size was estimated by comparison with RNA size standards (Gibco-BRL, Gaithersburg, MD USA). A simplified filter paper sandwich blotting method (Jones and Jones, 1992; Nie *et al.*, 2000b) was used for the hybridization process at 42°C overnight, without a prehybridization step. The radio-labelled cDNA probes were generated by random primer labelling of 25 ng cDNA with [³²P]deoxy-CTP (50 µCi/reaction). Unincorporated nucleotides were removed with a MicroSpin S-200 HR column (AMRAD Pharmacia Biotech, Melbourne, Australia). Following hybridization, the blots were rinsed twice with 5 x SSPE at 37°C, then twice for 15 min each at 37°C with 2 x SSC/0.1% SDS (w/v) (1x SSC = 150 mM NaCl, 15 mM Na₃citrate, pH 7.4). In some cases, additional washes were also performed with 0.5 or 1 x SSC/0.1% SDS for 15 min at 60°C. To determine lane to lane loading variation, each blot was also probed with a mouse cDNA probe for glyceraldehyde-3-phosphate dehydrogenase (GAPDH) or 18S ribosomal RNA. Between hybridizations, blots were stripped by incubation at 80°C for 3 h in 1 mM EDTA / 0.1% SDS followed by rinsing in H₂O.

### RT-PCR and T/A Cloning

For reverse transcriptase-polymerase chain reaction (RT-PCR), 1 µg DNA-free total RNA was reverse-transcribed at 46°C for 1-1.5 h in 20 µl reaction mixture, using 100 ng random hexanucleotide primers and AMV reverse transcriptase (Boehringer-Mannheim, Nunawading, Vic., Australia) with the cDNA synthesis buffer. The PCR was performed in a total volume of 40 µl with 1-1.5 µl of the RT reaction, 1x PCR buffer, 20 µM dNTPs, 10 pmol forward and reverse primers and 2.5 units of Taq DNA polymerase (Boehringer-Mannheim), in 3 stages as follows:
(a) one cycle of an incubation for 5 min at 95°C, 1 min at 52°C-60°C, and 2 min at 72°C;
(b) 32 cycles with a denaturation for 45 sec at 95°C, annealing at 52°⁻C-60°C for 50 sec and extension at 72°C for 1 min; and
(c) incubation for 5 min at 72°C.

The PCR products were analysed on 1.5% agarose gel and stained with ethidium bromide. Bands of interest were cut out from the agarose gels, purified with the Qiaquick gel extraction kit (Qiagen Pty Ltd., Clifton Hill, Vic., Australia), cloned into a pGEM-T easy vector (Promega) according to the manufacturer's instructions and sequenced on an automated sequencer (Applied Biosystems, ABI Prism^{Tm}, 377 DNA Sequencer) using the ABI Prism BigDye terminator cycle sequencing ready reaction kit.

### Example 1 DDPCR Analysis and Identification of Clone 10.9 by Northern Blotting

To identify genes which are potentially critical for the initial process of embryo implantation in the mouse, we compared the uterine gene expression pattern of implantation and inter-implantation sites in the mouse uterus on day 4.5 of pregnancy, using the DDPCR technique. A few bands for which the intensities were different between the two sites were detected on DDPCR gels (Nie *et al.*, 2000b). One of these bands, band 10, was fully analysed, and is described herein.

DDPCR was performed as previously described (Nie *et al.*, 2000b) and was essentially as described originally by Liang and Pardee (1992, 1993). DNA-free RNA from the implantation and interimplantation sites was used as the template for the first-strand cDNA synthesis. The cDNA was then amplified by PCR using one random primer (10 mer) and one oligo-d_{T} anchored primer in the presence of ³³P-dATP. The PCR products were subsequently analysed on 6% high-resolution polyacrylamide/urea gel, and visualised by autoradiography.

Uterine mRNA expression on day 4.5 of pregnancy was compared between implantation sites and inter-implantation sites. The 80 PCR primer combinations (20 random 10mers combined with 4 oligo-dT anchored primers) used in the DDPCR analysis are shown in Table 2.

**Table 2**

| The 80 (4 x 20) primer combinations used in the DDPCR analysis | | | | | | | |
|---|---|---|---|---|---|---|---|
| 3' primers: Oligo-(dT) anchored primers, custom-made | | | | | | | |
| Primer | Code | Sequence | | | | | |
| 1 | T12MA | TTTTTTTTTTTT (G, A, C) A | | | (SEQ ID NO:1) | | |
| 2 | T12MC | TTTTTTTTTTTT (G, A, C) C | | | (SEQ ID NO: 2) | | |
| 3 | T12MG | TTTTTTTTTTTT (G, A, C) G | | | (SEQ ID NO: 3) | | |
| 4 | T12MT | TTTTTTTTTTTT (G, A, C) T | | | (SEQ ID NO: 4) | | |

| 5' Primers: 10 mers, from OPERON | | | | | | | |
|---|---|---|---|---|---|---|---|
| Primer | Code | Sequence | SEQ ID | Primer | Code | Sequence | SEQ ID |
| 1 | OPA-01 | CAGGCCCTTC | No. 5 | 11 | OPA-11 | CAATCGCCGT | No. 15 |
| 2 | OPA-02 | TGCCGAGCTG | No. 6 | 12 | OPA-12 | TCGGCGATAG | No. 16 |
| 3 | OPA-03 | AGTCAGCCAC | No. 7 | 13 | OPA-13 | CAGCACCCAC | No. 17 |
| 4 | OPA-04 | AATCGGGCTG | No. 8 | 14 | OPA-14 | TCTGTGCTGG | No. 18 |
| 5 | OPA-05 | AGGGGTCTTG | No. 9 | 15 | OPA-15 | TTCCGAACCC | No. 19 |
| 6 | OPA-06 | GGTCCCTGAC | No. 10 | 16 | OPA-16 | AGCCAGCGAA | No. 20 |
| 7 | OPA-07 | GAAACGGGTG | No. 11 | 17 | OPA-17 | GACCGCTTGT | No. 21 |
| 8 | OPA-08 | GTGACGTAGG | No. 12 | 18 | OPA-18 | AGGTGACCGT | No. 22 |
| 9 | OPA-09 | GGGTAACGCC | No. I3 | 19 | OPA-19 | CAAACGTCGG | No. 23 |
| 10 | OPA-10 | GTGATCGCAG | No. 14 | 20 | OPA-20 | GTTGCGATCC | No. 24 |

To avoid embryonic contamination, the embryos were removed from the implantation sites under light microscope visualization. After the DDPCR analysis, the differential display pattern was further verified by Northern blotting analysis, and cDNAs from the confirmed bands were sub-cloned into the pGEM-T vector (Promega, Madison, WI, USA) and sequenced manually.

On the DDPCR gel, band 10 was much more intense in interimplantation sites compared to implantation sites in all individual animals tested, as shown in Figure 1A. To verify that this band indeed represents gene(s) which are differentially expressed between the two sites, the cDNA products of band 10 were extracted from the DDPCR gel, re-amplified, and cloned into the pGEM-T vector, and Northern blot analysis was performed using the cloned inserts as probes. Among the 10 clones analysed, the cDNA of clone 10.9 specifically detected differential expression of mRNA between the two sites on day 4.5 of pregnancy on the Northern blot, with much higher mRNA levels present in interimplantation sites than in implantation sites; this is illustrated in Figure 1B. A 2.8 kb transcript was detected on this initial blot. This confirmed that clone 10.9 contained the cDNA representing the original expression pattern of band 10 on the DDPCR gel. Of the other clones analysed, clones 10.2 and 11.2 showed results similar to the DDPCR results.

### Example 2 Sequence Analysis of Clone 10.9

Band 10 resulted from the DDPCR amplification of day 4.5 interimplantation site mRNA with the following two primers: 5' primer, TCTGTGCTGG (OPA-14; SEQ ID NO:18) and 3' primer, T12MG (SEQ ID NO:3), whose sequences are set out in Table 2 above. After confirming that clone 10.9 contained the cDNA representing band 10, the nucleotide sequence of this clone was determined, and is set out in SEQ ID NO:25.

**Table 3**

| |
|---|
| The sequence of clone 10.9 (359 bp) derived from band 10 of DDPCR gel (SEQ ID NO:25) |
| (The underlined nucleotides represent the primers used during DDPCR amplification) |
| |

This sequence contained 359 nucleotides, and the ends of the sequence indeed contained the unique and expected primer sequences of TCTGTGCTGG at the 5' end and the reverse complementary sequence of T12MG at the 3' end (underlined in Table 3). This confirmed that the cDNA in clone 10.9 was the direct PCR product amplified from the specific primers applied during DDPCR amplification.

When compared to the GenBank database, no other sequences were found to be very homologous to clone 10.9, other than a few short expressed sequence tags(ESTs) from mouse uterus, mouse mammary gland, rat mast cell protein 6, rat PC-12 cells, and mouse skin, indicating that this clone represents a novel cDNA sequence. These comparisons are summarised in Table 4.

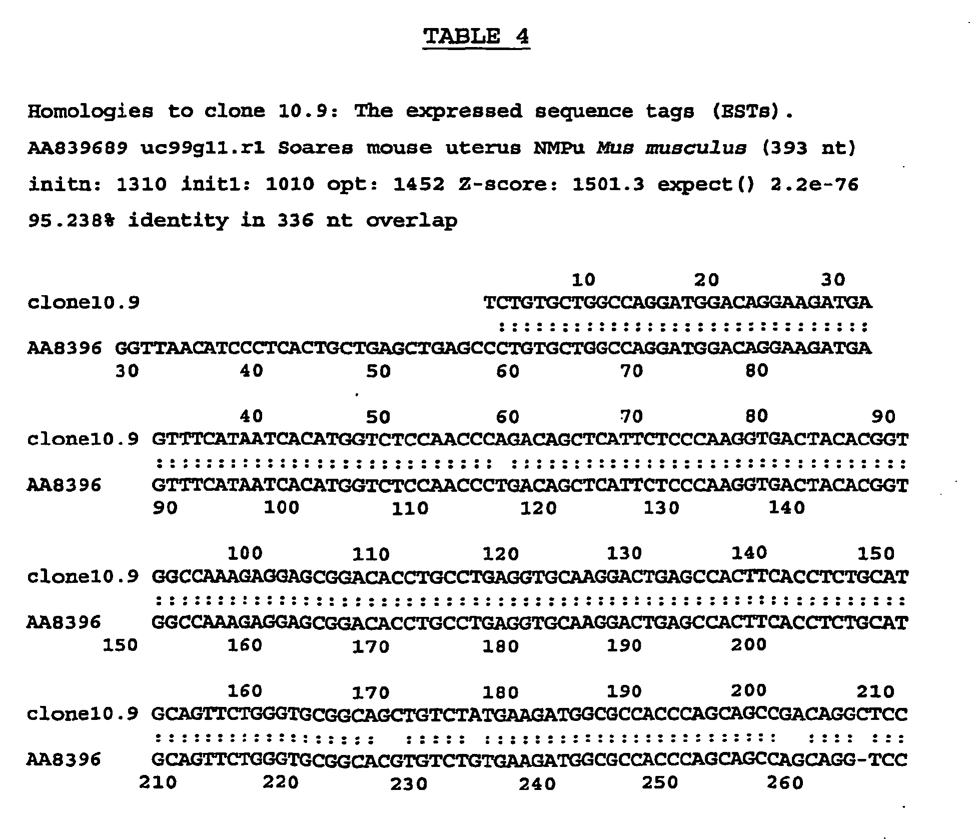

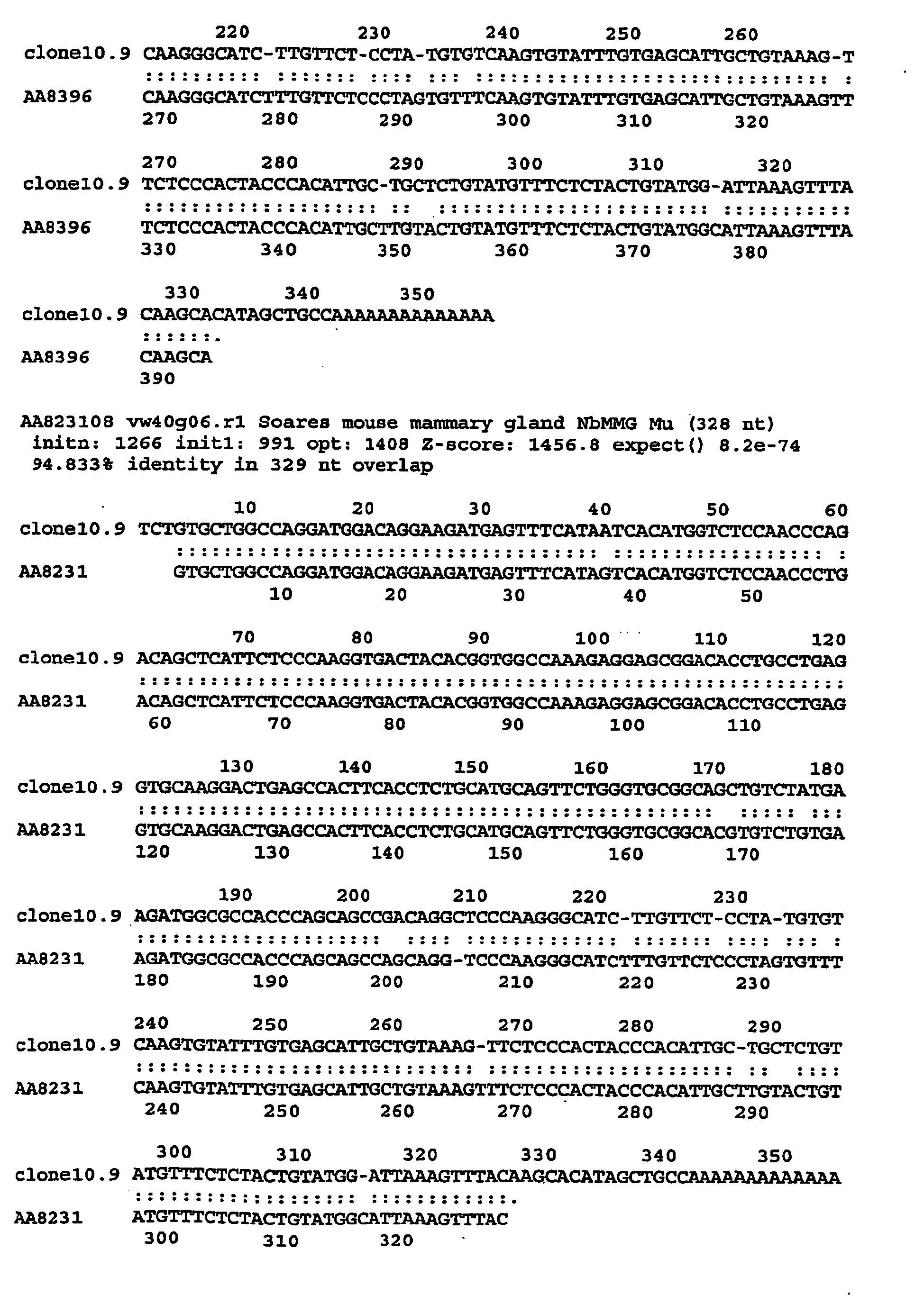

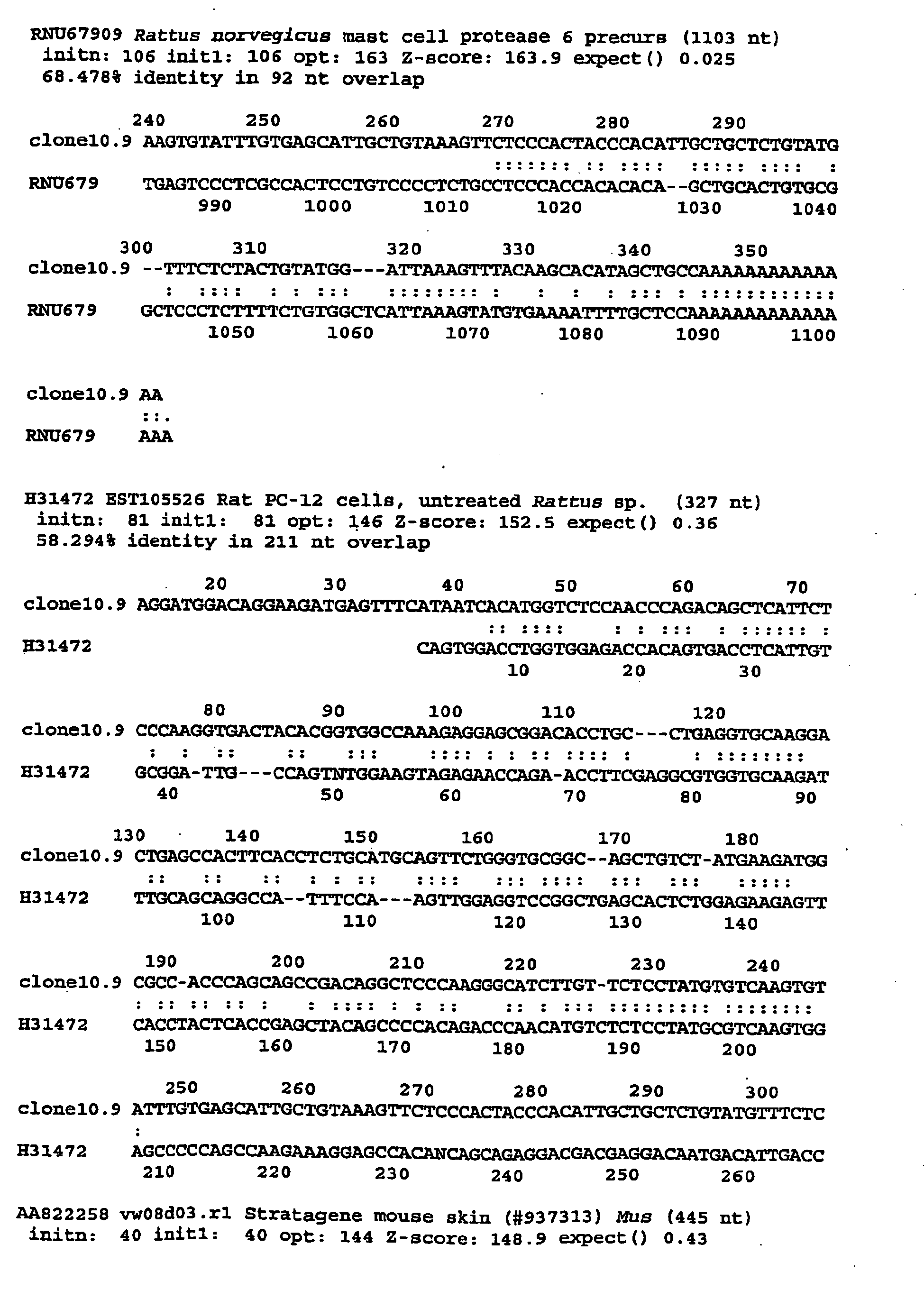

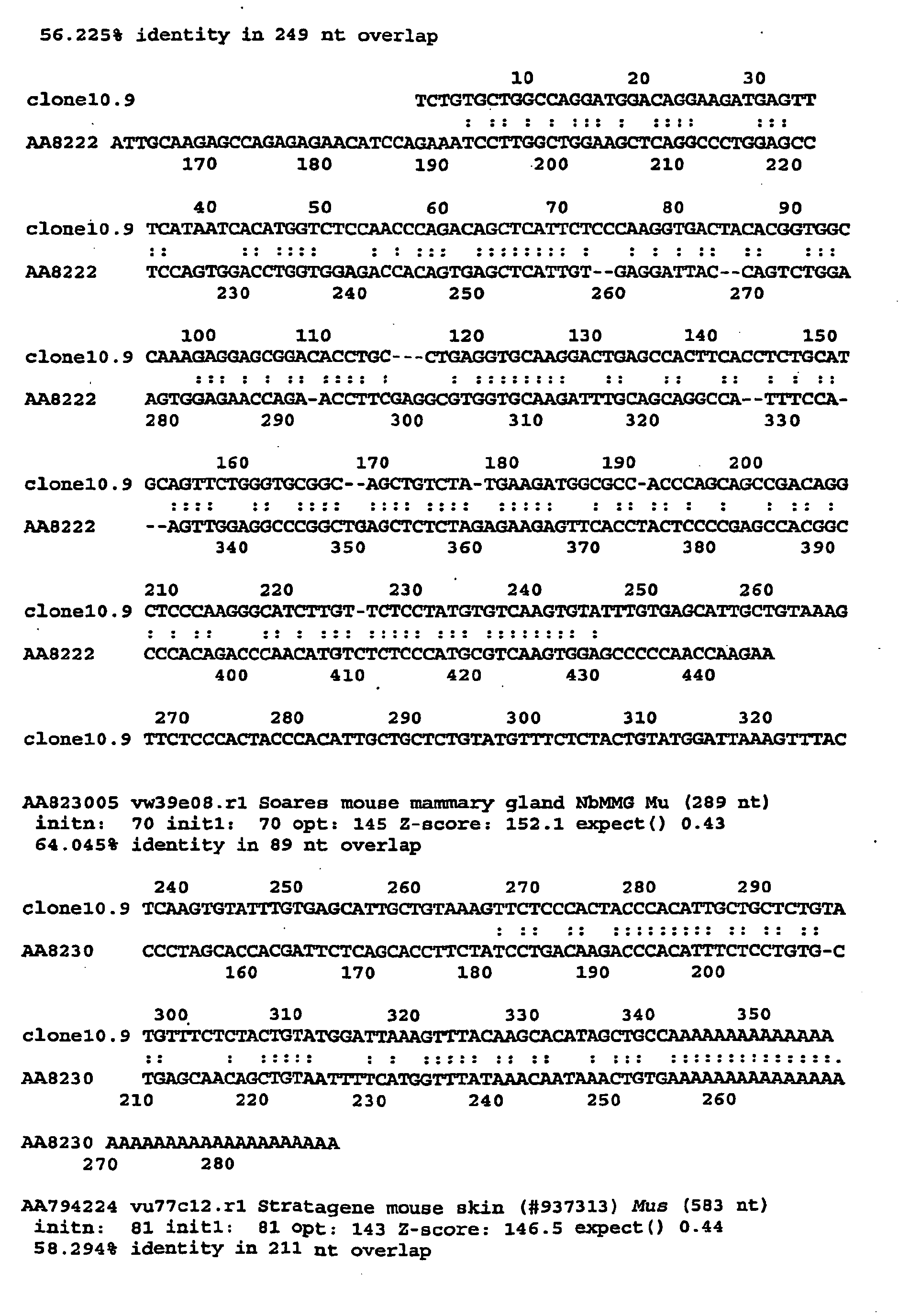

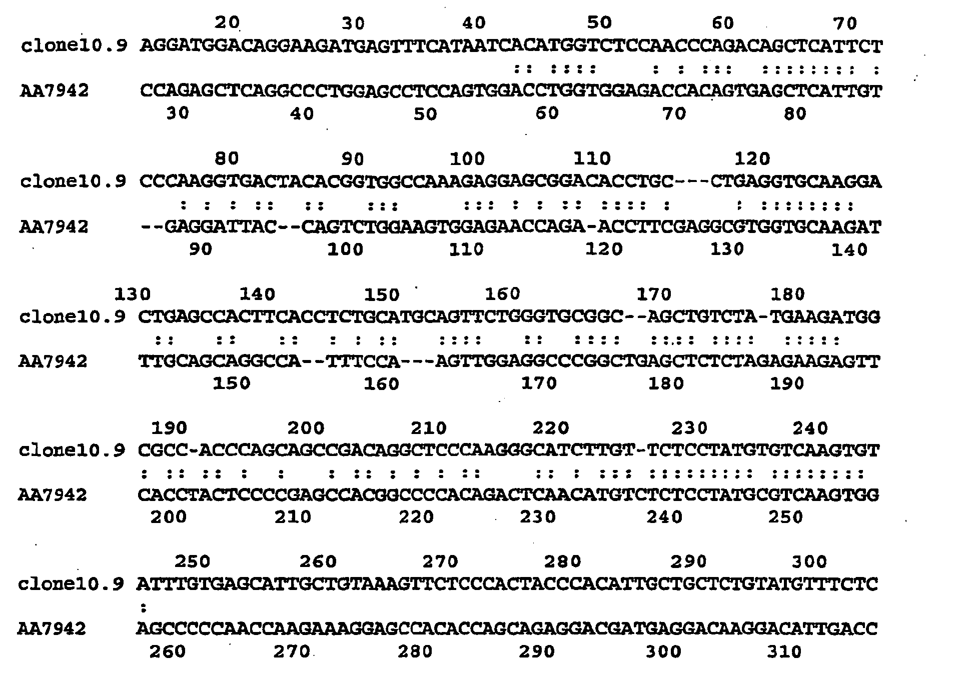

### Example 3 Cloning of the Full Length cDNA Sequence

In order to obtain the full length cDNA sequence represented by clone 10.9, a mouse uterine cDNA library (Clontech, Palo Alto, CA) was screened using radiolabelled clone 10.9 cDNA as a probe; this was prepared as described above for Northern analysis, using the standard method (Sambrook *et al.*, 1989).

Three clones were obtained; all of these appeared to lack the start codon, so 5' RACE was used in order to obtain the 5' end sequence. To obtain the full length cDNA sequence and to search for possible isoforms, standard 5' and 3' rapid amplification of cDNA ends (RACE) was also performed, using the 5'/3' RACE kit (Roche, Castle Hill, NSW, Australia).

The longest sequence obtained from these approaches contained 2450 nucleotides, and is shown in Figure 2 and SEQ ID NO:26. This sequence included an open reading frame of 1377 bp, with the start codon ATG being at nucleotide (nt) 127-129 and the stop codon TGA at nt 1504-1506. It also included a G/C-rich (72%) 5' untranslated region of 126 bp and a 3' untranslated region of 944 bp (Figure 2).

The open reading frame could be translated into an amino acid sequence of 459 residues (Figure 2 and SEQ ID NO:27). The predicted protein had a molecular mass of about 49 kDa, with a calculated isoelectric point of 7.08. The N-terminal end of the sequence contained a long stretch of hydrophobic region which may represent a signal peptide.

A comparison of the cDNA and deduced protein sequences with all entries in the GenBank and Swissprot databanks revealed that the most similar entries in the database were human and mouse HtrA. At the cDNA level, this sequence is 63% identical to the mouse (Accession No.: AF172994) and 65% to the human (2 entries, Accession No.: D87258 and Y07921) HtrA cDNA sequences. At the protein level, it is 56% identical to the mouse (Accession No.: AAD49422) and 58% to the human (2 entries, Accession Nos.: BAA13322 and CAA69226) HtrA proteins.

As noted for the human HtrA (Zumbrunn & Trueb, 1996), this protein also has a substantial similarity to the family of IGF-binding proteins. In particular, the 16 cysteine residues which are conserved in all IGF-binding proteins are present in this protein as well; thus it is expected that the N-terminal of this novel protein represents an IGF-binding domain. The C-terminal part of this protein is closely related to the mouse and human HtrA, which was found to be homologous to the HtrA/Do proteases from bacteria (Zumbrunn & Trueb, 1996). These HtrA proteins belong to a family of serine proteases which possess the amino acid sequence GNSGGAL (SEQ ID NO:28; in bacterial HtrA) or GNSGGPL (SEQ ID NO:29; in mammalian HtrA) in their active sites, and another TNAHV (SEQ ID NO:30) sequence in the vicinity of the active site (Zumbrunn & Trueb, 1996). Interestingly, the serine protease active site sequence GNSGGPL was found at position 309-315, and the additional TNAHV residues was located at position 194-198 in this novel protein as shown in Figure 2. Therefore, we believe that the novel protein represents a functional serine protease. We conclude that we have isolated a novel cDNA which codes for a serine protease with an IGF-binding motif.

### Example 4 Isolation of the human protease

Using a 785 bp probe (nucleotide 76-860 of the cDNA shown in Figure 2) derived from the mouse cDNA sequence described in Example 2 as a probe, a human multiple tissue expression array (MTE) (Clontech, Palo Alto, CA) was screened, and the heart was identified as one of the most strongly positive tissues. A human heart cDNA library, in which cDNAs were cloned unidirectionally into the Uni-ZAP XR vector (Stratagene Cat # 937257) was screened, using two probes derived from the mouse sequence. Probe 1 contained nucleotide 76-484 and probe 2 contained nucleotide 621-1540 of the mouse cDNA sequence shown in Figure 2. Three clones were obtained, and all contained the full open reading frames. Of these three clones, two were identical; the three clones were found to represent two different isoforms, and the two cDNA sequences are presented in Figure 3A (SEQ ID NO:31; long form, 2543 bp) and Figure 3B (SEQ ID NO:32; short form, 1953 bp) respectively. These two cDNAs code for two proteins: the long isoform codes for a protein of 453 amino acids and the short isoform codes for a protein of 357 amino acids, whose sequences are set out in Figure 4A (SEQ ID NO:33) and Figure 4B (SEQ ID NO:34) respectively.

These two isoforms are identical at the cDNA level, up to nt 1243 on the longer isoform and nt 1158 on the short isoform, as shown in Figure 5A. At the protein level, the short isoform is substantially smaller than the longer one, but otherwise they are exactly the same except for the few amino acids at the C-terminal ends, as shown in Figure 5B. It is considered that the two isoforms are derived from alternative splicing of the same primary RNA molecule.

When compared to the mouse sequences described herein, the human longer isoform is 79% identical at the cDNA level and 93% identical at the protein level to the mouse longer isoform, and the short isoform is 87% identical at the cDNA level and 92% identical at the protein level to the mouse short isoform. Therefore these human sequences are the true counterparts of the mouse sequences. However, when compared to the human HtrA sequences, the longer isoform is only 67% identical at the cDNA level and 61% identical at the protein level to the human HtrA, and the shorter form is 71% identical at the cDNA level and 65% identical at the protein level to the human HtrA. Therefore these newly cloned human sequences are quite different from those of the human HtrA.

As observed for the mouse sequences, the 16 cysteine residue IGF-binding motif and the serine proteases motifs GNSGGPL and TNAHV are also present in the human sequences; therefore these two human isoforms also represent serine proteases with IGF-binding domains.

### Example 5 Identification of Two Isoforms of the Mouse Enzyme

The possible existence of similar long and short isoforms to those demonstrated for the human enzyme in Example 4 was examined in the mouse. Sequence comparison between the human and mouse indicated that the mouse cDNA sequence shown in Figure 2 probably represents the longer isoform. On the assumption that the splicing characteristic in the mouse would be the same as that in the human, a possible splice site was located on the mouse cDNA sequence at around nt 1185 (as shown in Figure 2).

A forward primer (5' GGC ATC AAC ACG CTC AA 3' (SEQ ID NO:35), nt 1096-1112 of SEQ ID NO:26) upstream from this possible splicing site was designed, and 3' RACE was performed using this forward primer plus an Oligo d(T)-anchor primer (5' GAC CAC GCG TAT CGA TGT CGA CTT TTT TTT TTT TTT TTV 3' (SEQ ID NO:36), available from the 5'/3' RACE kit) and mRNA was isolated from day 10.5 placenta. Surprisingly, two bands with the sizes expected for the presumed two isoforms were indeed amplified (data not shown); however, the intensity of the shorter isoform band was much lower than that of the longer isoform one. This indicated that in the mouse, in addition to the cDNA cloned in Example 2 (SEQ ID NO:26), another isoform differing at the 3' end did exist in the mouse, and that the expression level of the longer isoform may be much higher than that of the short isoform.

These two 3' RACE products were subsequently cloned and sequenced, and it was confirmed that the longer one represented the 3' end of the cDNA sequence cloned in Example 2 (SEQ ID NO:26), and the shorter one represented the 3' end of a cDNA encoding another isoform.

In order to clone the full cDNA sequence of this shorter isoform and to confirm that the two isoforms are different only at the 3' end, a mouse uterine cDNA library specific to the pregnant uterus was constructed using mouse uterine tissues obtained on day 4.5 and 5.5 of pregnancy. Poly-(A)⁺ mRNA was isolated from total RNA of day 4.5-5.5 pregnant uterus using the PolyATract mRNA Isolation System (Promega). The resulting mRNA (5 µg) was used to construct a mouse cDNA library specific to the pregnant mouse uterus, using the ZAP Express cDNA synthesis and ZAP Express cDNA Gigapack III Gold Cloning Kit (Stratagene, La Jolla, CA, USA). This cDNA library was screened using a short isoform-specific sequence (476bp) derived from the 3'RACE cloning as a probe. The sequence of the probe is set out in Table 5 (SEQ ID NO:37).

**TABLE 5**

| |
|---|
| A 476 bp probe used as short-isoform specific sequence (SEQ ID NO:37) |
| |

Several clones were analysed, and the full length cDNA sequence was obtained; this is presented in Figure 6A (SEQ ID NO:38). This shorter isoform cDNA contained 1897 nt compared to 2450 nt for the longer isoform; the two sequences are exactly the same until nt 1195, but beyond this point they are very different, indicating that they are indeed derived from alternative splicing. In the open reading frame, the short isoform cDNA contained a stop codon TGA at nt 1216-1218 (Figure 6A) instead of nt 1504-1506 in the long isoform (Figure 2); therefore the short isoform cDNA codes for a protein of 363 amino acids, instead of 459 for the long isoform cDNA. The protein sequence is shown in Figure 6B (SEQ ID NO:39).

However, all the characteristics such as the cysteine residues, active serine protease sites etc described earlier for the longer isoform are presented by the short isoform (Figure 6C), indicating that although the shorter protein is still an active serine protease, its function or sub-cellular location may differ. The difference between the two may also lie in the substrate specificity or sub-cellular localisation.

### Example 6 Determination of mRNA Expression in the Mouse Uterus During Early Pregnancy

After determination of the full cDNA sequence encoding the novel protein, additional Northern analyses were performed to systematically determine the expression pattern of this gene in the uterus in relation to the time of implantation and early pregnancy. A 785 bp cDNA sequence (SEQ ID NO:40; nt 76-860 of the longer isoform cDNA, shown in Figure 2) representing the common region of the two isoform cDNAs was used as a probe to detect both isoforms on the same gel; the sequence of this probe is set out in Table 6.

**TABLE 6**

| |
|---|
| A 785 bp sequence common to both mouse isoform (SEQ ID NO:40) used as a probe |
| |

Total RNA from the uterus of non-pregnant mice (estrus) and pregnant mice at the initial stage of implantation (day 4.5 of pregnancy) through to fully established implantation and placentation (day 10.5 of pregnancy) was analysed, and the results are shown in Figure 7. Very low expression was observed in non-pregnant mice, and a marginally higher level was seen on day 3.5 of pregnancy. Around days 4.5 and 5.5 of pregnancy the expression was still quite low, but it was relatively higher in the interimplantation sites compared to the implantation sites. Around day 6.5 of pregnancy, similar levels of expression were detected in both implantation and interimplantation sites. Beyond day 6.5, a dramatic up-regulation of this gene occurred, and by day 8.5-10.5, the mRNA level was several fold higher than that detected in the interimplantation sites on day 4.5-5.5. Dissection of the maternal-fetal unit on day 10.5 revealed that this up-regulation mainly occurred in the placental tissues. Interestingly, the band pattern detected by these analyses showed that only the longer isoform was expressed, and that the expression of the short form was at a level below the detection sensitivity of the Northern blot technique.

Northern blotting studies using human tissues sampled at different stages of the endometrial cycle and in early pregnant tissues showed the expression of the novel protease, and of HtrA, with different patterns of expression being observed for these two enzymes. A 384bp probe was used for this experiment, and its sequence (SEQ ID NO:41) is set out in Table 7.

**TABLE 7**

| |
|---|
| A 384bp human sequence (SEQ ID NO:41) used as a probe |
| |

Reverse transcriptase polymerase chain reaction (RT-PCR) also detected the expression of HtrA and of both isoforms of PRSP in the endometrium across the human endometrial cycle, in early and late human pregnant tissues (placenta and decidua), in pre- and post-menopausal ovary, ovary, heart and skeletal muscle, as shown in Figure 12.

### Example 7 mRNA Expression During the Estrous Cycle

The influence of the estrous cycle on the expression of this gene in the non-pregnant uterus was examined by determining the level of its mRNA by Northern analysis. The study utilised 16 individual mice at different stages of the cycle (metestrus, diestrus, proestrus and estrus), grouped to represent 4 cycles, and results for two cycles are shown in Figure 8A. The cDNA sequence common to both isoforms (SEQ ID NO:40) was used as a probe, as described in Example 6. In general, the expression level was low at estrus and proestrus, and increased during metestrus and diestrus. These results indicated a possible influence of the ovarian hormones estrogen and progesterone on the expression of this gene in the uterus. However, the absolute level of mRNA during the estrous cycle is equivalent to or lower than that detected in the interimplantation sites on day 4.5 of pregnancy (Figure 4A); thus it would be much lower than that expressed by the placenta later on in pregnancy. Again only the longer isoform was detected.

### Example 8 Effects of Progesterone and Estradiol in Ovariectomized Mice

To verify that the ovarian steroids can regulate the expression of the novel gene in the uterus, estradiol and/or progesterone were administered to ovariectomized mice and the expression level was determined by Northern analysis. A total of 16 animals, consisting of four replicate groups, was used for this study. Very similar patterns of expression were observed in all four groups, and results for one group are shown in Figure 8B. The control ovariectomized mice, which were treated with vehicle (oil) alone, showed very little expression. Animals treated with estradiol or progesterone alone had the same level of expression as the controls, while the animals treated with both steroids showed higher expression levels. This indicates that the ovarian hormones do regulate the expression of the novel gene, but that both estrogen and progesterone are required to induce its expression.

### Example 9 Tissue Distribution of mRNA Expression

Multi-tissue Northern analysis was performed to investigate the tissue distribution of mRNA expression of the protease. As shown in Figure 9, the protease was not widely expressed in mice. When an equal amount of total RNA was compared, the day 10.5 placenta showed the highest level of expression; this placental level is several fold higher than that seen in the interimplantation sites on day 4.5 of pregnancy. Of the 12 tissues tested, apart from the uterus, the testis, ovary and heart had moderate expression, while muscle and lung had low expression. On this Northern blot a faint band representing the short isoform was detected in the placenta, but the level was very low.

The human MTE array was probed with a sequence common to both isoforms, using the sequence used in Example 6 (SEQ ID NO:41). The results, shown in Figure 10, indicated that heart, ovary, and uterus all expressed the novel protease. However, the expression pattern was quite different when HtrA was probed on the same MTE, indicating that these two enzymes are distributed quite differently.

Probing a commercial Northern blot (Clontech, Palo Alto, CA) with the same probe (SEQ ID NO:41) also identified the expression of the two isoforms in human placenta, heart and other tissues, including lung, liver, kidney and skeletal muscle tissue.

### Example 10 Southern Analysis of Mouse Genomic DNA

Mouse genomic DNA was isolated from the uterus and the kidney, and the DNA was subjected to Southern analysis. Total genomic DNA was isolated from non-pregnant uterus and the kidney using the DNeasy Tissue Kit (Qiagen). A total amount of 10 µg was digested separately with an excess of several restriction endonucleases (Tag 1, Hind III, ECoRI BamHI) at 37°C for 14 hours and fractionated on 0.8% agarose gel. The DNA was then blotted on to positively-charged nylon membranes (Hybond-N, Amersham) using the standard Southern blotting procedure (Sambrook *et al.*, 1989) and probed with radiolabelled cDNA as described for the Northern analysis.

Similar results were obtained for the two tissue types; thus only the result with the uterus will be discussed. Figure 11 shows the results of Southern analysis of mouse genomic DNA from non-pregnant uterus digested separately with TaqI, HindIII, EcoRI and BamHI and probed with a radiolabelled cDNA probe representing both isoforms (SEQ ID NO:40). In all cases, the digestion pattern was quite simple, indicating that this gene is represented by a single copy in the genome.

### Example 11 Detection of PRSP and HtrA in Cycling and Pregnant Human Endometrium

Semiquantitative Reverse transcriptase polymerase chain reaction (RT-PCR) Southern blot analysis was performed to investigate the mRNA expression of PRSP (long and short forms) and HtrA in human endometrium during the menstrual cycle and early pregnancy. Samples of human heart and skeletal muscle were used as positive controls.

Primers used for long form PRSP were:
Upper primer: 5' ATG CGG ACG ATC ACA CCA AG 3' SEQ ID NO:42
Lower Primer: 5' CGC TGC CCT CCG TTG TCT G 3' SEQ ID NO:43
An expected band of 337 bp was detected.

Primers used for the short form PRSP were:
Upper primer: 5' GAG GGC TGG TCA CAT GAA GA 3' SEQ ID NO:44
Lower Primer: 5' GCT CCG CTA ATT TCC AGT 3' SEQ ID NO:45
An expected band of 320 bp was detected.

Primers used for HtrA were:
Upper primer: 5' AAA GCC ATC ACC AAG AAG AAG TAT 3' SEQ ID NO:46
Lower Primer: 5' TCC TCA TCC GTC ATC CAC 3' SEQ ID NO:47
An expected band of 384 bp was detected.

The results are shown in Figure 12. Both the short and long form mRNA of PRSP were detected in the human endometrium during the menstrual cycle. They were also expressed in the first trimester decidua and placenta. HtrA was also detected in all samples. However, the expression pattern of PRSP was different from that of HtrA.

### Example 12 In Situ Hybridization of mRNA in the Uterus of Mice During Early Pregnancy

The cell types which express the mRNA of this protease in the uterus were identified by in situ hybridization. Sense and anti-sense digoxygenin (DIG)-labelled RNA probes for the novel protease having the sense sequence set out in Table 8 and its anti-sense equivalent were generated using the DIG RNA Labeling kit (Boehringer Mannheim), and the concentrations determined according to the manufacturer's instructions.

**TABLE 8a**

| |
|---|
| A 781 bp mouse sequence (SEQ ID NO:48) used as a probe for in situ hybridization. |
| |

Five micron sections of formalin-fixed paraffin-embedded tissues were subjected to in situ hybridization as described by Komminoth, (1992). Some sections were counterstained with Mayer's hematoxylin.

Preliminary data, shown in Figure 13, indicated that during the period of implantation on days 4.5 and 5.5 of pregnancy, the mRNA encoding the novel enzyme was predominantly localised in the glandular cells in the interimplantation sites and in the decidual cellsin the implantation sites.

In subsequent experiments, the results of which are shown in Figures 14 to 16, the 344 bp sense probe whose sequence is shown in Table 8b and its antisense equivalent were used as probes.

**TABLE 8b**

| |
|---|
| A 344 bp mouse sequence (SEQ ID NO:49) used as a probe for in situ hybridization. |
| |

Figures 14 to 16 show the results of *in situ* hybridization detection of PRSP mRNA mouse uterus on days 5.5, 8.5 and 10.5 of pregnancy. For studies of human tissues, a 396 bp probe for a sequence common to both isoforms of human PRSP mRNA was used for *in situ* hybridization studies, the results of which are shown in Figures 17, 18 and 19. The sequence of this probe is shown in Table 8c.

**TABLE 8c**

| |
|---|
| A 396 bp human sequence (SEQ ID NO:50) used as a probe for in situ hybridization |
| |

Figure 17 shows the results of in situ hybridization detection of PRSP mRNA in cycling human endometrium at day 9 of the menstrual cycle. Figures 18 and 19 show the results of detection of PRSP mRNA in cycling rhesus monkey uterus on day 10 after ovulation, and in pregnant rhesus monkey uterus (implantation site) on day 28 of pregnancy, respectively.

### Example 13 Antibodies Directed against the Novel Enzyme

Antibodies against the novel protease and against HtrA were produced using conventional methods. Sheep were immunized with peptides derived from the mouse protein. The following peptides were synthesised using conventional solid phase synthetic methods, and used as antigens:
1. Amino acids 133-142; sequence PSGLHQLTSPC (SEQ ID NO:51)
2. Amino acid 116-126; sequence ALQVSGTPVRQC (SEQ ID NO:52)
3. A sequence common to both isoforms; amino acids 313-324 sequence GPLVNLDGEVIGC(SEQ ID NO:53)
4. HTrA; sequence ISINGQSVVTANC (SEQ ID NO:54)

Peptides 1-3 are from the mouse PRSP sequence, which is highly homologous to that of the human PRSP protein. It will be appreciated that other peptides could also be used.

An additional cysteine was added at the C-terminal end of each peptide to allow conjugation. The peptides were conjugated to diptheria toxoid, and the conjugated protein homogenized in an adjuvant comprising QuilA/DEAE-Dextran/Montanide 888, as described in Prowse (2000) prior to each injection. Sheep were immunized with the material at 4 weekly intervals for 3 or more injections, and bled between 1 and 2 weeks following the second and subsequent injections. The immunisation scheme is illustrated in Figure 20.

The presence of anti-PRSP antibodies in the sheep serum following immunisation against specific peptides of PRSP was examined by dot blot. Peptides were dotted and dried onto Hybond-P^{™} membranes (Amersham Life Sciences). After blocking the non-specific binding sites with 5% (w/v) skim milk powder in TBS with 0.1% Tween 20 for 1 h, blots were incubated for 1 h at room temperature with a 1:2,000 dilution of serum. Blots were then incubated with horseradish peroxidase-labelled donkey anti goat/sheep IgG (Silenus) diluted to 1:20,000. All antibody dilutions were in 5% (w/v) skim milk powder in TBS with 0.1% Tween 20. Blots were developed by chemiluminescence (ECL Plus system, Amersham). As a negative control, pre-immune serum from the same animal was used and a non-related peptide was tested on each blot.

In addition, total IgG was prepared by ammonium sulphate precipitation following capryllic acid treatment of whole serum. The presence of specific antibodies in the total IgG was also examined by dot blot.

Results for antibodies raised against peptide (2) aa 116-126 are shown in Figure 21. The presence of specific antibodies in both the whole sheep serum and in total IgG prepared from the serum was demonstrated by specific reactions with the spots containing the specific peptides of PRSP. The specificity of the antibodies was further demonstrated by the following evidence:
(1) no reaction was detected with pre-immune serum or total IgG (at the same concentration as the antibody) prepared from the pre-immune serum;
(2) no reaction was detected on spots containing irrelevant peptides of equivalent size;
(3) a dose-dependent reaction was detected with serial dilution of the specific peptides.

### Example 14 Western blotting studies of human and mouse tissues

Specific IgG was further purified from the total IgG (ammonium sulphate precipitate) by affinity purification using a HiTrap affinity column (Amersham Pharmacia Biotech). The expression of PRSP protein in the mouse and human uterus was detected with the affinity purified antibodies by Western blot. Proteins were extracted from one sample of human endometrium on day 25 of the menstrual cycle, one sample of non-pregnant mouse uterus and one mouse placenta on day 10.5 of pregnancy. Weighed tissue was homogenised in 6% SDS, 0.14M Tris (pH 6.8) and 22.4% glycerol (2ml per 100mg of tissue) with a protease inhibitor cocktail (Calbiochem, Croyden, Australia; 5µl per 100mg of tissue). The homogenate was then passed sequentially through 21, 23 and 25 gauge needles followed by centrifugation at 14,000g at 4°C for 15 min. 15µg of total protein from each supernatant, together with molecular weight markers (Kaleidoscope prestained standards; Biorad) were subjected to SDS-PAGE on a 12% gel under reducing conditions. The proteins were transferred to Hybond-P^{™} membranes (Amersham Life Sciences, Sydney). After blocking non-specific binding sites with 5% (w/v) skim milk powder in TBS with 0.1% Tween 20 for 1 h, blots were incubated for 1 h at room temperature with 100 µg/ml of affinity-purified IgG in 5% (w/v) skim milk powder in TBS with 0.1% (v/v) Tween 20. Blots were then incubated with horseradish peroxidase-labelled donkey anti goat/sheep IgG (Silenus) diluted to 1: 20,000 and developed by chemiluminescence (ECL Plus system, Amersham). The presence of PRSP protein in the serum of pregnant women was also detected by Western blot analysis of 2 µl serum following TCA precipitation.

As shown in Figure 22, Western blot analysis detected the expression of PRSP protein in human endometrium and mouse uterus, indicating the presence of PRSP protein in tissues where its mRNA was detected. The bands detected correlated well with the anticipated size of the protein in both the human and mouse. In the human, two bands corresponding to the two isoforms of PRSP were detected, indicating the expression of both isoforms of PRSP protein. This agrees very well with the mRNA data, where both the long and short form of PRSP mRNA was detected in the human endometrium. In the mouse, only one form of PRSP and much higher expression was detected in the placenta on day 10 of pregnancy, compared with the non-pregnant uterus. This is consistent with the mRNA expression data where an abundant level of only the long form of PRSP was detected in the pregnant uterus.

As shown in Figure 23, Western blot analysis also detected PRSP protein in the serum of pregnant women. The origin of this protein is considered to be the developing placenta during pregnancy. Thus the maternal serum profile of PRSP during pregnancy may be associated with placental development and function, and it is anticipated that the serum profile of PRSP might provide a marker for predicting placenta-related complications of pregnancy.

### Example 15 Expression of PRSP during implantation and gestation

Northern analysis of PRSP mRNA in the fetus, placenta and uterus during placentation and later gestation was carried out. Two Northern blots (RNWAY Laboratories) containing 2 µg of poly A⁺ RNA isolated from
(1) mouse placenta from day 10.5 to 18.5 pregnancy and
(2) mouse fetus from day 4.5 to 18.5 pregnancy were analysed.

The 785 bp cDNA sequence described in Example 6, representing the common region of the two isoform cDNAs of mouse PRSP, was used as a probe.

As shown in Figure 24A, expression of PRSP mRNA was detected in all placental samples, with the highest expression on day 10.5 of pregnancy. The level of expression decreased from day 14.5, and reached relatively low levels on day 18.5.

High expression of PRSP mRNA was detected in the fetus on day 7.5, 8.5 and 9.5 of pregnancy, as shown in Figure 24B. However, it should be noted that on these days the fetal sample includes the fetus, its developing placenta and the maternal deciduum, which is a mass of uterine decidual cells enclosing a single embryo. Thus the high expression detected on these days might reflect the expression in the fetus, the developing placenta and the deciduum. It is clear that the expression in the fetus before day 7.5 and after day 9.5 of pregnancy was minimal.

### Example 16 Northern analysis of PRSP in a range of human tissue

A human multi-organ Northern blot (Clontech) containing 1 µg of poly A⁺ RNA isolated from each of a range of human tissues was probed with a 457 bp cDNA sequence representing the common region of the two isoform cDNAs of human PRSP. The sequence of this probe is set out in Table 9.

**TABLE 9**

| |
|---|
| The 457 bp sequence (SEQ ID NO:55) common to both isoforms of human PRSP mRNA used as a probe for Northern blotting |
| |

Strong positive signals were detected in the heart, skeletal muscle and placenta. Lung, small intestine and kidney showed low expression while liver, thymus, colon and brain showed minimal expression. No expression was detected in the peripheral blood leukocytes and the spleen. The transcript sizes detected were around 2.4 kb. It is very interesting to note that two bands, representing the two isoforms of PRSP mRNA, were detected in the placenta, heart, skeletal muscle and kidney. The long form was predominant in the lung and small intestine, and the short form was predominant in the brain.

### Example 17 Northern analysis of PRSP mRNA in the first trimester placenta and decidua

Total RNA was isolated from first trimester pregnant human decidua and placenta, and the expression of PRSP mRNA was analysed by Northern blotting. The same 457 bp cDNA sequence as that used in Example 16, representing the common region of the two isoform cDNAs of human PRSP, was used. The results are shown in Figure 26. Strong positive signals were detected in both the placenta and decidua. Two bands of approximately 2.4 kb, representing the two isoforms of PRSP mRNA, were detected in all samples.

### Example 18 Expression of the Novel Serine Protease

The mature human protease is expressed as a fusion protein in vitro in mammalian cells such as Chinese hamster ovary or human embryonic kidney 293 cells. Initially, the protein is expressed without a tag, and the supernatant/cytoplasmic proteins tested for serine protease activity. Subsequently, the fusion protein is designed to contain a polyhistidine (His₆) sequence tag at the C-terminus for rapid purification on ProBond resin and detection with an anti-His antibody. If necessary to retain bioactivity of the protein, the tag may be cleaved using enterokinase sites included in the fused protein.

### Discussion

The present investigation aimed to identify and characterise genes which are uniquely regulated at the sites of embryo implantation in mouse uterus, using the technique of RNA differential display (DDPCR). We applied the technique of DDPCR to search for genes which are differentially regulated between implantation and interimplantation sites in the mouse uterus on day 4.5 of pregnancy, when the uterus shows the first morphological changes associated with pregnancy. We reasoned that up- or down-regulation of these genes would be potentially important for conversion of the uterus from the non-receptive to the receptive state.

We have isolated a cDNA coding for a novel mouse protein which is differentially expressed between the implantation and interimplantation sites in the uterus on day 4.5 of pregnancy. We detected several bands exhibiting different expression between the two sites, one of which we identified as a novel serine protease. The cDNA encoding the novel protease was isolated, and this cDNA was used to isolate cDNA encoding the corresponding human protease.

Initially the mouse protease was found to be expressed only in small amounts, mainly in the interimplantation sites during the period of embryo implantation (days 4.5-5.5 of pregnancy). Interestingly, the expression was up-regulated around day 6.5, when placentation initiates; by day 10.5, when the placenta is essentially fully formed, the mRNA level was several fold higher than that on day 6.5, and this high expression was primarily localised to the placenta and decidua.

Structurally the novel protease is related to both human and mouse HtrA, the mammalian homologue of the E. coli heat shock endoprotease, HtrA. However, there is only about 50% homology between the mouse protein and human HtrA. At the N-terminal end, the novel protein has an IGF-binding domain, which may modulate its protease activity. Given the importance of the IGF system in the implantation and placentation processes during pregnancy, this novel serine protease may represent one of the proteases which regulates the availability of IGFs by actions on one or more components of the IGF-IGFBP system; hence it may be essential for the formation and function of the placenta during pregnancy.

In the mouse, although two isoforms were found, only the longer one was expressed to any significant extent; the expression of the short isoform was very low. Further attempts are being made to detect it by using the short form-specific sequence as a probe. The significance of the presence of two isoforms and the expression pattern of the protein in placenta at later gestational stages are also being investigated.

The novel protein is expressed in the mouse uterus from day 3.5 of pregnancy, and is mainly localised in the glands in the interimplantation sites around the period of embryo implantation (day 4.5 to 5.5). The expression pattern changed from day 6.5, at which time the implantation sites also started to express the gene; this may be because the initiation of the placentation process occurs at around this time, and indicates that the gene is involved in the placentation process from the outset. On day 8.5-10.5, when the placenta is being actively formed, the expression was dramatically up-regulated.

On the basis of the basic protein structure and putative domains predicted for this protease, its observed expression pattern in the uterus, and the known involvement of the IGF system in pregnancy and in the formation and function of the placenta, the protease of the invention may be very important in the determination and control of the availability of the active IGFs at the molecular level. Thus it may be an essential protein for the success of placentation and pregnancy.

In the human, two isoforms of PRSP were also found, and both forms were expressed in the endometrium and first trimester decidua and placenta; neither isoform was dominant. PRSP was also detected in the serum of pregnant women.

The genomic structure of PRSP gene was also analysed. The PRSP gene was localised on chromosome 5 in the mouse and on chromosome 4 in the human. In both species, the PRSP gene contains 10 exons. In both the human and mouse, the long isoform of PRSP protein was found to result from transcribing all exons except exon 7, and the short isoform protein was found to result from utilising exons 1-7.

### REFERENCES:

Abrahamsohn PA and Zorn TMT Implantation and decidualization in rodents. J Exp Zool 1993; 266: 603-628.
Balbas P, Bolivar F. Design and construction of expression plasmid vectors in Escherichia coli. Methods Enzymol. 1990;185:14-37.
Ballance DJ, Buxton FP, Turner G. Transformation of Aspergillus nidulans by the orotidine-5'-phosphate decarboxylase gene of Neurospora crassa. Biochem Biophys Res Commun. 1983 Apr 15;112(1):284-9.
Barnes D, Sato G. Methods for growth of cultured cells in serum-free medium. Anal Biochem. 1980 Mar 1;102(2):255-70.
Bayer EA, Wilchek M. Protein biotinylation. Methods Enzymol. 1990;184:138-60.
Beach and Nurse, Nature. 1981, 290:140-142
Birnsteil et al., Cell. 1985, 41:349-359.
Bolivar et al., Gene. 1987, 2:95-113.
Bottenstein J, Hayashi I, Hutchings S et al., The growth of cells in serum-free hormone-supplemented media. Methods Enzymol. 1979;58:94-109.
Brodeur et al., In: Monoclonal Antibody Production Techniques and Applications, Marcel Dekker, Inc., New York, 1987, pp.51-63.
Brown EL, Belagaje R, Ryan MJ et al., Chemical synthesis and cloning of a tyrosine tRNA gene. Methods Enzymol. 1979;68:109-51.
Bruggermann et al., Year in Immunology 1993; 7:33.
Carter P. Improved oligonucleotide-directed mutagenesis using M13 vectors. Methods Enzymol. 1987;154:382-403.
Caruther et al., Meth. Enzymol. 1985, 154:287-313.
Case ME, Schweizer M, Kushner SR et al., Efficient transformation of Neurospora crassa by utilizing hybrid plasmid DNA. Proc Natl Acad Sci U S A. 1979 Oct;76(10):5259-63.
Chomczynski P and Sacchi N Single-step method of RNA isolation by acid guanidium thiocyanate-phenol-chloroform extraction. Anal.Biochem.1987; 162: 156-159.
Colbere-Garapin F, Horodniceanu F et al., A new dominant hybrid selective marker for higher eukaryotic cells. J Mol Biol. 1981 Jul 25;150 (1):1-14.
Craik CS, Largman C, Fletcher T et al., Redesigning trypsin: alteration of substrate specificity. Science. 1985 Apr 19; 228 (4697) :291-7.
Cregg et al., Bio/Technology. 1987, 5:479-485.
Creighton In: Proteins: Structure and Molecular Properties (Ed: W.H. Freeman & Co). 1983, pp.79-86.
Cunningham BC, Wells JA. High-resolution epitope mapping of hGH-receptor interactions by alanine-scanning mutagenesis. Science. 1989 Jun 2;244 (4908) :1081-5.
Das SK, Lim H, Paria BC et al. Cyclin D3 in the mouse uterus is associated with the decidualization process during early pregnancy. J.Mol.Endocrinol.1999; 22: 91-101.
David GS, Reisfeld RA. Protein iodination with solid state lactoperoxidase. Biochemistry. 1974 Feb 26;13(5):1014-21.
deBoer et al., Proc. Natl. Acad. Sci. USA. 1983, 80:21-25.
Depicker A, Stachel S, Dhaese P et al., Nopaline synthase: transcript mapping and DNA sequence. J Mol Appl Genet. 1982;1(6):561-73.
Dodson et al., Nuc. Acids Res. 1982, 10:2625-2637.
Engels et al.,, Agnew. Chem. Int. Ed. Engl. 1989, 28:716-734.
Fitch et al.; Proc. Nat. Acad. Sci. USA. 1983, 80:1382-1386
Emr SD. Heterologous gene expression in yeast. Methods Enzymol. 1990;185:231-3.
Frohman MA, Dush MK, Martin GR. Rapid production of full-length cDNAs from rare transcripts: amplification using a single gene-specific oligonucleotide primer. Proc Natl Acad Sci U S A. 1988 Dec;85(23):8998-9002.
Graham FL, Smiley J, Russell WC et al., Characteristics of a human cell line transformed by DNA from human adenovirus type 5. J Gen Virol. 1977 Jul;36 (1) :59-74.
Goeddel DV, Shepard HM, Yelverton E, Leung D, Crea R, Sloma A, Pestka S. Synthesis of human fibroblast interferon by E. coli. Nucleic Acids Res. 1980 Sep 25;8(18):4057-74.
Goeddel DV, Heyneker HL, Hozumi T, Arentzen R, Itakura K, Yansura DG, Ross MJ, Miozzari G, Crea R, Seeburg PH. Direct expression in Escherichia coli of a DNA sequence coding for human growth hormone. Nature. 1979 Oct 18;281(5732):544-8.
Goeddel DV, Heyneker HL, Hozumi T et al., Media and growth requirements. Methods Enzymol. 1979;58:44-93.
Ham RG, McKeehan WL. Media and growth requirements. Methods Enzymol. 1979;58:44-93.
Fierrera-Estrella L, Van den Broeck G, Maenhaut R et al., Light-inducible and chloroplast-associated expression of a chimaeric gene introduced into Nicotiana tabacum using a Ti plasmid vector. Nature. 1984 Jul 12-18;310(5973):115-20.
Higuchi et al., In: PCR Protocols, Academic Press, 1990; pp.177-183.
Hitzeman RA, Clarke L, Carbon J. Isolation and characterization of the yeast 3-phosphoglycerokinase gene (PGK) by an immunological screening technique. J Biol Chem. 1980 Dec 25;255(24) :12073-80.
Hoogenboom HR, Winter G. By-passing immunisation. Human antibodies from synthetic repertoires of germline VH gene segments rearranged in vitro. J Mol Biol. 1992 Sep 20;227(2):381-8.
Horwitz BH, DiMaio D. Saturation mutagenesis using mixed oligonucleotides and M13 templates containing uracil. Methods Enzymol. 1990;185:599-611.
Hsu et al., Am. J. Clin. Path. 1980, 75:734-738. Huet-Hudson YM, Chakraborty C, De SK et al. Estrogen regulates the synthesis of epidermal growth factor in mouse uterine epithelial cells. Mol.Endocrinol.1990; 4: 510-523.
Jakobovits A, Moore AL, Green LL et al. Germ-line transmission and expression of a human-derived yeast artificial chromosome. Nature. 1993b Mar 18; 362 (6417) : 255-8.
Jakobovits A, Vergara GJ, Kennedy JL et al., Analysis of homozygous mutant chimeric mice: deletion of the immunoglobulin heavy-chain joining region blocks B-cell development and antibody production. Proc Natl Acad Sci U S A. 1993a Mar 15;90(6):2551-5.
Jiminez et al.,, Nature. 1980, 287:869-871.
Jones EW. Proteinase mutants of Saccharomyces cerevisiae. Genetics. 1977 Jan; 85 (1) :23-33.
Jones PT, Dear PH, Foote J et al., Replacing the complementarity-determining regions in a human antibody with those from a mouse. Nature. 1986 May 29-Jun 4;321 (6069) :522-5.
Jones RW and Jones MJ Simplified filter paper sandwich blot provides rapid, background-free Northern blots. BioTechniques. 1992; 12: 685-688.
Keller et al., In: DNA Probes, Stockton Press, 1989, pp.149-213.
Kelly JM, Hynes MJ. Transformation of Aspergillus niger by the amdS gene of Aspergillus nidulans. EMBO J. 1985 Feb;4(2):475-9.
Kingsman AJ, Clarke L, Mortimer RK et al., Replication in Saccharomyces cerevisiae of plasmid pBR313 carrying DNA from the yeast trpl region. Gene. 1979 Oct; 7 (2) :141-52.
Kingsman SM, Cousens D, Stanway CA et al., High-efficiency yeast expression vectors based on the promoter of the phosphoglycerate kinase gene. Methods Enzymol. 1990;185:329-41.
Kohler G, Milstein C. Continuous cultures of fused cells secreting antibody of predefined specificity. Nature. 1975 Aug 7;256(5517):495-7.
Komminoth P Digoxigenin as an alternative probe labeling for in situ hybridization. Diagn Mol Pathol1992; 1: 142-150.
Kozbor D, Tripputi P, Roder JC et al., A human hybrid myeloma for production of human monoclonal antibodies. J Immunol. 1984 Dec;133 (6) :3001-5.
Kriegler M. Assembly of enhancers, promoters, and splice signals to control expression of transferred genes. Methods Enzymol. 1990;185:512-27.
Kruse & Patterson (eds.) In: Tissue Culture, Academic Press, 1977.
Langer R, Brem H, Tapper D. Biocompatibility of polymeric delivery systems for macromolecules. J Biomed Mater Res. 1981 Mar;15(2):267-77.
Lee F, Yokota T, Otsuka T et al., Isolation of cDNA for a human granulocyte-macrophage colony-stimulating factor by functional expression in mammalian cells. Proc Natl Acad Sci U S A. 1985 Jul; 82 (13):4360-4.
Levinson AD. Expression of heterologous genes in mammalian cells. Methods Enzymol. 1990;185:485-7.
Liang P and Pardee AB Differential display of eukaryotic messenger RNA by means of the polymerase chain reaction. Science 1992; 257: 967-970.
Liang P and Pardee AB Distribution and cloning of eukaryotic mRNAs by means of differential display: refinement and optimization. Nucleic Acids Res.1993; 14: 3269-3275.
Luckow et al., Bio/Technology. 1988, 6:47-55.
Maeda S, Kawai T, Obinata M et al., Production of human alpha-interferon in silkworm using a baculovirus vector. Nature. 1985 Jun 13-19;315(6020):592-4.
Marks JD, Hoogenboom HR, Bonnert TP et al., By-passing immunization. Human antibodies from V-gene libraries displayed on phage. J Mol Biol. 1991 Dec 5; 222(3) : 581-97.
Mather JP. Establishment and characterization of two distinct mouse testicular epithelial cell lines. Biol Reprod. 1980 Aug;23 (1) :243-52.
Mather JP, Zhuang LZ, Perez-Infante V et al., Culture of testicular cells in hormone-supplemented serum-free medium. Ann N'Y Acad Sci. 1982;383:44-68.
Miller et al., In: Genetic Engineering, Plenum Publishing, 1986, vol. 8, pp.277-279.
Miozzari G, Crea R, Seeburg PH. Direct expression in Escherichia coli of a DNA sequence coding for human growth hormone. Nature. 1979 Oct 18;281(5732):544-8.
Morrison SL, Johnson MJ, Herzenberg LA et al., Chimeric human antibody molecules: mouse antigen-binding domains with human constant region domains. Proc Natl Acad Sci U S A. 1984 Nov; 81 (21) :6851-5.
Mullis KB, Faloona FA. Specific synthesis of DNA in vitro via a polymerase-catalyzed chain reaction. Methods Enzymol. 1987;155:335-50.
Narang SA, Hsiung HM, Brousseau R. Improved phosphotriester method for the synthesis of gene fragments. Methods Enzymol. 1979;68:90-8.
Needleman SB, Wunsch CD. A general method applicable to the search for similarities in the amino acid sequence of two proteins. J Mol Biol. 1970 Mar;48(3):443-53.
Nie G-Y, Butt AR, Salamenson LA et al. Hormonal and non-hormonal agents at implantation as targets for contraception. Reprod Fertil Dev1997; 9: 65-76.
Nie G-Y, Li Y, Hampton AL et al. Identification of monoclonal nonspecific suppressor factor beta (MNSFbeta) as one of the genes differentially expressed at implantation sites compared to interimplantation sites in the mouse uterus. Mol Reprod Dev2000b; 55: 351-363.
Nie G-Y, Li Y, Wang J et al. Complex regulation of calcium-binding protein D9k (Calbindin-D9k) in the mouse uterus during early pregnancy and at the site of embryo implantation. Biol Reprod2000a; 62 : 27-36.
Ochman et al., In: PCR Protocols, Academic Press, 1990; pp.219-227.
Okayama H, Berg P. A cDNA cloning vector that permits expression of cDNA inserts in mammalian cells. Mol Cell Biol. 1983 Feb;3 (2) :280-9.
Pain D, Surolia A. Preparation of protein A-peroxidase monoconjugate using a heterobifunctional reagent, and its use in enzyme immunoassays. J Immunol Methods. 1981;40(2):219-30.
Perry LJ, Wetzel R. Disulfide bond engineered into T4 lysozyme: stabilization of the protein toward thermal inactivation. Science. 1984 Nov 2;226(4674):555-7.
Prowse S ANZCCART News 2000 13(3):7
Psychoyos A In: Handbook of physiology, vol II (female reproductive system), part 2., American Physiological Society, Washington, 1973, pp. 187-215. Rademacher TW, Parekh RB, Dwek RA. Glycobiology. Annu Rev Biochem. 1988;57:785-838.
Rangagwala et al., Bio/Technology. 1991, 9:477-479..
Riechmann L, Clark M, Waldmann H et al., Reshaping human antibodies for therapy. Nature. 1988 Mar 24;332 (6162):323-7.
Ringold G, Dieckmann B, Lee F. Co-expression and amplification of dihydrofolate reductase cDNA and the Escherichia coli XGPRT gene in Chinese hamster ovary cells. J Mol Appl Genet. 1981;1(3):165-75.
Robb L, Li R, Hartley L et al. Infertility in female mice lacking the receptor for interleukin 11 is due to a defective uterine response to implantation. Nature Medicine1998; 4: 303-308.
Rugh, R. The mouse. Its reproduction and development. 1994. New York, Oxford University Press.
Saiki RK, Gelfand DH, Stoffel S et al., Primer-directed enzymatic amplification of DNA with a thermostable DNA polymerase. Science. 1988 Jan 29;239(4839):487-91.
Sambrook, J., Fritsch, E. F., and Maniatis, T. Molecular cloning: a laboratory manual. 1989. Cold Spring Harbor, NY, Cold Spring Harbor Laboratory Press.
Sambrook et al., (eds.) In: Molecular Cloning, Cold Spring Harbor Laboratory Press, 1989, pp. 1.74-1.84 and 16.30-16.55.
Sanger et al., Proc. Nat. Acad. Sci. USA. 1979, 72:3918-3921.
Sidman KR, Steber WD, Schwope AD et al., Controlled release of macromolecules and pharmaceuticals from synthetic polypeptides based on glutamic acid. Biopolymers. 1983 Jan;22(1):547-56.
Siebenlist U, Simpson RB, Gilbert W. E. coli RNA polymerase interacts homologously with two different promoters. Cell. 1980 Jun;20(2):269-81.
Spoerel NA, Kafatos FC. Isolation of full-length genes: walking the chromosome. Methods Enzymol. 1987;152:598-603.
Sreekrishna et al., Biochemistry. 1989, 28:4117-4125.
Stewart CL, Kaspar P, Brunet LJ et al. Blastocyst implantation depends on maternal expression of leukaemia inhibitory factor. Nature 1992; 359: 76-79.
Stinchcomb DT, Struhl K, Davis RW. Isolation and characterisation of a yeast chromosomal replicator. Nature. 1979 Nov 1;282 (5734):39-43.
Tilburn J, Scazzocchio C, Taylor GG et al., Transformation by integration in Aspergillus nidulans. Gene. 1983 Dec; 26 (2-3) :205-21.
Triglia T, Peterson MG, Kemp DJ. A procedure for in vitro amplification of DNA segments that lie outside the boundaries of known sequences. Nucleic Acids Res. 1988 Aug 25;16(16):8186.
Tschemper et al., Gene. 1980, 10:157-166.
Urlaub G, Chasin LA. Isolation of Chinese hamster cell mutants deficient in dihydrofolate reductase activity. Proc Natl Acad Sci U S A. 1980 Jul; 77(7) :4216-20.
Vallette F, Mege E, Reiss A et al., Construction of mutant and chimeric genes using the polymerase chain reaction. Nucleic Acids Res. 1989 Jan 25;17 (2) : 723-33.
Verhoeyen M, Milstein C, Winter G. Reshaping human antibodies: grafting an antilysozyme activity. Science. 1988 Mar 25;239 (4847):1534-6.
Wagner et al., In: PCR Topics, Springer-Verlag, 1991, pp.69-71.
Wang et al., In: PCR Protocols, Academic Press, 1990; pp.70-75.
Wells JA, Vasser M, Powers DB. Cassette mutagenesis: an efficient method for generation of multiple mutations at defined sites. Gene. 1985;34(2-3):315-23.
Wong GG, Witek JS, Temple PA et al., Human GM-CSF: molecular cloning of the complementary DNA and purification of the natural and recombinant proteins. Science. 1985 May 17;228 (4701):810-5.
Wu R, Wu T, Ray A Adaptors, linkers, and methylation. Methods Enzymol. 1987;152:343-9.
Yang YC, Ciarletta AB, Temple PA, Chung MP, Kovacic S, Witek-Giannotti JS, Leary AC, Kriz R, Donahue RE, Wong GG, et al. Human IL-3 (multi-CSF): identification by expression cloning of a novel hematopoietic growth factor related to murine IL-3. Cell. 1986 Oct 10;47 (1):3-10.
Yelton MM, Hamer JE, Timberlake WE. Transformation of Aspergillus nidulans by using a trpC plasmid. Proc Natl Acad Sci U S A. 1984 Mar;81(5):1470-4.
Zhu L-J, Cullinan-Bove K, Polihronis M et al. Calcitonin is a progesterone-regulated marker that forecasts the receptive state of endometrium during implantation. Endocrinology 1998; 139: 3923-3934.
Zola Monoclonal Antibodies: A Manual of Techniques, CRC Press Inc, 1987; pp.147-158
Zoller et al., Meths.Enz. 1983; 100: 468-500.
Zoller et al., Meths.Enz. 1987; 154: 329-350.
Zumbrunn J, Trueb B. Primary structure of a putative serine protease specific for IGF-binding proteins. FEBS Lett. 1996 Dec 2;398(2-3):187-92.

## Claims

1. An in vitro method of detecting, diagnosing, or monitoring a condition in a mammal which involves a change in PRSP expression, which condition is infertility caused by inability to achieve or sustain embryo implantation or to sustain pregnancy, the method comprising the step of measuring the amount or activity of a pregnancy related serine protease (PRSP) protein in a biological sample from said mammal suffering from or at risk of such a condition, in which the PRSP protein comprises the sequence provided as SEQ ID NO. 27, 39, 33. or 34.

2. A method according to claim 1, in which the method comprises determining the amount of the PRSP protein.

3. A method according to claim 2, in which the presence or amount of the PRSP protein is detected using an anti-PRSP antibody.

4. A method according to claim 3, in which the anti-PRSP antibody is directed against one of the following segments of the mouse protease:
(i) residues 307-318 of SEQ ID NO. 33;
(ii) residues 116-126 of SEQ ID NO. 27;
(iii) residues 133-142 of SEQ ID NO. 27;
(iv) an epitope of a murine homologue of SEQ ID NO:33 that is shared with human PRSP.

5. A method according to claim 1, in which the method comprises determining the activity of the PRSP protein.

6. A method according to claim 1, in which the method comprises determining the expression of nucleic acid encoding the PRSP protein.

7. A method according to claim 6, in which expression of nucleic acid encoding the PRSP protein is determined by assaying for expression of a nucleic acid sequence comprising SEQ ID NO. 25, 26, 31, 32 or 38.

8. A method according to claim 7, in which expression of a nucleic acid sequence comprising SEQ ID NO. 25, 26, 31, 32 or 38 is determined using as a probe comprising at least 15 contiguous nucleic acid residues specific for SEQ ID NO. 25, 26, 31, 32 or 38.

9. A method according to claim 8, in which the probe comprises at least part of SEQ ID NO. 40 or the first 1243 nucleotides of SEQ ID NO. 31.

10. A method according to claim 1, in which the infertility is caused by an inability to achieve or sustain embryo implantation.

11. A method according to claim 1, in which the infertility caused by an inability to achieve or sustain embryo implantation or to sustain pregnancy is related to insufficiency of placentation.

12. A method according to claim 1, in which the infertility caused by an inability to achieve or sustain embryo implantation or to sustain pregnancy is due to luteal phase defect, failure of implantation, pre-eclampsia, early abortion, intrauterine growth restriction, abnormal uterine bleeding, cancers and parturition or endometriosis.

13. A method according to claim 1, in which the biological sample is a biological fluid, a uterine or bladder washing, a tissue, cells or a tissue or cell extract.

14. A method according to claim 1, in which total RNA of a sample of placental or uterine tissue is assayed for the presence of mRNA for SEQ ID NO. 27, 39, 33 or 34, wherein a decrease in the amount of mRNA is indicative of impaired fertility or impending miscarriage.

15. Use of a PRSP protein comprising the sequence provided as SEQ ID NO. 27, 39, 33 or 34 in the manufacture of a marker for detecting, diagnosing, or monitoring a condition which involves a change in PRSP expression which condition is infertility caused by an inability to achieve or sustain embryo implantation or to sustain pregnancy.

16. Use according to claim 15, in which the infertility is caused by an inability to achieve or sustain embryo implantation.

17. Use according to claim 15, in which the infertility caused by an inability to achieve or sustain embryo implantation or to sustain pregnancy is related to insufficiency of placentation.

18. Use according to claim 15, in which the infertility caused by an inability to achieve or sustain embryo implantation or to sustain pregnancy is due to luteal phase defect, failure of implantation, pre-eclampsia, early abortion, intrauterine growth restriction, abnormal uterine bleeding, cancers, parturition, or endometriosis.

19. An in vitro method of diagnosing impending miscarriage in a mammal comprising measuring the amount or activity of PRSP in a biological sample from said mammal, the PRSP comprising the sequence provided as SEQ ID NO. 27, 39, 33 or 34 or a functionally active variant thereof.

## Patentansprüche

1. In-vitro-Verfahren zum Nachweis bzw. zur Bestimmung, Diagnose oder Überwachung eines Zustands in einem Säugetier bzw. Säuger, der eine Veränderung der PRSP-Exprimierung bzw. Expression beinhaltet, wobei der Zustand eine durch eine Unfähigkeit, eine Embryoimplantation bzw. -einnistung zu erzielen oder aufrecht zu erhalten oder eine Schwangerschaft bzw. Trächtigkeit aufrecht zu erhalten bewirkte Unfruchtbarkeit ist, wobei das Verfahren den Schritt beinhaltet, die Menge oder Aktivität eines schwangerschafts- bzw. trächtigkeits-bezogenen Serinprotease (PRSP)-Proteins in einer bio-logischen Probe von dem von einem derartigen Zustand be-toffenen oder ein Risiko für einen derartigen Zustand aufweisenden Säugetier zu messen, wobei das PRSP-Protein die Sequenz beinhaltet, die als SEQ ID Nr. 27, 39, 33 oder 34 angegeben bzw. bereitgestellt ist.

2. Verfahren nach Anspruch 1, wobei das Verfahren das Bestimmen der Menge des PRSP-Proteins umfasst.

3. Verfahren nach Anspruch 2, bei dem das Vorliegen oder die Menge des PRSP-Proteins mittels eines Anti-PRSP-Antikörpers bestimmt wird.

4. Verfahren nach Anspruch 3, bei dem der Anti-PRSP-Antikörper gegen eines der nachfolgenden Segmente bzw. Abschnitte der Maus-Protease gerichtet ist:
(i) Reste 307-318 der SEQ ID Nr. 33;
(ii) Reste 116-126 der SEQ ID Nr. 27;
(iii) Reste 133-142 der SEQ ID Nr. 27;
(iv) ein Epitop eines Maus-Homologen der SEQ ID Nr. 33, das mit menschlicher PRSP geteilt wird bzw. dieser entspricht.

5. Verfahren nach Anspruch 1, wobei das Verfahren das Bestimmen der Aktivität des PRSP-Proteins umfasst.

6. Verfahren nach Anspruch 1, wobei das Verfahren das Bestimmen der Exprimierung von Nukleinsäure, die für das PRSP-Protein kodiert, umfasst.

7. Verfahren nach Anspruch 6, bei dem die Exprimierung von Nukleinsäure, die für das PRSP-Protein kodiert, durch einen Assay auf die Exprimierung einer Nukleinsäuresequenz, die die SEQ ID Nr. 25, 26, 31, 32 oder 38 beinhaltet, bestimmt wird.

8. Verfahren nach Anspruch 7, bei dem die Exprimierung einer Nukleinsäuresequenz, die die mit SEQ ID Nr. 25, 26, 31, 32 oder 38 beinhaltet, mittels einer Sonde mit wenigstens 15 zusammenhängenden Nukleinsäureresten, die für SEQ ID Nr. 25, 26, 31, 32 oder 38 spezifisch sind, bestimmt wird.

9. Verfahren nach Anspruch 8, bei dem die Sonde wenigstens einen Teil der SEQ ID Nr. 40 oder die ersten 1243 Nukleotide der SEQ ID Nr. 31 beinhaltet.

10. Verfahren nach Anspruch 1, bei dem die Unfruchtbarkeit durch eine Unfähigkeit, eine Embryoeinnistung zu erzielen oder aufrecht zu erhalten bewirkt wird.

11. Verfahren nach Anspruch 1, bei dem die Unfruchtbarkeit, die durch eine Unfähigkeit, eine Embryoeinnistung zu erzielen oder aufrecht zu erhalten oder eine Schwangerschaft aufrecht zu erhalten bewirkt wird, mit einer nicht ausreichenden Plazentation in Beziehung steht.

12. Verfahren nach Anspruch 1, bei dem die Unfruchtbarkeit, die durch eine Unfähigkeit, eine Embryoeinnistung zu erzielen oder aufrecht zu erhalten oder eine Schwangerschaft aufrecht zu erhalten bewirkt wird, auf einem Defekt in der Lutealphase, einem Fehler bei der Einnistung, einer Präeklampsie, einem Frühabort, einer intrauterinen Wachstumsbeschränkung, einer abnormalen Uterusblutung, Krebserkrankungen und Geburtsvorgängen oder einer Endrometriose beruht.

13. Verfahren nach Anspruch 1, bei dem die biologische Probe ein biologisches Fluid, eine Uterus- oder Blasenspülung, ein Gewebe, Zellen oder ein Gewebe- oder Zellextrakt ist.

14. Verfahren nach Anspruch 1, bei dem die gesamte RNA einer Probe von Plazenta- oder Uterusgewebe mittels eines Assays auf das Vorliegen von mRNA für SEQ ID Nr. 27, 39, 33 oder 34 hin untersucht wird, wobei eine Abnahme in der mRNA-Menge eine beeinträchtigte Fruchtbarkeit oder eine bevorstehende Fehlgeburt anzeigt.

15. Verwendung eines PRSP-Proteins, das die als SEQ ID Nr. 27, 39, 33 oder 34 angegebene Sequenz beinhaltet, für die Herstellung eines Markers zur Bestimmung, Diagnose oder Überwachung eines Zustands, der eine Veränderung der PRSP-Exprimierung anzeigt, wobei der Zustand eine durch eine Unfähigkeit eine Embryoeinnistung zu erzielen oder aufrecht zu erhalten oder eine Schwangerschaft aufrecht zu erhalten bedingte Unfruchtbarkeit ist.

16. Verwendung nach Anspruch 15, bei dem die Unfruchtbarkeit durch eine Unfähigkeit, eine Embryoeinnistung zu erzielen oder aufrecht zu erhalten, bedingt ist.

17. Verwendung nach Anspruch 15, bei der die Unfruchtbarkeit, die durch eine Unfähigkeit, eine Embryoeinnistung zu erzielen oder aufrecht zu erhalten oder eine Schwangerschaft aufrecht zu erhalten bedingt ist, mit einer unzureichenden Plazentation in Beziehung steht.

18. Verwendung nach Anspruch 15, bei der die Unfruchtbarkeit, die durch eine Unfähigkeit, eine Embryoeinnistung zu erzielen oder aufrecht zu erhalten oder eine Schwangerschaft aufrecht zu erhalten bedingt ist, auf einem Defekt in der Lutealphase, einem Fehler bei der Einnistung, einer Präeklampsie, einem Frühabort, einer intrauterinen Wachstumsbeschränkung, einer abnormalen Uterusblutung, Krebserkrankungen, Geburtsvorgängen oder einer Endrometriose beruht.

19. In-vitro-Verfahren zur Diagnose einer bevorstehenden Fehlgeburt in einem Säugetier, das umfasst, die Menge oder Aktivität von PRSP in einer biologischen Probe von dem Säugetier zu messen, wobei die PRSP die als SEQ ID Nr. 27, 39, 33 oder 34 angegebene Sequenz oder eine funktional aktive Variante hiervon beinhaltet.

## Revendications

1. Procédé in vitro de détection, diagnostic ou surveillance d'une condition d'un mammifère qui implique un changement dans l'expression PRSP, laquelle condition étant la stérilité provoquée par l'incapacité à réaliser ou à maintenir une implantation embryonnaire ou à maintenir la gestation, le procédé comportant l'étape consistant à mesurer la quantité ou l'activité d'une protéine sérine protéase (PRSP) associée à la gestation dans un échantillon biologique dudit mammifère souffrant de cette condition ou potentiellement sujet à celle-ci, la protéine PRSP comportant la séquence délivrée sous la forme ou indiquée dans SEQ ID NO. 27, 39, 33 ou 34.

2. Procédé selon la revendication 1, dans lequel le procédé comporte l'étape consistant à déterminer la quantité de la protéine PRSP.

3. Procédé selon la revendication 2, dans lequel la présence ou la quantité de la protéine PRSP est détectée en utilisant un anticorps anti-PRSP.

4. Procédé selon la revendication 3, dans lequel l'anticorps anti-PRSP est dirigé contre l'un des segments suivants de la protéase chez la souris
(i) résidus 307-318 de SEQ ID No. 33 ;
(ii) résidus 116-126 de SEQ ID No. 27 ;
(iii) résidus 133-142 de SEQ ID No. 27 ;
(iv) un épitope d'un murin homologue de SEQ ID NO. 33 qui est partagé avec la ou qui corresponde à la PRSP humaine.

5. Procédé selon la revendication 1, dans lequel le procédé comporte l'étape consistant à déterminer l'activité de la protéine PRSP.

6. Procédé selon la revendication 1, dans lequel le procédé comporte l'étape consistant à déterminer l'expression de l'acide nucléique codant la protéine PRSP.

7. Procédé selon la revendication 6, dans lequel l'expression de l'acide nucléique codant la protéine PRSP est déterminée en testant l'expression d'une séquence d'acide nucléique comportant SEQ ID NO. 25, 26, 31, 32 ou 38.

8. Procédé selon la revendication 7, dans lequel l'expression d'une séquence d'acide nucléique comportant SEQ ID NO. 25, 26, 31, 32 ou 38 est déterminée en utilisant une sonde comportant au moins 15 résidus d'acide nucléique contigus spécifiques pour SEQ ID NO. 25, 26, 31, 32 ou 38.

9. Procédé selon la revendication 8, dans lequel la sonde comporte au moins une partie de SEQ ID NO. 40 ou les 1243 premiers nucléotides de SEQ ID NO. 31.

10. Procédé selon la revendication 1, dans lequel la stérilité est provoquée par l'incapacité à réaliser ou à maintenir une implantation embryonnaire.

11. Procédé selon la revendication 1, dans lequel la stérilité provoquée par l'incapacité à réaliser ou à maintenir une implantation embryonnaire ou à maintenir une gestation est associée à une insuffisance de placentation.

12. Procédé selon la revendication 1, dans lequel la stérilité provoquée par l'incapacité à réaliser ou à maintenir une implantation embryonnaire ou à maintenir une gestation est due à une défaillance de la phase lutéale, à un échec de l'implantation, à une pré-éclampsie, à un avortement précoce, à un retard de croissance intra-utérin, à un saignement utérin anormal, à des cancers et à une parturition ou à une endométriose.

13. Procédé selon la revendication 1, dans lequel l'échantillon biologique est un fluide biologique, un lavage utérin ou vésical, un tissu, des cellules ou un tissu ou une extraction de cellules.

14. Procédé selon la revendication 1, dans lequel l'ARN total d'un échantillon de tissu placentaire ou utérin est testé relativement à la présence de mARN pour SEQ ID NO. 27, 39, 33 ou 34, une diminution de la quantité de mARN étant indicative d'une diminution de la fécondité ou d'un avortement spontané imminent.

15. Utilisation d'une protéine PRSP comportant la séquence fournie sous la forme SEQ ID NO. 27, 39, 33 ou 44 lors de la conception d'un marqueur pour détecter, diagnostiquer ou surveiller une condition qui implique un changement dans l'expression PRSP, laquelle condition étant la stérilité provoquée par l'incapacité à réaliser ou à maintenir une implantation embryonnaire ou à maintenir la gestation.

16. Utilisation selon la revendication 15, dans laquelle la stérilité est provoquée par l'incapacité à réaliser ou à maintenir une implantation embryonnaire.

17. Utilisation selon la revendication 15, dans laquelle la stérilité provoquée par l'incapacité à réaliser ou à maintenir une implantation embryonnaire ou à maintenir une gestation est associée à une insuffisance de placentation.

18. Utilisation selon la revendication 15, dans laquelle la stérilité provoquée par l'incapacité à réaliser ou à maintenir une implantation embryonnaire ou à maintenir une gestation est due à une défaillance de la phase lutéale, à un échec de l'implantation, à une pré-éclampsie, à un avortement précoce, à un retard de croissance intra-utérin, à un saignement utérin anormal, à des cancers et à une parturition ou à une endométriose.

19. Procédé in vitro consistant à diagnostiquer un avortement spontané imminent comportant de mesurer la quantité ou l'activité de la PRSP dans un échantillon biologique dudit mammifère, la PRSP comportant la séquence fournie sous la forme ou indiquée dans SEQ ID NO. 27, 39, 33 ou 34 ou un variant de celle-ci actif d'un point de vue fonctionnel.
